(19) Europäisches Patentamt
European Patent Office
Office européen des brevets

(11) EP 3 976 598 B1

# (12) EUROPEAN PATENT SPECIFICATION

(45) Date of publication and mention of the grant of the patent:
**27.03.2024 Bulletin 2024/13**

(21) Application number: **20730747.1**

(22) Date of filing: **29.05.2020**

(51) International Patent Classification (IPC):
***C07D 401/12*** *(2006.01)* ***A61K 31/454*** *(2006.01)*
***A61P 25/00*** *(2006.01)*

(52) Cooperative Patent Classification (CPC):
**C07D 401/12; A61P 25/00**

(86) International application number:
**PCT/IB2020/055105**

(87) International publication number:
**WO 2020/240490 (03.12.2020 Gazette 2020/49)**

## (54) SELECTIVE HISTAMINE H3 ANTAGONIST ACID ADDITION SALTS AND PROCESS FOR THE PREPARATION THEREOF

SÄUREADDITIONSSALZE EINES SELEKTIVEN HISTAMIN-H3-ANTAGONISTEN UND VERFAHREN ZU IHRER HERSTELLUNG

SELS D'ADDITION D'ACIDE ANTAGONISTES DE L'HISTAMINE H3 SÉLECTIFS ET LEUR PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2019 HU 1900193**

(43) Date of publication of application:
**06.04.2022 Bulletin 2022/14**

(73) Proprietor: **Richter Gedeon Nyrt.**
**1103 Budapest (HU)**

(72) Inventors:
- **SEBÕK, Ferenc**
**2217 Gomba (HU)**
- **MEISZTERICS, Anikó**
**1138 Budapest (HU)**
- **DEMETER, Ádám**
**1163 Budapest (HU)**

(74) Representative: **Biacs, Mónika**
**Gedeon Richter Plc.**
**Intellectual Property Department**
**Gyömröi út 19-21**
**1103 Budapest (HU)**

(56) References cited:
**WO-A1-2014/136075**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 976 598 B1

## Description

### THE FIELD OF THE INVENTION

The present invention relates to physically and chemically stable salts of the selective histamine $H_3$ receptor antagonist compound of 1-[4-(4-{3-[(2R)-2-methyl-pyrrolidin-1-yl]-propoxy}-phenoxy)-piperidin-1-yl]-ethanone of formula (1)

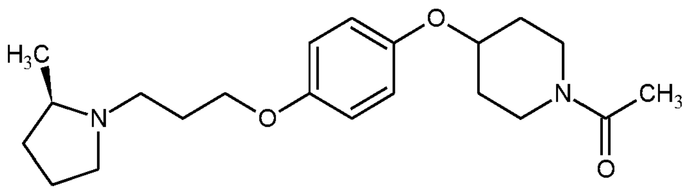

formula (1)

and/or polymorphs thereof and/or hydrates/solvates thereof, the process for the preparation thereof, pharmaceutical compositions comprising them, and for use in the treatment and/or prevention of conditions requiring the modulation of histamine $H_3$ receptors (e.g. Alzheimer's disease, obesity, schizophrenia, myocardial ischaemia, migraine, autism spectrum disorder). The salts are defined in the claims.

### THE BACKGROUND OF THE INVENTION

The histamine $H_3$ receptor antagonists were extensively studied aiming to produce drugs that would enable the treatment of different diseases, such as Alzheimer's disease, obesity, schizophrenia, myocardial ischaemia, migraine, nasal congestion etc. (Leurs et al., Nat. Rev. Drug. Disc. 2005, 4(2):107-120; Berlin et al., J. Med. Chem. 2011, 54(1):26-53). Numerous compound showed promising preclinical results and entered clinical phase in diseases such as excessive daytime sleepiness (EDS) associated with Parkinson's disease, obstructive sleep apnea, epilepsy, schizophrenia, dementia, and attention deficit hyperactivity disorder (Kuhne et al., Exp. Opin. Inv. Drugs 2011, 20(12):1629-1648). It has been suggested that histamine $H_3$ receptor antagonists/inverse agonists may also be suitable for pharmacotherapeutic treatment of sleep disorders (Barbier and Bradbury, CNS Neurol. Disord. Drug Targets 2007, 6(1):31-43), but so far, only one histamine $H_3$ receptor antagonist, pitolisant (under the Wakix brand), has been granted marketing authorization for the treatment of narcolepsy with or without cataplexy in adults (Kollb-Sielecka et al., Sleep Med. 2017, 33:125-129).

WO 2014/136075 describes the synthesis of chemically modifiable, selective and drug-like $H_3$ antagonists and inverse agonists. The preparation and characterization of such phenoxypiperidine-derived compounds are disclosed therein that bind to $H_3$ receptor with high affinity and high selectivity and are drug-like.

Among the compounds disclosed in WO 2014/136075, the hydrochloride salt of 1-[4-(4-{3-[(2R)-2-methyl-pyrrolidin-1-yl]-propoxy}-phenoxy)-piperidin-1-yl]-ethanone of formula (1) is highlighted. In the preparation of the compound as described in Example 11, the starting material was 4-(4-{3-[(2R)-2-methyl-pyrrolidin-1-yl]-propoxy}-phenoxy)-piperidine dihydrochloride salt. After the base is released, the reaction mixture is treated with acetyl chloride in dichloromethane, and after the aqueous extraction work-up of the reaction mixture, the dried solution of the resulting base of formula (1) in dichloromethane was evaporated. To a solution of the crude product in dichloromethane excess hydrochloric acid in ethyl acetate was added. The precipitate was filtered off with ethyl acetate and washed with diethyl ether to give a crystalline product, the hydrochloride salt of 1-[4-(4-{3-[(2R)-2-methyl-pyrrolidin-1-yl]-propoxy}-phenoxy)-piperidin-1-yl]-ethanone.

A general requirement for active ingredients in the development of a pharmaceutical composition is that the active ingredient has the appropriate physical, physico-chemical and chemical parameters. Examples of such parameters include solubility, in particular water solubility. Another important feature that should be taken into account in industrial-scale production is the easy handling and the good isolability, which is extremely important for the economicalness of the manufacturing process. A further important aspect is that the solid form of the active ingredient has appropriate physical and chemical stability, for example, not hygroscopic, and does not degrade significantly. Furthermore, different polymorphic forms of a given salt may have different solid phase characteristics, physical and chemical stability.

From a drug development perspective, the water-binding tendency of a substance, the degree of hygroscopicity (ability of absorbency), is of paramount importance, since ambient humidity means a meaningful interaction in addition to the temperature. The degree of hygroscopicity of active ingredients affects the handling, storage, stability, formulability and many other qualities of the substance. There are several approaches and methods to characterize the hygroscopic

properties of the active ingredients, and to categorize the degree of hygroscopicity, which is summarized in detail by Newman et al. (Newman et al., J. Pharm. Sci. 2007, 97(3):1047-1059). Typically, non-hygroscopic, slightly hygroscopic, moderately hygroscopic, very hygroscopic, as well as deliquescent categories are used in the literature, while in the pharmacopeia (European Pharmacopeia 9.0, 5.11 Character Section in Monographs) the less hygroscopic, hygroscopic, highly hygroscopic and deliquescent categories are used depending on the weight gain at the given temperature and relative humidity under the test conditions, in a given time. There are static and dynamic measurement methods for the investigation of hygroscopic tendency. Among the dynamic measurements Dynamic Vapor Sorption (DVS) analysis is a technique commonly used in the pharmaceutical industry, which typically measures mass change of the substance (sorption and desorption curve) as a function of relative humidity in isothermic conditions, from which the nature, mechanism and phase transitions of the sorption process can be inferred.

For testing hygroscopicity of active substances it is particularly important to determine whether the substance is susceptible to deliquescence, i.e. what is the point at which the solid material is in dissolved state when interacting with the ambient humidity (Mauer et al., Pharm. Dev. Techn. 2010, 15(6):582-594). Deliquescence of the substance occurs when the relative humidity (RH) reaches or exceeds the critical relative humidity (CRH) when a film corresponding to a saturated solution of the substance is formed on the surface of the solid substance. By further increasing the humidity the substance continuously takes up moisture, leading to drastic weight gain due to the complete dissolution of the material and dilution of the resulting solution. Even a slight surface deliquescence of the substance might have a significant effect on the chemical stability of the compound, since typically in case of compounds with acidic or basic characteristic such microenvironment might occur that leads to the degradation of acid or alkali-sensitive compounds. Deliquescence and strong ability to absorb moisture of the crystalline drugs are typically due to their good solubility.

Determination of the critical relative humidity is feasible by gravimetric method, e.g. with DVS, where relative humidity is changed in suitably selected steps and a sufficiently long time is used to the onset of quasi-equilibrium. After reaching the critical relative humidity, the sorption curve shows a more or less sharp change in the slope, typically followed by a monotonous rise and a significant increase in mass, the extent of which and the shape of the sorption curve cannot be associated with the formation of a hydrate form.

## SUMMARY OF THE INVENTION

The base form of the salts of the present invention, the 1-[4-(4-{3-[(2R)-2-methyl-pyrrolidin-1-yl]-propoxy}-phenoxy)-piperidin-1-yl]-ethanone of formula (1), cannot be isolated in crystalline form, but as oil.

The aim was to obtain a solid form (salt and/or polymorph) of 1-[4-(4-{3-[(2R)-2-methylpyrrolidin-1-yl]-propoxy}-phenoxy)-piperidin-1-yl]-ethanone which possesses appropriate properties with regard to the above mentioned aspects, exhibiting adequate physical and chemical stability, slightly hygroscopic, not deliquescent, thereby its isolation is facilitated, handling is better and has excellent solubility.

It has been found during the preparation of the crystalline form of the hydrochloride acid addition salts of the 1-[4-(4-{3-[(2R)-2-methyl-pyrrolidin-1-yl]-propoxy}-phenoxy)-piperidin-1-yl]-ethanone base, that two crystalline polymorphs (Form A and Form B) of the monohydrochloride stoichiometry can be produced. In addition, the crystalline dihydrochloride salt of the compound can also be produced besides the monohydrochloride.

Surprisingly, it has been found that in contrast to the 1-[4-(4-{3-[(2R)-2-methyl-pyrrolidin-1-yl]-propoxy}-phenoxy)-piperidin-1-yl]-ethanone monohydrochloride and dihydrochloride salts the novel dihydrobromide, sulfate, oxalate, monocitrate and dicitrate salts have outstanding properties, are less hygroscopic, easier to be isolated, their physical and chemical stability are more favorable, and have excellent solubility. All of these advantageous properties of the novel 1-[4-(4-{3-[(2R)-2-methylpyrrolidin-1-yl]-propoxy}-phenoxy)-piperidin-1-yl]-ethanone dihydrobromide, sulfate, oxalate, monocitrate, and dicitrate salts make them suitable for the development of a pharmaceutical composition for the treatment of diseases targeting the selective modulation of $H_3$ receptor.

The present invention relates to dihydrobromide, sulfate, oxalate, monocitrate and dicitrate salts of 1-[4-(4-{3-[(2R)-2-methyl-pyrrolidin-1-yl]-propoxy}-phenoxy)-piperidin-1-yl]-ethanone, and/or polymorphs thereof and/or hydrates/solvates thereof, the process for the preparation thereof, pharmaceutical compositions comprising them, and the use thereof in the treatment and/or prevention of conditions requiring the modulation of histamine $H_3$ receptors (e.g. Alzheimer's disease, obesity, schizophrenia, myocardial ischaemia, migraine, autism spectrum disorder).

## BRIEF DESCRIPTION OF THE FIGURES

**Figure 1** X-ray powder diffraction (XRPD) pattern of 1-[4-(4-{3-[(2R)-2-methyl-pyrrolidin-1-yl]-propoxy}-phenoxy)-piperidin-1-yl]-ethanone monohydrochloride salt Form A (Example 6).

**Figure 2** Dynamic vapor sorption (DVS) isotherm plot of 1-[4-(4-{3-[(2R)-2-methyl-pyrrolidin-1-yl]-propoxy}-phenoxy)-piperidin-1-yl]-ethanone monohydrochloride salt Form A (Example 6).

**Figure 3** X-ray powder diffraction (XRPD) pattern of 1-[4-(4-{3-[(2R)-2-methyl-pyrrolidin-1-yl]-propoxy}-phenoxy)-piperidin-1-yl]-ethanone monohydrochloride salt Form B (Example 7).

**Figure 4** Infrared spectrum (IR) of 1-[4-(4-{3-[(2R)-2-methyl-pyrrolidin-1-yl]-propoxy}-phenoxy)-piperidin-1-yl]-ethanone monohydrochloride salt Form B (Example 7).

**Figure 5** Raman spectrum (Raman) of 1-[4-(4-{3-[(2R)-2-methyl-pyrrolidin-1-yl]-propoxy}-phenoxy)-piperidin-1-yl]-ethanone monohydrochloride salt Form B (Example 7).

**Figure 6** Dynamic vapor sorption (DVS) isotherm plot of 1-[4-(4-{3-[(2R)-2-methyl-pyrrolidin-1-yl]-propoxy}-phenoxy)-piperidin-1-yl]-ethanone monohydrochloride salt Form B (Example 7).

**Figure 7** X-ray powder diffraction (XRPD) pattern of 1-[4-(4-{3-[(2R)-2-methyl-pyrrolidin-1-yl]-propoxy}-phenoxy)-piperidin-1-yl]-ethanone dihydrochloride salt (Example 2).

**Figure 8** Termogravimetric (TG) curve of 1-[4-(4-{3-[(2R)-2-methyl-pyrrolidin-1-yl]-propoxy}-phenoxy)-piperidin-1-yl]-ethanone dihydrochloride salt (Example 2).

**Figure 9** Differential scanning calorimetry (DSC) thermogram of 1-[4-(4-{3-[(2R)-2-methyl-pyrrolidin-1-yl]-propoxy} -phenoxy)-piperidin-1-yl]-ethanone dihydrochloride salt (Example 2).

**Figure 10** Infrared spectrum (IR) of 1-[4-(4-{3-[(2R)-2-methyl-pyrrolidin-1-yl]-propoxy}-phenoxy)-piperidin-1-yl]-ethanone dihydrochloride salt (Example 2).

**Figure 11** Raman spectrum (Raman) of 1-[4-(4-{3-[(2R)-2-methyl-pyrrolidin-1-yl]-propoxy}-phenoxy)-piperidin-1-yl]-ethanone dihydrochloride salt (Example 2).

**Figure 12** Dynamic vapor sorption (DVS) isotherm plot of 1-[4-(4-{3-[(2R)-2-methyl-pyrrolidin-1-yl]-propoxy}-phenoxy)-piperidin-1-yl]-ethanone dihydrochloride salt (Example 2).
Hiba! A hivatkozasi forrás nem található. Dynamic vapor sorption curves of the salts tested (relative weight change% - relative humidity%) at 25°C (a) deliquescent salts (b) not deliquescent salts.

**Figure 15** X-ray powder diffraction (XRPD) pattern of 1-[4-(4-{3-[(2R)-2-methyl-pyrrolidin-1-yl]-propoxy}-phenoxy)-piperidin-1-yl]-ethanone monocitrate salt Form A (Example 17).

**Figure 16** Infrared spectrum (IR) of 1-[4-(4-{3-[(2R)-2-methyl-pyrrolidin-1-yl]-propoxy}-phenoxy)-piperidin-1-yl]-ethanone monocitrate salt Form A (Example 17).

**Figure 17** Raman spectrum (Raman) of 1-[4-(4-{3-[(2R)-2-methyl-pyrrolidin-1-yl]-propoxy}-phenoxy)-piperidin-1-yl]-ethanone monocitrate salt Form A (Example 17).

**Figure 18** X-ray powder diffraction (XRPD) pattern of 1-[4-(4-{3-[(2R)-2-methyl-pyrrolidin-1-yl]-propoxy}-phenoxy)-piperidin-1-yl]-ethanone monocitrate salt Form B (Example 18).

**Figure 19** Infrared spectrum (IR) of 1-[4-(4-{3-[(2R)-2-methyl-pyrrolidin-1-yl]-propoxy}-phenoxy)-piperidin-1-yl]-ethanone monocitrate salt Form B (Example 18).

**Figure 20** Raman spectrum (Raman) of 1-[4-(4-{3-[(2R)-2-methyl-pyrrolidin-1-yl]-propoxy}-phenoxy)-piperidin-1-yl]-ethanone monocitrate salt Form B (Example 18).

**Figure 21** Dynamic vapor sorption (DVS) isotherm plot of 1-[4-(4-{3-[(2R)-2-methyl-pyrrolidin-1-yl]-propoxy}-phenoxy)-piperidin-1-yl]-ethanone monocitrate salt (Example 17).

**Figure 22** X-ray powder diffraction (XRPD) pattern of 1-[4-(4-{3-[(2R)-2-methyl-pyrrolidin-1-yl]-propoxy}-phenoxy)-piperidin-1-yl]-ethanone dicitrate salt (Example 20).

**Figure 23** Infrared spectrum (IR) of 1-[4-(4-{3-[(2R)-2-methyl-pyrrolidin-1-yl]-propoxy}-phenoxy)-piperidin-1-yl]-ethanone dicitrate salt (Example 20).

**Figure 24** Raman spectrum (Raman) of 1-[4-(4-{3-[(2R)-2-methyl-pyrrolidin-1-yl]-propoxy}-phenoxy)-piperidin-1-yl]-ethanone dicitrate salt (Example 20).

**Figure 25** Dynamic vapor sorption (DVS) isotherm plot of 1-[4-(4-{3-[(2R)-2-methyl-pyrrolidin-1-yl]-propoxy}-phenoxy)-piperidin-1-yl]-ethanone dicitrate salt (Example 20).

**Figure 26** X-ray powder diffraction (XRPD) pattern of 1-[4-(4-{3-[(2R)-2-methyl-pyrrolidin-1-yl]-propoxy}-phenoxy)-piperidin-1-yl]-ethanone dihydrobromide salt (Example 9).

**Figure 27** Infrared spectrum (IR) of 1-[4-(4-{3-[(2R)-2-methyl-pyrrolidin-1-yl]-propoxy}-phenoxy)-piperidin-1-yl]-ethanone dihydrobromide salt (Example 9).

**Figure 28** Raman spectrum (Raman) of 1-[4-(4-{3-[(2R)-2-methyl-pyrrolidin-1-yl]-propoxy}-phenoxy)-piperidin-1-yl]-ethanone dihydrobromide salt (Example 9).

**Figure 29** Dynamic vapor sorption (DVS) isotherm plot of 1-[4-(4-{3-[(2R)-2-methyl-pyrrolidin-1-yl]-propoxy}-phenoxy)-piperidin-1-yl]-ethanone dihydrobromide salt (Example 9).

**Figure 30** X-ray powder diffraction (XRPD) pattern of 1-[4-(4-{3-[(2R)-2-methyl-pyrrolidin-1-yl]-propoxy}-phenoxy)-piperidin-1-yl]-ethanone sulfate salt (Example 10).

**Figure 31** Infrared spectrum (IR) of 1-[4-(4-{3-[(2R)-2-methyl-pyrrolidin-1-yl]-propoxy}-phenoxy)-piperidin-1-yl]-ethanone sulfate salt (Example 10).

**Figure 32** Raman spectrum (Raman) of 1-[4-(4-{3-[(2R)-2-methyl-pyrrolidin-1-yl]-propoxy}-phenoxy)-piperidin-1-yl]-ethanone sulfate salt (Example 10).

**Figure 33** Dynamic vapor sorption (DVS) isotherm plot of 1-[4-(4-{3-[(2R)-2-methyl-pyrrolidin-1-yl]-propoxy}-phenoxy)-piperidin-1-yl]-ethanone sulfate salt (Example 10).

**Figure 34** X-ray powder diffraction (XRPD) pattern of 1-[4-(4-{3-[(2R)-2-methyl-pyrrolidin-1-yl]-propoxy}-phenoxy)-piperidin-1-yl]-ethanone oxalate salt (Example 13).

**Figure 35** Infrared spectrum (IR) of 1-[4-(4-{3-[(2R)-2-methyl-pyrrolidin-1-yl]-propoxy}-phenoxy)-piperidin-1-yl]-ethanone oxalate salt (Example 13).

**Figure 36** Raman spectrum (Raman) of 1-[4-(4-{3-[(2R)-2-methyl-pyrrolidin-1-yl]-propoxy}-phenoxy)-piperidin-1-yl]-ethanone oxalate salt (Example 13).

**Figure 37** Dynamic vapor sorption (DVS) isotherm plot of 1-[4-(4-{3-[(2R)-2-methyl-pyrrolidin-1-yl]-propoxy}-phenoxy)-piperidin-1-yl]-ethanone oxalate salt (Example 13).

## DETAILED DESCRIPTION OF THE INVENTION

The base form of the salts of the present invention, the 1-[4-(4-{3-[(2R)-2-methyl-pyrrolidin-1-yl]-propoxy}-phenoxy)-piperidin-1-yl]-ethanone of formula (1), cannot be isolated in crystalline form, but as oil. The base according to the procedure described in Example 11 of WO 2014/136075 can be obtained by evaporating the dichloromethane solution of the resulting product or, after isolation of the hydrochloride salt - in a manner obvious to the skilled person - by base releasing.

The hydrochloride acid addition salts of 1-[4-(4-{3-[(2R)-2-methyl-pyrrolidin-1-yl]-propoxy}-phenoxy)-piperidin-1-yl]-ethanone base (Example 1) are prepared in crystalline form (Example 2 to Example 8). It has been found that two crystalline polymorphs (Form A and Form B) of the salt characterized by monohydrochloride stoichiometry can be produced (Example 4 to Example 8), of which X-ray powder diffraction (XRPD) patterns, infrared (IR) and Raman spectra, and dynamic vapor sorption (DVS) isotherm plot are shown in Figure 1 to Figure **6.** Both monohydrochloride polymorphs (Form A and Form B) are highly hygroscopic and prone to deliquescence. Based on the DVS analysis at 25°C, Form A

has, above 40% relative humidity, and Form B has, yet above 30% relative humidity, a high, continuous weight gain in the sorption process which is caused by the deliquescence of the substance.

In further experiments, it was found that crystalline dihydrochloride salt (diHCl) of the compound can also be produced (Example 2 and Example 3) in addition to the monohydrochloride, of which X-ray powder diffraction (XRPD) pattern, termogravimetric (TG) curve, differential scanning calorimetry (DSC) thermogram, infrared (IR) and Raman spectra, and dynamic vapor sorption (DVS) isotherm plot are shown in Figure 7 to Figure ***12.*** The compound of 1-[4-(4-{3-[(2R)-2-methyl-pyrrolidin-1-yl]-propoxy}-phenoxy)-piperidin-1-yl]-ethanone contains a single strongly basic center (pyrrolidine nitrogen), which is capable of forming stoichiometric salt with equimolar hydrochloride, thus the formation of dihydrochloride stoichiometry is not expected in view of the acid/base character of the compound. Based on TG and DSC analysis, the second molar amount of hydrochloride is less strongly bound to the crystal lattice, behaving as a volatile component. The compound is thermally poorly stable, according to the TG analysis the loss of volatile HCl can already be observed at room temperature, but becomes intensive at about 70 to 80°C (Figure 8). Parallelly to this process, according to the DSC and microscopic analysis, the sample starting from approx. 100°C melts during decomposition (Figure 9).

A further disadvantage of the dihydrochloride form is that, it is highly hygroscopic, according to the DVS (dynamic vapor sorption) analysis at 25°C, a significant monotonic weight increase is observed on the sorption curve above 60% relative humidity, showing the deliquescence of the substance (Figure 12).

The hygroscopic nature of the mono- and dihydrochloride salts of 1-[4-(4-{3-[(2R)-2-methylpyrrolidin-1-yl]-propoxy}-phenoxy)-piperidin-1-yl]-ethanone poses many issues in terms of the pharmaceutical development, handling, storing, stability and formulability of the compound. It has been observed that hydrochloride salts are already susceptible to deliquescence under the conditions of isolation, their filtering and handling are thus problematic. The degradation tendency of the substance is also clearly related to its hygroscopic nature, as the deacetylation of the compound may occur due to exposure to acid in the presence of moisture.

It is therefore necessary to produce salts of 1-[4-(4-{3-[(2R)-2-methyl-pyrrolidin-1-yl]-propoxy}-phenoxy)-piperidin-1-yl]-ethanone that are less hygroscopic, easier to handle, physically and chemically more stable than mono- and dihydrochloride salts.

In our experiments, dihydrobromide salt (Example 9), sulfate salt (Example 10 to Example 12), oxalate salt (Example 13 and Example 14), monocitrate salt (Example 15 to Example 18) and dicitrate salt (Example 19 to Example 22) of 1-[4-(4-{3-[(2R)-2-methyl-pyrrolidin-1-yl]-propoxy}-phenoxy)-piperidin-1-yl]-ethanone was prepared in crystalline form, which are more preferred than the mono- and dihydrochloride salts, as these are less hygroscopic (Table 1), thus easier to isolate and handle, and their stability is much more favorable (Table 2).

X-ray powder diffraction, IR and Raman data suitable to characterize polymorphs of crystalline salts of 1-[4-(4-{3-[(2R)-2-methyl-pyrrolidin-1-yl]-propoxy}-phenoxy)-piperidin-1-yl]-ethanone are shown in Table 3 to Table ***5.***

Thus, the present invention relates to pharmaceutically acceptable, less hygroscopic, acid addition salts of 1-[4-(4-{3-[(2R)-2-methyl-pyrrolidin-1-yl]-propoxy}-phenoxy)-piperidin-1-yl]-ethanone that can be formed with organic or inorganic acids and/or polymorphs thereof and/or hydrates/solvates thereof. The acid addition salts are salts derived from hydrogen bromide, sulfuric acid, oxalic acid, or citric acid.

Preferably, the present invention relates to 1-[4-(4-{3-[(2R)-2-methyl-pyrrolidin-1-yl]-propoxy}-phenoxy)-piperidin-1-yl]-ethanone dihydrobromide, sulfate, oxalate, monocitrate and dicitrate salts and/or polymorphs thereof and/or hydrates/solvates thereof.

The present invention also relates to the preparation of pharmaceutically acceptable, less hygroscopic, acid addition salts of 1-[4-(4-{3-[(2R)-2-methyl-pyrrolidin-1-yl]-propoxy}-phenoxy)-piperidin-1-yl]-ethanone that can be formed with organic or inorganic acids, selected from dihydrobromide, sulfate, oxalate, monocitrate and dicitrate salts thereof, and/or polymorphs thereof and/or hydrates/solvates thereof.

The present invention also relates to 1-[4-(4-{3-[(2R)-2-methyl-pyrrolidin-1-yl]-propoxy}-phenoxy)-piperidin-1-yl]-ethanone dihydrobromide, sulfate, oxalate, monocitrate and dicitrate salts for use in the treatment and/or prevention of conditions requiring the modulation of histamine $H_3$ receptors.

The present invention relates to the use of 1-[4-(4-{3-[(2R)-2-methyl-pyrrolidin-1-yl]-propoxy}-phenoxy)-piperidin-1-yl]-ethanone salts in the manufacture of a pharmaceutical composition.

The present invention also relates to a pharmaceutical composition comprising a therapeutically effective amount of 1-[4-(4-{3-[(2R)-2-methyl-pyrrolidin-1-yl]-propoxy}-phenoxy)-piperidin-1-yl]-ethanone dihydrobromide, sulfate, oxalate, monocitrate and dicitrate salts together with pharmaceutically acceptable excipients.

The present invention also relates to pharmaceutical composition of the previous paragraph for use in the treatment and/or prevention of conditions requiring the modulation of histamine $H_3$ receptors, preferably in the treatment and/or prevention of autism spectrum disorder.

For example, the preparation of salts from the base can be carried out as follows: the 1-[4-(4-{3-[(2R)-2-methyl-pyrrolidin-1-yl]-propoxy}-phenoxy)-piperidin-1-yl]-ethanone base is dissolved in a suitable solvent or mixture of solvents, followed by the addition of the acid or a salt thereof - formed by a base weaker than 1-[4-(4-{3-[(2R)-2-methyl-pyrrolidin-1-yl]-propoxy}-phenoxy)-piperidin-1-yl]-ethanone - or a solution thereof, to the mixture. In addition, the 1-[4-(4-{3-[(2R)-2-methyl-pyrrolidin-1-yl]-propoxy}-phenoxy)-piperidin-1-yl]-ethanone base can be prepared from a salt thereof, and after releasing the base, after the appropriate separation and/or solvent exchange, the desired salt is formed by addition of the acid, without isolation of the base. If necessary, the reaction mixture is concentrated, the precipitated product is isolated by filtration at room temperature or after cooling, then dried, if necessary, at an appropriate temperature. If necessary, the resulting salt is crystallized by addition of a suitable antisolvent from its solution at room temperature or after reflux, and the precipitated product is isolated by filtration, then dried, if necessary, at an appropriate temperature.

The salts of the present invention can be well isolated and as a result of the process obtainable in high purity, which makes them particularly valuable for pharmaceutical use. In terms of implementation of the present invention, the monocitrate and dicitrate salts are particularly preferred for the preparation of a pharmaceutical composition, in which case the best quality and most stable product is obtained in excellent yields. Monocitrate and dicitrate salts are poorly hygroscopic, do not show deliquescence, their physical and chemical stability, as well as solubility are excellent.

Both citrate salts have a higher melting point than the dihydrochloride salt. It the case of monocitrate, approx. a 15 °C, while in the case of dicitrate, approx. a 30 °C of melting point increase can be observed which indicates greater stability and is more advantageous for the preparation of a pharmaceutical composition. The monocitrate salt is stable under normal laboratory conditions in the form of monohydrate (monocitrate Form A), but by increasing the temperature from room temperature to approx. 70 to 90°C it loses weakly bound structural water and converts to anhydrate form (monocitrate Form B). The dried sample also takes up its stoichiometric water content relatively quickly when interacting with ambient humidity. The dicitrate salt is stable in the form of anhydrate, does not convert to hydrate form, and has in a development view a favorable, sufficiently high melting point.

Comparison of the dynamic vapor sorption curves measured at 25°C of the investigated salts of 1-[4-(4-{3-[(2R)-2-methyl-pyrrolidin-1-yl]-propoxy}-phenoxy)-piperidin-1-yl]-ethanone is depicted on Figure 13 that shows the relative weight change (~ percentage change in weight relative to a weight at 0% relative humidity) as a function of relative humidity (RH%).

On the sorption curve of monohydrochloride salt Form A (Figure 2), about 3% of water is bound up to 40% RH and then liquefies at higher humidity (95% RH - relative weight change: 97%).

On the sorption curve of monohydrochloride salt Form B (Figure 6), only 0.3% of water is bound up to 30% RH, presumably by surface adsorption, and then liquefies at higher humidity (95% RH - relative weight change: 61%).

On the sorption curve of the dihydrochloride salt (Figure 12), about 0.7% of water is bound up to 60% RH relative to the dried mass, and then liquefies at higher humidity (at 70% RH already shows 17% relative weight gain, at 90% RH the relative weight change is 63%). In the case of the DVS measurement of the dihydrochloride salt, unlike the general method description, no measurements were made in the measurement cycle at 5% and 95% relative humidity, but this difference does not significantly affect the determination of the onset of deliquescence (see below).

On the sorption curve of the dihydrobromide salt (Figure 29), it absorbs about 6% moisture up to 70% RH and then liquefies at higher humidity (95% RH - relative weight change: 90%).

On the sorption curve of the sulfate salt (Figure 33), about 5% of water is bound up to 80% RH and then liquefies at higher humidity (95% RH - relative weight change: 53%).

On the the sorption curve of the oxalate salt (Figure 37), a relative weight gain of about 4.7% relative to the dried weight is observed in the 10-50% RH range, which seems to be constant up to approx. 70% RH. This weight change refers to the formation of a hydrate form having a stoichiometry nearly that of a monohydrate. Further water-take up begins above 80% RH, but the oxalate salt does not even show deliquescence above 90% RH under test conditions. In the desorption cycle between 20 to 70% RH, it stabilizes at 7.1 to 7.3% relative weight, indicating the formation of stoichiometry corresponding to the dihydrate form. Thus, the oxalate salt stabilizes in the form of a hydrate having different compositions depending on the humidity.

On the DVS curves of the monocitrate salt (Figure 21) the sample's monohydrate (Form A) and anhydrate (Form B) states can be well isolated. Above 30% RH it takes up 2.6 to 4.3% of water relative to the dried state of the substance, which is close to the theoretical calculated value of monocitrate monohydrate (3.2%). The monohydrate form has proved to be so stable that during desorption the monohydrate Form A is converted to the anhydrate Form B only below 10% RH. The monocitrate salt did not show deliquescence even above 90% RH.

On the sorption curve of the dicitrate salt (Figure 25), 3.2% of water is bound up to 80% RH, 6.8% up to 90% RH, does not show deliquescence above 90% RH, and its weight reversibly decreases during the desorption phase.

The generally observed hygroscopic nature of the 1-[4-(4-{3-[(2R)-2-methyl-pyrrolidin-1-yl]-propoxy}-phenoxy)-piperidin-1-yl]-ethanone salts is *inter alia* related to the good solubility thereof. In simulated gastric fluid (SGF without pepsin, pH = 1.3), the dihydrochloride salt has a solubility of greater than 59 mM, the solubility of the monocitrate salt is greater than 44 mM, and the solubility of the dicitrate salt is 469 mM.

The deliquescence tendency of each salt is characterized by the critical relative humidity (CRH) value (Table 1), which was determined based on the sorption curves measured according to the measurement parameter settings below, with DVS analysis at 25°C isotherm conditions, according to the following.

Derivative of the sorption curve was formed on the sorption curve between 10 to 90% RH by determining the differences in relative weight changes relative to 10% RH change:

$$\Delta m = m2 - m1$$

where m1 and m2 are the quasi-equilibrium relative mass changes (~ percentage change in weight relative to a weight at 0% relative humidity) for the given percentage of the relative humidity of the sorption curve RH1 and RH2, and

$$\Delta RH = RH2 - RH1 = 10.$$

If the given sorption step is $\Delta m/\Delta RH \geq 0.5$, then RH1 is considered to be the critical relative humidity (CRH) value indicating the end point of the physical stability of the substance. The value thus determined is a good match with the onset of a significant monotonic weight gain observed visually on the sorption curve. Above the critical relative humidity value, it is the process of deliquescence of the substance that determines the weight gain observed on the sorption curve.

**Table 1.** Critical Relative Humidity (CRH) and Δm/ΔRH values based on DVS analysis at 25°C characterizing the deliquescence of the tested salts are.

| **Salt** | **monoHCl Form B** | **monoHCl Form A** | **diHCl** | **diHBr** | **sulfate** | **oxalate** | **mono citrate** | **dicitrate** |
|---|---|---|---|---|---|---|---|---|
| **CRH** | **30%** | **40%** | **60 %** | **70 %** | **80 %** | **not deliquescent** | | |
| **$RH_1$** | **Δm/ΔRH** | | | | | | | |
| **0%** | 0.0 | 0.0 | 0.0 | 0.0 | 0.4 | 0.0 | 0.0 | 0.0 |
| **10%** | 0.0 | 0.0 | 0.0 | 0.0 | 0.0 | 0.1 | 0.0 | 0.0 |
| **20%** | 0.0 | 0.0 | 0.0 | 0.0 | 0.0 | 0.1 | 0.2 | 0.0 |
| **30%** | **0.6** | 0.3 | 0.0 | 0.0 | 0.0 | 0.2 | 0.1 | 0.0 |
| **40%** | 0.7 | **1.1** | 0.0 | 0.1 | 0.0 | 0.1 | 0.0 | 0.0 |
| **50%** | 0.4 | 0.5 | 0.0 | 0.1 | 0.0 | 0.0 | 0.0 | 0.0 |
| **60%** | 0.6 | 0.6 | **1.7** | 0.3 | 0.0 | 0.0 | 0.0 | 0.0 |
| **70%** | 0.9 | 1.1 | 2.4 | **3.1** | 0.1 | 0.0 | 0.0 | 0.0 |
| **80%** | 1.5 | 2.6 | 2.1 | 3.3 | **1.6** | 0.1 | 0.0 | 0.1 |

Compared to monohydrochloride salts, it can be established that diHBr, sulfate salts begin to show deliquescence at significantly higher critical relative humidity, which indicates a reduced hygroscopic tendency associated with their greater physical stability. Surprisingly, the oxalate, monocitrate, and dicitrate salts are not deliquescent under the conditions of the DVS analysis, and are the physically most stable ones.

Increased stability to ambient humidity is beneficial for longer-term physical and chemical stability of the active ingredient. The relationship between the reduced hygroscopic nature and the increased chemical stability associated with it is shown in the most preferred citrate salts in comparison to the dihydrochloride salt.

Table 2 shows the HPLC purity test results of a 10-day solid stress stability study of dihydrochloride, monocitrate and dicitrate salts. It is clear from the results that the dihydrochloride salt is slightly degraded by heat while it degrades significantly under the combined effect of heat and humidity. In contrast, the monocitrate and dicitrate salts are stable under these conditions and are significantly more advantageous.

**Table 2.** HPLC purity test results of a 10-day solid stress stability study of the dihydrochloride, monocitrate and dicitrate salts

| | Condition | | | | |
|---|---|---|---|---|---|
| **Storage condition** | **starting sample** | **40 °C, 75%RH** | **50°C, 75%RH** | **50°C** | **75°C** |
| | | **open glass** | **open glass** | **sealed glass** | **sealed glass** |
| **Salt form** | All contaminant (area%) | | | | |
| **dihydrochloride** | 0.85% | 59.56% | 51.32% | 0.98% | 1.50% |
| **monocitrate** | 0.19% | 0.19% | 0.19% | 0.20% | 0.19% |
| **dicitrate** | 0.23% | 0.24% | 0.25% | 0.23% | 0.24 % |

**Table** 3. X-ray powder diffraction characteristics of the 1-[4-(4-{3-[(2R)-2-methyl-pyrrolidin-1-yl]-propoxy} -phenoxy)-piperidin-1-yl]-ethanone crystalline salts and polymorphs thereof

| dihydrochloride | | |
|---|---|---|
| **Peak position [°2Th.]** | **d-spacing [Ä]** | **Rel. int. [%]** |
| 2.8 | 31.5 | 20 |
| 14.2 | 6.3 | 6 |
| 15.2 | 5.8 | 98 |
| 15.8 | 5.6 | 67 |
| 16.8 | 5.3 | 57 |
| 17.0 | 5.2 | 36 |
| 17.7 | 5.0 | 9 |
| 18.8 | 4.7 | 12 |
| 19.7 | 4.5 | 7 |
| 20.3 | 4.4 | 15 |
| 22.0 | 4.0 | 37 |
| 25.6 | 3.5 | 100 |
| 26.1 | 3.4 | 15 |
| 28.7 | 3.1 | 19 |
| 30.7 | 2.9 | 9 |
| 31.2 | 2.9 | 11 |
| **monohydrochloride Form A** | | |
| **Peak position [°2Th.]** | **d-spacing [Ä]** | **Rel. int. [%]** |
| 6.0 | 14.7 | 15 |
| 12.1 | 7.3 | 13 |
| 14.4 | 6.2 | 9 |
| 15.0 | 5.9 | 9 |
| 15.3 | 5.8 | 44 |
| 16.1 | 5.5 | 100 |
| 16.7 | 5.3 | 31 |
| 17.6 | 5.0 | 8 |
| 18.1 | 4.9 | 10 |
| 18.6 | 4.8 | 39 |
| 19.2 | 4.6 | 39 |
| 20.8 | 4.3 | 52 |
| 21.2 | 4.2 | 16 |
| 21.5 | 4.1 | 13 |
| 22.5 | 4.0 | 29 |

(continued)

| monohydrochloride Form A | | |
|---|---|---|
| Peak position [°2Th.] | d-spacing [Ä] | Rel. int. [%] |
| 22.6 | 3.9 | 24 |
| 23.0 | 3.9 | 11 |
| 24.0 | 3.7 | 26 |
| 25.5 | 3.5 | 12 |
| 25.7 | 3.5 | 15 |
| 27.0 | 3.3 | 24 |
| 27.7 | 3.2 | 19 |
| 30.2 | 3.0 | 8 |
| 30.9 | 2.9 | 7 |
| **monohydrochloride Form B** | | |
| **Peak position [°2Th.]** | **d-spacing [Ä]** | **Rel. int. [%]** |
| 15.3 | 5.8 | 91 |
| 15.6 | 5.7 | 50 |
| 16.0 | 5.5 | 100 |
| 16.3 | 5.5 | 47 |
| 16.8 | 5.3 | 42 |
| 17.3 | 5.1 | 20 |
| 17.9 | 5.0 | 21 |
| 18.1 | 4.9 | 25 |
| 20.0 | 4.4 | 20 |
| 21.3 | 4.2 | 49 |
| 22.1 | 4.0 | 8 |
| 23.3 | 3.8 | 8 |
| 24.5 | 3.6 | 10 |
| 25.9 | 3.4 | 71 |
| 27.0 | 3.3 | 12 |
| 27.7 | 3.2 | 13 |
| 28.1 | 3.2 | 7 |
| 28.5 | 3.1 | 11 |
| 30.0 | 3.0 | 6 |
| 30.6 | 2.9 | 9 |
| **dicitrate** | | |
| **Peak position [°2Th.]** | **d-spacing [Ä]** | **Rel. int. [%]** |
| 10.1 | 8.7 | 40 |
| 12.0 | 7.4 | 100 |
| 12.8 | 6.9 | 39 |
| 14.1 | 6.3 | 28 |
| 15.9 | 5.6 | 24 |
| 16.8 | 5.3 | 12 |
| 17.1 | 5.2 | 32 |
| 17.9 | 5.0 | 11 |
| 18.2 | 4.9 | 17 |
| 19.0 | 4.7 | 58 |
| 19.3 | 4.6 | 45 |
| 19.6 | 4.5 | 52 |
| 20.2 | 4.4 | 13 |

(continued)

| dicitrate | | |
|---|---|---|
| **Peak position [°2Th.]** | **d-spacing [Ä]** | **Rel. int. [%]** |
| 20.5 | 4.3 | 66 |
| 21.1 | 4.2 | 21 |
| 22.1 | 4.0 | 8 |
| 22.8 | 3.9 | 41 |
| 23.5 | 3.8 | 16 |
| 24.1 | 3.7 | 11 |
| 25.9 | 3.4 | 9 |
| 26.2 | 3.4 | 13 |
| 27.0 | 3.3 | 15 |
| 27.4 | 3.3 | 11 |
| 28.8 | 3.1 | 8 |
| 30.4 | 2.9 | 8 |
| **monocitrate Form A** | | |
| **Peak position [°2Th.]** | **d-spacing [Ä]** | **Rel. int. [%]** |
| 3.4 | 26.2 | 14 |
| 9.4 | 9.4 | 35 |
| 10.2 | 8.7 | 6 |
| 11.1 | 8.0 | 54 |
| 11.8 | 7.5 | 12 |
| 12.1 | 7.3 | 23 |
| 13.5 | 6.6 | 59 |
| 15.9 | 5.6 | 20 |
| 16.2 | 5.5 | 13 |
| 16.6 | 5.3 | 25 |
| 18.0 | 4.9 | 47 |
| 18.8 | 4.7 | 32 |
| 19.5 | 4.5 | 100 |
| 19.8 | 4.5 | 82 |
| 20.3 | 4.4 | 34 |
| 21.5 | 4.1 | 58 |
| 22.3 | 4.0 | 8 |
| 24.3 | 3.7 | 22 |
| 24.6 | 3.6 | 22 |
| 25.8 | 3.5 | 18 |
| 26.5 | 3.4 | 8 |
| 26.6 | 3.3 | 10 |
| 27.9 | 3.2 | 12 |
| 30.3 | 3.0 | 15 |
| 32.3 | 2.8 | 11 |
| 33.4 | 2.7 | 8 |
| **monocitrate Form B** | | |
| **Peak position [°2Th.]** | **d-spacing [Ä]** | **Rel. int. [%]** |
| 3.4 | 25.5 | 18 |
| 9.5 | 9.3 | 27 |
| 10.4 | 8.5 | 7 |
| 11.3 | 7.8 | 38 |

(continued)

| monocitrate Form B | | |
|---|---|---|
| Peak position [°2Th.] | d-spacing [Ä] | Rel. int. [%] |
| 11.9 | 7.5 | 14 |
| 12.2 | 7.2 | 11 |
| 13.4 | 6.6 | 12 |
| 13.7 | 6.4 | 28 |
| 14.0 | 6.3 | 28 |
| 15.5 | 5.7 | 13 |
| 15.8 | 5.6 | 17 |
| 16.4 | 5.4 | 20 |
| 17.8 | 5.0 | 32 |
| 18.1 | 4.9 | 13 |
| 19.1 | 4.7 | 100 |
| 19.5 | 4.6 | 27 |
| 19.9 | 4.5 | 35 |
| 20.4 | 4.3 | 48 |
| 21.0 | 4.2 | 7 |
| 21.9 | 4.1 | 10 |
| 22.3 | 4.0 | 38 |
| 22.7 | 3.9 | 12 |
| 24.4 | 3.6 | 20 |
| 25.1 | 3.6 | 17 |
| 26.0 | 3.4 | 17 |
| 28.3 | 3.2 | 10 |
| 31.2 | 2.9 | 8 |
| 32.4 | 2.8 | 5 |
| dihydrobromide | | |
| Peak position [°2Th.] | d-spacing [Ä] | Rel. int. [%] |
| 2.8 | 31.6 | 18 |
| 5.6 | 15.7 | 17 |
| 8.4 | 10.5 | 8 |
| 11.3 | 7.8 | 24 |
| 14.1 | 6.3 | 7 |
| 14.9 | 6.0 | 23 |
| 15.4 | 5.8 | 17 |
| 16.4 | 5.4 | 33 |
| 16.7 | 5.3 | 63 |
| 17.0 | 5.2 | 100 |
| 17.7 | 5.0 | 17 |
| 19.5 | 4.5 | 9 |
| 20.2 | 4.4 | 22 |
| 21.6 | 4.1 | 7 |
| 22.0 | 4.0 | 33 |
| 23.7 | 3.8 | 11 |
| 24.7 | 3.6 | 88 |
| 26.0 | 3.4 | 35 |
| 27.2 | 3.3 | 7 |
| 28.5 | 3.1 | 38 |
| 29.0 | 3.1 | 10 |

(continued)

| dihydrobromide | | |
|---|---|---|
| Peak position [°2Th.] | d-spacing [Ä] | Rel. int. [%] |
| 29.9 | 3.0 | 21 |
| 30.4 | 2.9 | 7 |
| 31.0 | 2.9 | 11 |
| 32.1 | 2.8 | 7 |
| **oxalate** | | |
| **Peak position [°2Th.]** | **d-spacing [Ä]** | **Rel. int. [%]** |
| 4.1 | 21.4 | 8 |
| 8.5 | 10.4 | 44 |
| 9.3 | 9.5 | 11 |
| 14.6 | 6.1 | 13 |
| 14.8 | 6.0 | 23 |
| 15.4 | 5.8 | 30 |
| 16.5 | 5.4 | 24 |
| 16.6 | 5.3 | 22 |
| 17.4 | 5.1 | 30 |
| 18.7 | 4.7 | 13 |
| 20.8 | 4.3 | 100 |
| 21.6 | 4.1 | 12 |
| 22.3 | 4.0 | 29 |
| 22.5 | 3.9 | 50 |
| 23.6 | 3.8 | 23 |
| 26.7 | 3.3 | 14 |
| 27.4 | 3.3 | 14 |
| 29.0 | 3.1 | 37 |
| 37.0 | 2.4 | 8 |
| **sulfate** | | |
| **Peak position** | **d-spacing** | **Rel. int.** |
| **[°2Th.]** | **[Ä]** | **[%]** |
| 7.8 | 11.4 | 5 |
| 11.4 | 7.7 | 8 |
| 11.7 | 7.6 | 10 |
| 12.6 | 7.0 | 6 |
| 13.4 | 6.6 | 3 |
| 14.2 | 6.2 | 2 |
| 14.7 | 6.0 | 6 |
| 15.6 | 5.7 | 19 |
| 16.1 | 5.5 | 3 |
| 16.5 | 5.4 | 18 |
| 17.1 | 5.2 | 9 |
| 17.7 | 5.0 | 9 |
| 18.0 | 4.9 | 37 |
| 18.5 | 4.8 | 77 |
| 19.6 | 4.5 | 40 |
| 19.9 | 4.5 | 55 |
| 21.0 | 4.2 | 5 |
| 21.6 | 4.1 | 3 |

(continued)

| sulfate | | |
|---|---|---|
| **Peak position** | **d-spacing** | **Rel. int.** |
| **[°2Th.]** | **[Å]** | **[%]** |
| 22.5 | 4.0 | 11 |
| 22.9 | 3.9 | 100 |
| 23.9 | 3.7 | 9 |
| 24.4 | 3.6 | 4 |
| 25.2 | 3.5 | 12 |
| 25.5 | 3.5 | 8 |
| 27.5 | 3.2 | 13 |
| 28.3 | 3.2 | 12 |
| 28.7 | 3.1 | 4 |
| 30.4 | 2.9 | 3 |
| 32.8 | 2.7 | 3 |

**Table** 4. Characteristic peaks measured in IR spectra of the 1-[4-(4-{3-[(2R)-2-methyl-pyrrolidin-1-yl]-propoxy} -phenoxy)-piperidin-1-yl]-ethanone crystalline salts and polymorphs thereof [$cm^{-1}$].

| Peaks | **Dicitrate** | **Monocitrate** Form A | **Monocitrate** Form B | **Oxalate** | **Sulfate** | **diHCl** | **HCl** Form B | **diHBr** |
|---|---|---|---|---|---|---|---|---|
| 1 | 3523 | 3466 | 2962 | 3431 | 3389 | 3364 | 3429 | 3419 |
| 2 | 3038 | 3011 | 2872 | 2938 | 2955 | 3044 | 2953 | 3043 |
| 3 | 2951 | 2962 | 1732 | 2881 | 2615 | 2959 | 2695 | 2958 |
| 4 | 2521 | 2859 | 1637 | 2513 | 2513 | 2931 | 1621 | 2930 |
| 5 | 1966 | 1731 | 1595 | 1987 | 1590 | 2903 | 1507 | 2901 |
| 6 | 1727 | 1618 | 1508 | 1706 | 1507 | 2874 | 1456 | 2843 |
| 7 | 1687 | 1594 | 1477 | 1693 | 1487 | 2604 | 1392 | 2614 |
| 8 | 1587 | 1507 | 1452 | 1624 | 1453 | 2519 | 1366 | 2520 |
| 9 | 1507 | 1479 | 1398 | 1507 | 1374 | 2184 | 1271 | 1840 |
| 10 | 1489 | 1454 | 1367 | 1470 | 1360 | 1773 | 1218 | 1685 |
| 11 | 1475 | 1394 | 1316 | 1455 | 1269 | 1682 | 1131 | 1616 |
| 12 | 1426 | 1318 | 1288 | 1367 | 1220 | 1616 | 1118 | 1508 |
| 13 | 1394 | 1285 | 1271 | 1220 | 1112 | 1508 | 1038 | 1469 |
| 14 | 1358 | 1270 | 1217 | 1134 | 1044 | 1468 | 998 | 1441 |
| 15 | 1315 | 1217 | 1172 | 1044 | 1010 | 1441 | 970 | 1413 |
| 16 | 1276 | 1175 | 1131 | 1008 | 975 | 1422 | 829 | 1337 |
| 17 | 1216 | 1133 | 1068 | 976 | 924 | 1337 | 781 | 1284 |
| 18 | 1130 | 1043 | 1043 | 948 | 855 | 1285 | 748 | 1232 |
| 19 | 1116 | 1004 | 1000 | 832 | 816 | 1232 | 595 | 1133 |
| 20 | 1062 | 974 | 975 | 805 | 777 | 1162 | 527 | 1117 |
| 21 | 1030 | 937 | 951 | 752 | 722 | 1118 | | 1091 |
| 22 | 1000 | 908 | 908 | 722 | 646 | 1092 | | 1074 |
| 23 | 962 | 847 | 818 | 648 | 624 | 1075 | | 1053 |
| 24 | 948 | 806 | 746 | 593 | 591 | 1053 | | 1033 |
| 25 | 933 | 771 | 664 | 478 | 516 | 1030 | | 1005 |
| 26 | 872 | | | | | 1006 | | 938 |
| 27 | 825 | | | | | 975 | | 949 |
| 28 | 805 | | | | | 939 | | 938 |
| 29 | 782 | | | | | 918 | | 817 |

(continued)

| Peaks | **Dicitrate** | **Monocitrate** Form A | **Monocitrate** Form B | **Oxalate** | **Sulfate** | **diHCl** | **HCl** Form B | **diHBr** |
|---|---|---|---|---|---|---|---|---|
| 30 | 736 | | | | | 897 | | 755 |
| 31 | 695 | | | | | 818 | | 718 |
| 32 | 661 | | | | | 754 | | 670 |
| 33 | 606 | | | | | 722 | | 584 |
| 34 | 588 | | | | | 671 | | 571 |
| 35 | 497 | | | | | 576 | | 516 |
| 36 | 477 | | | | | 516 | | 495 |
| 37 | 443 | | | | | 496 | | |

**Table 5.** Characteristic peaks measured in Raman spectra of the 1-[4-(4-{3-[(2R)-2-methylpyrrolidin-1-yl]-propoxy}-phenoxy)-piperidin-1-yl]-ethanone crystalline salts and polymorphs thereof [cm-1].

| Peaks | **Dicitrate** | **Monocitrate** Form A | **Monocitrate** Form B | **Oxalate** | **Sulfate** | **diHCl** | **HCl Form B** | **diHBr** |
|---|---|---|---|---|---|---|---|---|
| 1 | 3070 | 3065 | 3086 | 3077 | 3076 | 3073 | 3074 | 3072 |
| 2 | 3011 | 3012 | 3011 | 3033 | 3059 | 3048 | 3051 | 3045 |
| 3 | 2971 | 2979 | 2982 | 2984 | 2992 | 3023 | 2981 | 3022 |
| 4 | 2933 | 2961 | 2961 | 2935 | 2935 | 2958 | 2932 | 2983 |
| 5 | 1719 | 2931 | 2930 | 2883 | 2907 | 2935 | 2875 | 2958 |
| 6 | 1611 | 2905 | 2881 | 2757 | 2893 | 2878 | 2767 | 2935 |
| 7 | 1585 | 2888 | 2853 | 1733 | 1613 | 2766 | 1615 | 2902 |
| 8 | 1489 | 2849 | 1718 | 1707 | 1584 | 1662 | 1583 | 2876 |
| 9 | 1457 | 1715 | 1628 | 1611 | 1487 | 1614 | 1469 | 1687 |
| 10 | 1394 | 1618 | 1615 | 1477 | 1453 | 1583 | 1442 | 1613 |
| 11 | 1274 | 1603 | 1586 | 1443 | 1378 | 1468 | 1362 | 1583 |
| 12 | 1256 | 1585 | 1476 | 1364 | 1360 | 1442 | 1311 | 1469 |
| 13 | 1191 | 1480 | 1454 | 1305 | 1304 | 1325 | 1257 | 1442 |
| 14 | 1158 | 1457 | 1365 | 1268 | 1257 | 1312 | 1164 | 1265 |
| 15 | 1132 | 1438 | 1307 | 1246 | 1172 | 1266 | 1133 | 1198 |
| 16 | 1053 | 1370 | 1251 | 1203 | 1152 | 1198 | 1095 | 1164 |
| 17 | 1031 | 1304 | 1169 | 1178 | 1133 | 1163 | 1041 | 1034 |
| 18 | 1000 | 1283 | 1134 | 1157 | 1106 | 1093 | 1005 | 910 |
| 19 | 936 | 1252 | 1043 | 1137 | 1053 | 1068 | 910 | 852 |
| 20 | 910 | 1242 | 999 | 1108 | 1011 | 1034 | 886 | 843 |
| 21 | 854 | 1169 | 930 | 1068 | 961 | 911 | 853 | 719 |
| 22 | 782 | 1134 | 857 | 1044 | 926 | 853 | 837 | 671 |
| 23 | 720 | 1053 | 718 | 1009 | 854 | 809 | 724 | 638 |
| 24 | 660 | 1039 | 665 | 981 | 821 | 722 | 668 | 519 |
| 25 | 640 | 1006 | 647 | 958 | 792 | 706 | 638 | 378 |
| 26 | 607 | 950 | 602 | 928 | 723 | 672 | 518 | 319 |
| 27 | 516 | 935 | 513 | 858 | 706 | 638 | 464 | 261 |
| 28 | 460 | 859 | 380 | 842 | 644 | 517 | 413 | |
| 29 | 371 | 840 | 308 | 807 | 625 | 496 | 378 | |
| 30 | 314 | 804 | 253 | 745 | 581 | 466 | | |
| 31 | 257 | 722 | | 725 | 519 | 418 | | |
| 32 | | 665 | | 708 | 482 | 379 | | |
| 33 | | 642 | | 648 | 469 | 299 | | |
| 34 | | 619 | | 621 | 420 | | | |

(continued)

| Peaks | **Dicitrate** | **Monocitrate** Form A | **Monocitrate** Form B | **Oxalate** | **Sulfate** | **diHCl** | **HCl Form B** | **diHBr** |
|---|---|---|---|---|---|---|---|---|
| 35 | | 515 | | 514 | 381 | | | |
| 36 | | 470 | | 489 | | | | |
| 37 | | 389 | | 456 | | | | |
| 38 | | 309 | | 428 | | | | |
| 39 | | 265 | | 382 | | | | |
| 40 | | | | 329 | | | | |
| 41 | | | | 298 | | | | |
| 42 | | | | 222 | | | | |

For solid phase analytical studies, the following experimental conditions were used:

Parameters of **FT-IR spectroscopy** measurements:

| | |
|---|---|
| Device | Thermo-Nicolet 6700 |
| Phase | KBr pastille |
| Spectral resolution | 4 $cm^{-1}$ |
| Detector | DTGS |
| Beamsplitter | XT-KBr |
| Mirror movement speed | 0.6329 |
| Number of scans | 100 |

Parameters of **FT-Raman spectroscopy** measurements:

| | |
|---|---|
| Device | Thermo-Nicolet NXR9650 |
| Measurement range | 3500 to 200 $cm^{-1}$ |
| Spectral resolution | 4 $cm^{-1}$ |
| Detector | Ge |
| Beamsplitter | $CaF_2$ |
| Mirror movement speed | 0.1581 |
| Number of scans | 256 |
| Laser performance | 500 mW |

Parameters of **X-ray powder diffraction** measurements:

| | |
|---|---|
| Device | PANanalytical X'Pert PRO MPD |
| Radiation | CuKa |
| Accelerating voltage | 40 kV |
| Anode current | 40 mA |
| Goniometer | PW3050/60 |
| Scanning speed | 0.0305 °/s |
| Increment | 0.0131 ° |
| Sample holder | PW1818/25 & 40 (transmission, sample between foils) |
| Sample holder spinner | PW3064/60 (reflection/transmission spinner) |

(continued)

| | |
|---|---|
| Spinning speed of sample holder | 1 spin/s |
| Detector | PIXcel (PW3018/00) |
| The uncertainty of the 2θ measurement | ± 0.2 ° |

Parameters **of TG** measurements:

| | |
|---|---|
| Device | TA Instruments TGA Q5000 or Discovery TGA 5500 |
| Heating speed | 10 °C/min |
| Sample weight | ~ 5 to 10 mg |
| Atmosphere | 60 mL/min $N_2$ |

Parameters of **DSC** measurements:

| | |
|---|---|
| Device | TA Instruments DSC Q1000 or Discovery DSC 2500 |
| Heating speed | 10 °C/min |
| Sample weight | ~ 1 to 2 mg |
| Type of jar | open Al jar |
| Atmosphere | 50 mL/min $N_2$ |

Parameters of **DVS** measurements:

| | |
|---|---|
| Device | SMS DVS Advantage 1 |
| dm/dt criteria | 0.002 %/min |
| time limit max./min. | 360 min / 10 min |
| Temperature | 25 °C |
| Cycle | 0-5-10-20-30-40-50-60-70-80-90-95-90-80-70-60-50-40-30-20-10-5-0 %RH |
| Gas flow | 150 mL/min $N_2$ |
| Solvent | water |

**Pharmaceutical compositions**

The salts of the present invention may be administered in any pharmaceutically acceptable manner, for example, orally, parenterally, buccally, sublingually, nasally, rectally or transdermally, appropriately to the formulation of the pharmaceutical composition. The therapeutically effective dose is between 0.01 and 40 mg/day.

The following formulation examples illustrate the pharmaceutical compositions of the present invention. However, the present invention is not limited to these compositions.

A) Solid oral dosage form

Tablet

| | |
|---|---|
| Active ingredient(s) | 0.005 - 90% |
| Filler | 1 - 99.9% |
| Binder | 0 - 20% |
| Desintegrant | 0 - 20% |
| Lubricant | 0 - 10% |
| Other specific excipient(s) | 0 - 50% |

B) Parenteral dosage form
Intravenouss injection

| | |
|---|---|
| Active ingredient(s) | 0.001 - 50% |
| Solvent | 10 - 99.9% |
| Co-solvent | 0 - 99.9% |
| Osmotic agent | 0 - 50% |
| Buffer | q.s. |

C) Other dosage form
Suppository

| | |
|---|---|
| Active ingredient(s) | 0.0003 - 50% |
| Suppository base | 1 - 99.9% |
| Surface-active agents | 0 - 20% |
| Lubricant | 0 - 20% |
| Preservative | q.s. |

## Examples

The invention is illustrated by the following Reference and working Examples without limiting the scope of the present invention.

### Reference Examples

#### Example 1

1-[4-(4-{3-[(2R)-2-methyl-pyrrolidin-1-yl]-propoxy}-phenoxy)-piperidin-1-yl]-ethanone

40 g of 1-[4-(4-{3-[(2R)-2-methyl-pyrrolidin-1-yl]-propoxy}-phenoxy)-piperidin-1-yl]-ethanone hydrochloride salt prepared according to Example 11 of WO 2014/136075 was dissolved in 480 mL of dichloromethane at 0 to 5 °C, and then 168 mL of 1M aqueous NaOH was added. After stirring for 10 minutes, the aqueous and organic phases were separated and the organic phase was washed twice with 120 mL of deionized water, dried over 25 g of natrium sulfate and filtered. The solution was concentrated in vacuo to an oil. Evaporation residue: 32.8 g of an oil.

#### Example 2

1-[4-(4-f3-[(2R)-2-methyl-pyrrolidin-1-yl]-propoxy}-phenoxy)-piperidin-1-yl]-ethanone dihydrochloride salt

2.0 g (5.55 mmol) of 1-[4-(4-{3-[(2R)-2-methyl-pyrrolidin-1-yl]-propoxy}-phenoxy)-piperidin-1-yl]-ethanone was dissolved in 20 mL of acetone at room temperature. The mixture was cooled to 0 to 5 °C and 0.8 mL of ≥37% hydrochloric acid solution was added dropwise. After stirring for 30 minutes at 0 to 5 °C, the crystals were filtered, covered with 1.5 mL of cold acetone, and dried at room temperature. White crystalline material. Yield: 1.7 g.

#### Example 3

1-[4-(4-f3-[(2R)-2-methyl-pyrrolidin-1-yl]-propoxy}-phenoxy)-piperidin-1-yl]-ethanone dihydrochloride salt

0.548 g of 1-[4-(4-{3-[(2R)-2-methyl-pyrrolidin-1-yl]-propoxy}-phenoxy)-piperidin-1-yl]-ethanone was dissolved in 1.1 mL of isopropanol at room temperature. To the solution of the base, 0.391 g of 30% hydrochloric acid isopropanol was added dropwise at room temperature. The precipitated slurry was filtered, and then dried for 2 hours under vacuum under nitrogen at 40 °C. Yield: 0.42g.

**Example 4**

1-[4-(4-{3-[(2R)-2-methyl-pyrrolidin-1-yl]-propoxy}-phenoxy)-piperidin-1-yl]-ethanone monohydrochloride salt Form A

11.831 mg of 1-[4-(4-{3-[(2R)-2-methyl-pyrrolidin-1-yl]-propoxy}-phenoxy)-piperidin-1-yl]-ethanone dihydrochloride salt was weighed in platinum jar and heat treated in TA Instruments TGA Q50 device until elimination of 1 mol of HCl, according to the following program:

1. Heating up to 90 °C with 10 °C/min heating rate
2. Hold at 90 °C for 103.7 minutes
3. Heating up to 95 °C with 10 °C/min heating rate
4. Hold at 95 °C for 12.9 minutes
5. Heating up to 100 °C at 10 °C/min heating rate
6. Hold at 100 °C for 180 minutes.

**Example 5**

1-[4-(4-{3-[(2R)-2-methyl-pyrrolidin-1-yl]-propoxy}-phenoxy)-piperidin-1-yl]-ethanone monohydrochloride salt Form A

0.4 mL of aqueous sodium bicarbonate solution (97.5 mg NaHCOs/1 mL $H_2O$) was added to 0.2 g of 1-[4-(4-{3-[(2R)-2-methyl-pyrrolidin-1-yl]-propoxy}-phenoxy)-piperidin-1-yl]-ethanone dihydrochloride salt. With the equimolar base used, 1 mol of HCl was liberated during the effervescence of the solution. 1 mL of 1,4-dioxane was added to the solution and an oil was obtained after evaporation. 20 to 30 mg of oil were mixed with 0.5 mL of methyl ethyl ketone, filtered and precipitated with 0.5 mL of diisopropyl ether to give an oil. It was seeded with the 1-[4-(4-{3-[(2R)-2-methyl-pyrrolidin-1-yl]-propoxy}-phenoxy)-piperidin-1-yl]-ethanone monohydrochloride salt obtained by thermal treatment in Example 4. After crystallization, the product was filtered and dried at room temperature. Yield: 27 mg.

**Example 6**

1-[4-(4-{3-[(2R)-2-methyl-pyrrolidin-1-yl]-propoxy}-phenoxy)-piperidin-1-yl]-ethanone monohydrochloride salt Form A

0.1 g of 1-[4-(4-{3-[(2R)-2-methyl-pyrrolidin-1-yl]-propoxy}-phenoxy)-piperidin-1-yl]-ethanone dihydrochloride salt was dissolved in 0.2 mL of deionized water and 0.2 mL of aqueous sodium bicarbonate solution (97.5 mg of $NaHCO_3$/1 mL of $H_2O$) was added. The resulting solution was concentrated at 50 °C and at 70 mbar, then dissolved in 5 mL of methyl ethyl ketone, filtered and washed with 1 mL of methyl ethyl ketone. To the solution 11.5 mL of diisopropyl ether was added and seeded with the product of Example 4, an oily precipitation was observed. The solution was concentrated to dryness, the "residue" was dissolved in 1 mL of dimethylformamide and 15 mL of methyl tert-butyl ether was added and then seeded with the product of Example 5. The next day, the precipitated crystalline product was isolated by filtration. Yield: 25 mg.

**Example 7**

1-[4-(4-{3-[(2R)-2-methyl-pyrrolidin-1-yl]-propoxy}-phenoxy)-piperidin-1-yl]-ethanone monohydrochloride salt Form B

2.0 g of 1-[4-(4-{3-[(2R)-2-methyl-pyrrolidin-1-yl]-propoxy}-phenoxy)-piperidin-1-yl]-ethanone base was dissolved in 20 mL of acetone at room temperature. The mixture was cooled to 0 to 5 °C and 0.4 mL of ≥37% hydrochloric acid solution was added dropwise. After 30 minutes of stirring at 0 to 5 °C it was concentrated to constant weight in a water bath at 40 °C under vacuum. Then, twice 30 mL of toluene was evaporated. White crystalline material. Yield: 1.5 g.

**Example 8**

1-[4-(4-{3-[(2R)-2-methyl-pyrrolidin-1-yl]-propoxy}-phenoxy)-piperidin-1-yl]-ethanone monohydrochloride salt Form B

0.548 g of 1-[4-(4-{3-[(2R)-2-methyl-pyrrolidin-1-yl]-propoxy}-phenoxy)-piperidin-1-yl]-ethanone base was dissolved in 0.55 mL of methyl tert-butyl ether at room temperature. Slowly, 0.18 g of 30% hydrochloric acid isopropanol was added dropwise at room temperature to the solution of the base. The initially biphasic mixture became miscible with stirring and then converted to a thick crystalline suspension. The precipitated suspension was filtered and dried for 2 hours under vacuum under nitrogen at 40 °C. Yield: 0.33 g.

**Working examples**

**Example 9**

1-[4-(4-{3-[(2R)-2-methyl-pyrrolidin-1-yl]-propoxy}-phenoxy)-piperidin-1-yl]-ethanone dihydrobromide salt

0.53 g of 1-[4-(4-{3-[(2R)-2-methyl-pyrrolidin-1-yl]-propoxy}-phenoxy)-piperidin-1-yl]-ethanone base was dissolved in 5 mL of ethyl acetate at room temperature, followed by the addition of a solution of acetic acid saturated with 0.8 mL of hydrobromic acid the salt was formed. After filtration it was washed twice with 1 mL of acetic acid saturated with hydrobromic acid. The dried product weighed 0.65 g.

**Example 10**

1-[4-(4-{3-[(2R)-2-methyl-pyrrolidin-1-yl]-propoxy}-phenoxy)-piperidin-1-yl]-ethanone sulfate salt

0.1 g of 1-[4-(4-{3-[(2R)-2-methyl-pyrrolidin-1-yl]-propoxy}-phenoxy)-piperidin-1-yl]-ethanone base oil was dissolved in 9 mL of acetone at room temperature and then 0.125 mL of 20.4% $H_2SO_4$ solution was slowly added dropwise. The resulting solution first became opalescent, then a crystalline suspension was obtained which was stirred at room temperature for 2 hours. The product was filtered and washed twice with 0.5 mL of acetone. It was dried under vacuum at 40 °C for 2 hours under nitrogen. Yield: 0.08 g.

**Example 11**

1-[4-(4-{3-[(2R)-2-methyl-pyrrolidin-1-yl]-propoxy}-phenoxy)-piperidin-1-yl]-ethanone sulfate salt

0.99 g of 1-[4-(4-{3-[(2R)-2-methyl-pyrrolidin-1-yl]-propoxy}-phenoxy)-piperidin-1-yl]-ethanone base oil was dissolved in 20 mL of acetone, then 1.3 mL of 18.4% $H_2SO_4$ solution was added. The mixture was seeded with the product of Example 10 (with the addition of 0.05 mL of water). The product was precipitated with 20 mL of acetone, stirred for half an hour, filtered, washed and dried at 40 °C under nitrogen. Yield: 0.814 g. Melting point of the product (based on DSC peak): 79.5 °C.

**Example 12**

1-[4-(4-{3-[(2R)-2-methyl-pyrrolidin-1-yl]-propoxy}-phenoxy)-piperidin-1-yl]-ethanone sulfate salt

1.008 g of 1-[4-(4-{3-[(2R)-2-methyl-pyrrolidin-1-yl]-propoxy}-phenoxy)-piperidin-1-yl]-ethanone base oil was mixed with 0.935 mL of 3M $H_2SO_4$ and stirred for 15 minutes. 20 mL of acetone was added and seeded with the product of Example 11 and then stirred at room temperature overnight. Filtered, dried under nitrogen at 40 °C to constant weight. Yield: 0.895g. Melting point of the product (based on DSC peak): 79.3 °C.

**Example 13**

1-[4-(4-{3-[(2R)-2-methyl-pyrrolidin-1-yl]-propoxy}-phenoxy)-piperidin-1-yl]-ethanone oxalate salt

0.1 g of 1-[4-(4-{3-[(2R)-2-methyl-pyrrolidin-1-yl]-propoxy}-phenoxy)-piperidin-1-yl]-ethanone base oil was dissolved in 0.1 mL of acetone at room temperature and a solution of 0.055 g of oxalic acid in 0.5 mL of acetone was added. The product was precipitated with 0.3 mL of ethyl acetate. Filtered and then dried under nitrogen to constant weight. Yield: 128 mg. Melting point of the product (based on DSC peak): 54.2 °C.

**Example 14**

1-[4-(4-{3-[(2R)-2-methyl-pyrrolidin-1-yl]-propoxy}-phenoxy)-piperidin-1-yl]-ethanone oxalate salt

0.5 g of 1-[4-(4-{3-[(2R)-2-methyl-pyrrolidin-1-yl]-propoxy}-phenoxy)-piperidin-1-yl]-ethanone base oil was dissolved 0.5 mL of acetone, and then a solution of 0.275 g of oxalic acid in 1.5 mL of acetone and 0.05 mL of water was added. The precipitated material was filtered and then stirred in a mixture of 0.05 mL of water and 2.75 mL of acetone in the presence of 0.1755 g of oxalic acid. The product obtained was filtered and dried. Yield: 392 mg.

**Example 15**

1-[4-(4-{3-[(2R)-2-methyl-pyrrolidin-1-yl]-propoxy}-phenoxy)-piperidin-1-yl]-ethanone monocitrate salt Form A

To 0.13 g of 1-[4-(4-{3-[(2R)-2-methyl-pyrrolidin-1-yl]-propoxy}-phenoxy)-piperidin-1-yl]-ethanone base oil 0.081 g of citric acid monohydrate was added at room temperature with stirring. 0.5 mL of acetone was added and stirred overnight. After the addition of further 1 mL of acetone on the following day, the mixture was stirred for an additional 30 minutes, then filtered and washed with 0.5 mL of acetone. The resulting sample was dried under vacuum under nitrogen at 25 °C. Yield: 0.163 g.

**Example 16**

1-[4-(4-{3-[(2R)-2-methyl-pyrrolidin-1-yl]-propoxy}-phenoxy)-piperidin-1-yl]-ethanone monocitrate salt Form A

To 0.513 g of 1-[4-(4-{3-[(2R)-2-methyl-pyrrolidin-1-yl]-propoxy}-phenoxy)-piperidin-1-yl]-ethanone base oil a solution of 0.315 g citric acid monohydrate in 5 mL of acetone was added at room temperature with stirring. The solution was seeded with the product of Example 15. After stirring for two hours, another 2 mL of acetone was added and stirred for a weekend. The mixture was filtered and washed with 5 mL of acetone. The resulting crystalline material was dried under vacuum under nitrogen at 25 °C. Yield 0.63 g. Karl-Fischer water content: 3.1%.

**Example 17**

1-[4-(4-{3-[(2R)-2-methyl-pyrrolidin-1-yl]-propoxy}-phenoxy)-piperidin-1-yl]-ethanone monocitrate salt Form A

1.020 g of 1-[4-(4-{3-[(2R)-2-methyl-pyrrolidin-1-yl]-propoxy}-phenoxy)-piperidin-1-yl]-ethanonebase oil was weighted to a 100 mL reactor and stirred with 15 mL of acetone at room temperature. To this solution 15 mL of a solution of citric acid monohydrate (0.872 g of citric acid monohydrate dissolved in 20 mL of acetone) was added at room temperature. In the meantime, it was seeded with a suspension of the monocitrate salt prepared in Example 16 (0.0767 g suspended in 0.5 mL of acetone). The resulting suspension was stirred at room temperature for 1 hour, then the precipitated salt was filtered and washed with 10 mL of acetone. The resulting crystalline material was dried at 25 °C under nitrogen. Yield: 1.385 g. Melting point of the product (DSC onset): 114.3 °C. Karl-Fischer water content: 3.5%.

**Example 18**

1-[4-(4-{3-[(2R)-2-methyl-pyrrolidin-1-yl]-propoxy}-phenoxy)-piperidin-1-yl]-ethanone monocitrate salt Form B

The monocitrate salt Form A of Example 17 was dried at 70 to 90 °C under nitrogen to constant weight.

**Example 19**

1-[4-(4-f3-[(2R)-2-methyl-pyrrolidin-1-yl]-propoxy}-phenoxy)-piperidin-1-yl]-ethanone dicitrate salt

0.5 g of 1-[4-(4-{3-[(2R)-2-methyl-pyrrolidin-1-yl]-propoxy}-phenoxy)-piperidin-1-yl]-ethanone base oil was dissolved in 1 mL of acetone, to which a solution of 0.962 g of citric acid monohydrate in 4 mL of acetone was added. After stirring for 1 h 15 min at reflux temperature, it was cooled to room temperature, then filtered and washed with 10 mL of acetone. The resulting sample was dried overnight at 25 °C under vacuum under nitrogen. Yield: 0.832 mg.

**Example 20**

1-[4-(4-(3-[(2R)-2-methyl-pyrrolidin-1-yl]-propoxy}-phenoxy)-piperidin-1-yl]-ethanone dicitrate salt

1.928 g of citric acid monohydrate was added to a 100 mL reactor and dissolved in 15 mL of acetone at room temperature. To this solution an acetone suspension (0.1039 g / 0.5 mL) of the dicitrate salt prepared in Example 19 was added. To this solution a solution of 15 mL of the base in acetone (1.695 g of 1-[4-(4-{3-[(2R)-2-methyl-pyrrolidin-1-yl]-propoxy}-phenoxy)-piperidin-1-yl]-ethanone base oil dissolved in 17.5 mL of acetone) was added. The solution was stirred at room temperature for 1 hour, then filtered off and washed with 10 mL of acetone. The resulting sample was dried for 1 day at 25 °C under nitrogen. Yield: 2.81g. Melting point of the product (DSC onset): 133.1 °C. Karl-Fischer water content: 0.4%.

**Example 21**

1-[4-(4-{3-[(2R)-2-methyl-pyrrolidin-1-yl]-propoxy}-phenoxy)-piperidin-1-yl]-ethanone dicitrate salt

17.6 kg of dichloromethane was introduced into the reactor and then inertized with nitrogen and the temperature was set to 0 to 5 °C. 1.1 kg of 1-[4-(4-{3-[(2R)-2-methyl-pyrrolidin-1-yl]-propoxy}-phenoxy)-piperidin-1-yl]-ethanone dihydrochloride was added, and then a mixture of 5.7 kg of purified water and 0.19 kg of NaOH was added while maintaining the temperature at 0 to 5 °C. After a reaction time of 15 to 20 minutes, the organic phase was conducted to another reactor, which was also inertized with nitrogen. 2200 mL of dichloromethane was added to the organic phase and, after stirring for 30 to 40 minutes, the organic phase was separated again. The following step was repeated twice: 3300 mL of purified water was added to the organic phase and after 30 to 40 minutes of stirring, the organic phase was separated again. A solution of 0.66 kg of NaCl in 2.6 L of purified water was added to the separated organic phase and, after stirring for 30 to 40 minutes, the organic phase was separated again. The organic phase was concentrated under 0.5 bar vacuum at max. 35 °C to the stirring limit (to 3 to 4 liters). Repeated three times, 8.8 kg acetone was added and the liberated base was concentrated under 0.7 bar vacuum at max. 45 °C to the stirring limit (to 3 to 4 liters). 1.1 kg of citric acid monohydrate was dissolved in 7.0 kg of acetone while maintaining the temperature of the solution at 20 to 25 °C. To the resulting citric acid solution 5.0 g of 1-[4-(4-{3-[(2R)-2-methyl-pyrrolidin-1-yl]-propoxy}-phenoxy)-piperidin-1-yl]-ethanone dicitrate seed crystals were added. To the resulting solution the solution of the concentrated 1-[4-(4-{3-[(2R)-2-methyl-pyrrolidin-1-yl]-propoxy}-phenoxy)-piperidin-1-yl]-ethanone base in acetone was added over 110 to 130 minutes, keeping the temperature between 20 to 25 °C. After addition, the mixture was heated to 55 to 60 °C and stirred at this temperature for 10 to 12 minutes and then cooled to 20 to 25 °C for an additional 10 hours. At the end of the stirring time, the material was centrifuged and dried. Yield: 1.398 kg. Melting point of the product (DSC onset): 132.7 °C.

**Example 22**

1-[4-(4-{3-[(2R)-2-methyl-pyrrolidin-1-yl]-propoxy}-phenoxy)-piperidin-1-yl]-ethanone dicitrate salt

70 g of 1-[4-(4-{3-[(2R)-2-methyl-pyrrolidin-1-yl]-propoxy}-phenoxy)-piperidin-1-yl]-ethanone dihydrochloride salt was weighted and dissolved in 840 mL of dichloromethane at 0 to 5 °C, followed by addition of a solution of 11.9 g of NaOH in 350 mL of deionized water. After stirring for 15 minutes, the mixture was separated and the aqueous phase was transferred to 140 mL of dichloromethane. The combined organic phase was extracted twice with 210 mL of deionized water and then with 210 mL of saturated brine. The solution was concentrated to an oil in vacuo (at max. 35 °C). The mixture was diluted three times with 700 mL of acetone and evaporated. The evaporation residue was complemented with acetone to 560 mL. The solution of 1-[4-(4-{3-[(2R)-2-methyl-pyrrolidin-1-yl]-propoxy}-phenoxy)-piperidin-1-yl]-ethanone in acetone was added to a solution of 64 g citric acid anhydrate in 560 mL of acetone and 6 mL of water (20 to 25 °C) over two hours, keeping at 20 to 25 °C. After stirring for 15 minutes at reflux, the suspension was cooled back to room temperature and after further stirring for 2 hours, the precipitate was filtered off, washed twice with 60 mL of acetone and dried at 50 °C under vacuum. Yield: 101.6 g. Melting point of the product (DSC onset): 132.6 °C.

## Claims

**1.** 1-[4-(4-{3-[(2R)-2-methyl-pyrrolidin-1-yl]-propoxy}-phenoxy)-piperidin-1-yl]-ethanone salts **characterized in that** based on the dynamic vapor sorption (DVS) analysis at 25 °C

a.) showing deliquescence only above 70% relative humidity, i.e. the critical relative humidity (CRH) value,

determined in the range of 10 to 90% RH of the sorption curve, indicating of the onset of deliquescence, is at least 70%; or

b.) not showing deliquescence, i.e. **characterized by** a value of 0.5 > $\Delta m/\Delta RH$ in the range of 10 to 90% RH of the sorption curve, wherein

$\Delta m = m_2 - m_1$, the difference of the quasi-equilibrium relative mass changes $m_2$ and $m_1$ corresponding to the $RH_2$ and $RH_1$ relative humidity values and wherein

$$\Delta RH = RH_2 - RH_1 = 10,$$

wherein the salts are selected from the group consisting of salts formed with sulfuric acid, oxalic acid and citric acid or the dihydrobromide salt.

**2.** The salts according to claim 1, wherein the salt is monocitrate salt.

**3.** The crystalline Form A of 1-[4-(4-{3-[(2R)-2-methyl-pyrrolidin-1-yl]-propoxy}-phenoxy)-piperidin-1-yl]-ethanone monocitrate salt according to claim 2 **characterized in that** characteristic reflections in the X-ray powder diffractogram thereof are present in scattering angle value ranges of 9.4; 11.1; 13.5; 18.0; 19.5; 19.8; 21.5° ± 0.2° 2θ, as measured at CuKa wavelength.

**4.** The crystalline Form A of 1-[4-(4-{3-[(2R)-2-methyl-pyrrolidin-1-yl]-propoxy}-phenoxy)-piperidin-1-yl]-ethanone monocitrate salt according to claim 2 **characterized in that** absorption bands in the infrared spectra thereof are present in value ranges of 3466; 3011; 2962; 1731; 1618; 1594; 1507; 1217; 1043; 665 $cm^{-1}$ ± 4 $cm^{-1}$.

**5.** The crystalline Form A of 1-[4-(4-{3-[(2R)-2-methyl-pyrrolidin-1-yl]-propoxy}-phenoxy)-piperidin-1-yl]-ethanone monocitrate salt according to claim 2 **characterized in that** absorption bands in the Raman spectra thereof are present in value ranges of 3065; 2961; 2931; 1715; 1618; 1480; 1242; 1134; 859; 840; 722 $cm^{-1}$ ± 4 $cm^{-1}$.

**6.** The crystalline Form B of 1-[4-(4-{3-[(2R)-2-methyl-pyrrolidin-1-yl]-propoxy}-phenoxy)-piperidin-1-yl]-ethanone monocitrate salt according to claim 2 **characterized in that** characteristic reflections in the X-ray powder diffractogram thereof are present in scattering angle value ranges of 9.5; 11.3; 13.4; 13.7; 14.0; 17.8; 19.1; 20.4; 22.3° ± 0.2° 2θ, as measured at CuKα wavelength.

**7.** The crystalline Form B of 1-[4-(4-{3-[(2R)-2-methyl-pyrrolidin-1-yl]-propoxy}-phenoxy)-piperidin-1-yl]-ethanone monocitrate salt according to claim 2 **characterized in that** absorption bands in the infrared spectra thereof are present in value ranges of 2962; 2872; 1732; 1637; 1595; 1508; 1217; 1068; 1043; 818 $cm^{-1}$ ± 4 $cm^{-1}$.

**8.** The crystalline Form B of 1-[4-(4-{3-[(2R)-2-methyl-pyrrolidin-1-yl]-propoxy}-phenoxy)-piperidin-1-yl]-ethanone monocitrate salt according to claim 2 **characterized in that** absorption bands in the Raman spectra thereof are present in value ranges of 3086; 2930; 2881; 1718; 1628; 1476; 1251; 857; 718 $cm^{-1}$ ± 4 $cm^{-1}$.

**9.** The salts according to claim 1, wherein the salt is dicitrate salt.

**10.** The crystalline form of 1-[4-(4-{3-[(2R)-2-methyl-pyrrolidin-1-yl]-propoxy}-phenoxy)-piperidin-1-yl]-ethanone dicitrate salt according to claim 9 **characterized in that** characteristic reflections in the X-ray powder diffractogram thereof are present in scattering angle value ranges of 10.2; 12.0; 12.8; 14.1; 19.0; 19.3; 20.5; 22.8° ± 0.2° 2θ, as measured at CuKa wavelength.

**11.** The crystalline form of 1-[4-(4-{3-[(2R)-2-methyl-pyrrolidin-1-yl]-propoxy}-phenoxy)-piperidin-1-yl]-ethanone dicitrate salt according to claim 9 **characterized in that** absorption bands in the infrared spectra thereof are present in value ranges of 3523; 3038; 2521; 1727; 1687; 1587; 1507; 1216; 782; 661 $cm^{-1}$ ± 4 $cm^{-1}$.

**12.** The crystalline form of 1-[4-(4-{3-[(2R)-2-methyl-pyrrolidin-1-yl]-propoxy}-phenoxy)-piperidin-1-yl]-ethanone dicitrate salt according to claim 9 **characterized in that** absorption bands in the Raman spectra thereof are present in value ranges of 3070; 2971; 2933; 1611; 1489; 936; 854; 782; 720; 660 $cm^{-1}$ ± 4 $cm^{-1}$.

**13.** The salts according to claim 1, wherein the salt is dihydrobromide salt.

**14.** The crystalline form of 1-[4-(4-{3-[(2R)-2-methyl-pyrrolidin-1-yl]-propoxy}-phenoxy)-piperidin-1-yl]-ethanone dihydrobromide salt according to claim 13 **characterized in that** characteristic reflections in the X-ray powder diffractogram thereof are present in scattering angle value ranges of 5.6; 11.3; 16.4; 16.7; 17.0; 24.7; 28.5° ± 0.2° 2θ, as measured at CuKa wavelength.

**15.** The crystalline form of 1-[4-(4-{3-[(2R)-2-methyl-pyrrolidin-1-yl]-propoxy}-phenoxy)-piperidin-1-yl]-ethanone dihydrobromide salt according to claim 13 **characterized in that** absorption bands in the infrared spectra thereof are present in value ranges of 3419; 2930; 2614; 1685; 1616; 1508; 1232; 817 $cm^{-1}$ ± 4 $cm^{-1}$.

**16.** The crystalline form of 1-[4-(4-{3-[(2R)-2-methyl-pyrrolidin-1-yl]-propoxy}-phenoxy)-piperidin-1-yl]-ethanone dihydrobromide salt according to claim 13 **characterized in that** absorption bands in the Raman spectra thereof are present in value ranges of 3072; 3022; 3045; 2935; 1613; 1265; 1164; 852 $cm^{-1}$ ± 4 $cm^{-1}$.

**17.** The salts according to claim 1, wherein the salt is sulfate salt.

**18.** The crystalline form of 1-[4-(4-{3-[(2R)-2-methyl-pyrrolidin-1-yl]-propoxy}-phenoxy)-piperidin-1-yl]-ethanone sulfate salt according to claim 17 **characterized in that** characteristic reflections in the X-ray powder diffractogram thereof are present in scattering angle value ranges of 7.8; 11.7; 15.6; 18.0; 18.5; 19.6; 19.9; 22.9° ± 0.2° 2θ, as measured at CuKα wavelength.

**19.** The crystalline form of 1-[4-(4-{3-[(2R)-2-methyl-pyrrolidin-1-yl]-propoxy}-phenoxy)-piperidin-1-yl]-ethanone sulfate salt according to claim 17 **characterized in that** absorption bands in the infrared spectra thereof are present in value ranges of 3389; 2955; 2615; 2513; 1590; 1507; 1220; 1044; 855; 591 $cm^{-1}$ ± 4 $cm^{-1}$.

**20.** The crystalline form of 1-[4-(4-{3-[(2R)-2-methyl-pyrrolidin-1-yl]-propoxy}-phenoxy)-piperidin-1-yl]-ethanone sulfate salt according to claim 17 **characterized in that** absorption bands in the Raman spectra thereof are present in value ranges of 3076; 3059; 2935; 1613; 1584; 1453; 1257; 1053; 854; 723 $cm^{-1}$ ± 4 $cm^{-1}$.

**21.** The salts according to claim 1, wherein the salt is oxalate salt.

**22.** The crystalline form of 1-[4-(4-{3-[(2R)-2-methyl-pyrrolidin-1-yl]-propoxy}-phenoxy)-piperidin-1-yl]-ethanone oxalate salt according to claim 21 **characterized in that** characteristic reflections in the X-ray powder diffractogram thereof are present in scattering angle value ranges of 8.5; 14.8; 15.4; 16.5; 17.4; 20.8; 22.5° ± 0.2° 2θ, as measured at CuKα wavelength.

**23.** The crystalline form of 1-[4-(4-{3-[(2R)-2-methyl-pyrrolidin-1-yl]-propoxy}-phenoxy)-piperidin-1-yl]-ethanone oxalate salt according to claim 21 **characterized in that** absorption bands in the infrared spectra thereof are present in value ranges of 3431; 2513; 1987; 1706; 1693; 1507; 1220; 832; 722 $cm-^{1}$ ± 4 $cm^{-1}$.

**24.** The crystalline form of 1-[4-(4-{3-[(2R)-2-methyl-pyrrolidin-1-yl]-propoxy}-phenoxy)-piperidin-1-yl]-ethanone oxalate salt according to claim 21 **characterized in that** absorption bands in the Raman spectra thereof are present in value ranges of 3077; 2935; 2883; 1611; 1477; 1443; 858; 842; 725 $cm^{-1}$ ± 4 $cm^{-1}$.

**25.** Process for the preparation of a compound according to any one of claims 1 to 24 **characterized in that** the 1-[4-(4-{3-[(2R)-2-methyl-pyrrolidin-1-yl]-propoxy}-phenoxy)-piperidin-1-yl]-ethanone base is dissolved in a suitable solvent or mixture of solvents, followed by the addition of the acid or a salt thereof - formed by a base weaker than 1-[4-(4-{3-[(2R)-2-methyl-pyrrolidin-1-yl]-propoxy}-phenoxy)-piperidin-1-yl]-ethanone - or a solution thereof, to the mixture, then the concentration of the reaction mixture is optionally increased, or without it, at room temperature or after cooling, the precipitated product is isolated by filtration.

**26.** Process for the preparation of a compound according to any one of claims 1 to 24 **characterized in that** the 1-[4-(4-{3-[(2R)-2-methyl-pyrrolidin-1-yl]-propoxy}-phenoxy)-piperidin-1-yl]-ethanone base is dissolved in a suitable solvent or mixture of solvents, followed by the addition of the acid or a salt thereof - formed by a base weaker than 1-[4-(4-{3-[(2R)-2-methyl-pyrrolidin-1-yl]-propoxy}-phenoxy)-piperidin-1-yl]-ethanone - or a solution thereof, to the mixture, then crystallized using a suitable antisolvent added to the solution thus obtained, at room temperature or after cooling, to isolate the precipitated product by filtration.

**27.** The process according to claim 25 **characterized in that** the 1-[4-(4-{3-[(2R)-2-methyl-pyrrolidin-1-yl]-propoxy}

-phenoxy)-piperidin-1-yl]-ethanone monocitrate and dicitrate salts according to claims 2 to 12 is prepared according to the following process:

(i) dissolving the 1-[4-(4-{3-[(2R)-2-methyl-pyrrolidin-1-yl]-propoxy}-phenoxy)-piperidin-1-yl]-ethanone base in acetone; and
(ii) crystallizing the citrate salt by adding a solution of citric acid in acetone to the solution of the base in acetone.

**28.** The process according to claim 25 **characterized in that** the 1-[4-(4-{3-[(2R)-2-methyl-pyrrolidin-1-yl]-propoxy} -phenoxy)-piperidin-1-yl]-ethanone monocitrate and dicitrate salts according to claims 2 to 12 is prepared according to the following process:

(i) dissolving the 1-[4-(4-{3-[(2R)-2-methyl-pyrrolidin-1-yl]-propoxy}-phenoxy)-piperidin-1-yl]-ethanone base in acetone; and
(ii) crystallizing the citrate salt by adding the solution of the base in acetone to a solution of citric acid in acetone.

**29.** The process according to claim 25 **characterized in that** the 1-[4-(4-{3-[(2R)-2-methyl-pyrrolidin-1-yl]-propoxy} -phenoxy)-piperidin-1-yl]-ethanone dicitrate salt according to claims 9 to 12 is prepared according to the following process:

(i) starting from the 1-[4-(4-{3-[(2R)-2-methyl-pyrrolidin-1-yl]-propoxy}-phenoxy)-piperidin-1-yl]-ethanone dihydrochloride salt, the base is released and the solution is evaporated after acetone solvent exchange; and
(ii) crystallizing the dicitrate salt by adding the solution of the base in acetone to a solution of citric acid in acetone.

**30.** The 1-[4-(4-{3-[(2R)-2-methyl-pyrrolidin-1-yl]-propoxy}-phenoxy)-piperidin-1-yl]-ethanone salts of any one of claims 1 to 24 for use in the treatment and/or prevention of conditions requiring the modulation of histamine H3 receptors.

**31.** Use of the 1-[4-(4-{3-[(2R)-2-methyl-pyrrolidin-1-yl]-propoxy}-phenoxy)-piperidin-1-yl]-ethanone salts of any one of claims 1 to 24 for the manufacture of a pharmaceutical composition.

**32.** Pharmaceutical composition comprising a therapeutically effective amount of the 1-[4-(4-{3-[(2R)-2-methyl-pyrrolidin-1-yl]-propoxy}-phenoxy)-piperidin-1-yl]-ethanone salts of any one of claims 1 to 24 together with one or more pharmaceutically acceptable excipients.

**33.** Pharmaceutical composition according to claim 31 for use in the treatment and/or prevention of conditions requiring the modulation of histamine $H_3$ receptors.

**34.** Pharmaceutical composition according to claim 31 for use in the treatment and/or prevention of autism spectrum disorder.

## Patentansprüche

**1.** 1-[4-(4-{3-[(2R)-2-Methylpyrrolidin-1-yl]-propoxy}-phenoxy)-piperidin-1-yl]-ethanonsalze, **dadurch gekennzeichnet, dass** sie auf Grundlage der dynamischen Dampfsorptionsanalyse (DVS-Analyse) bei 25 °C

a.) nur über 70 % relativer Luftfeuchtigkeit ein Zerfließen zeigen, d. h. der kritische Wert der relativen Luftfeuchtigkeit (CRH, critical relative humidity), bestimmt im Bereich von 10 bis 90 % RH der Sorptionskurve, der den Beginn des Zerfließens anzeigt, beträgt mindestens 70 %; oder
b.) kein Zerfließen zeigen, d. h. **gekennzeichnet durch** einen Wert von 0,5 > $\Delta m/\Delta RH$ im Bereich von 10 bis 90 % RH der Sorptionskurve, wobei
$\Delta m$ = m2-m1, wobei die Differenz der Quasi-Gleichgewichts-relativen Massenänderungen m2 und m1 den relativen Luftfeuchtigkeitswerten RH2 und RH1 entsprechen und wobei

$$\Delta RH = RH2\text{-}RH1 = 10,$$

wobei die Salze aus der Gruppe ausgewählt sind, die aus Salzen besteht, die mit Bromwasserstoff, Schwefelsäure, Oxalsäure und Zitronensäure gebildet werden.

2. Salze nach Anspruch 1, wobei das Salz Monocitratsalz ist.

3. Kristalline Form A von 1-[4-(4-{3-[(2R)-2-Methylpyrrolidin-1-yl]-propoxy}-phenoxy)-piperidin-1-yl]-ethanonmonocitratsalz nach Anspruch 2, **dadurch gekennzeichnet, dass** in dessen Röntgenpulverdiffraktogramm charakteristische Reflexe in Streuwinkel-Wertebereichen von 9,4; 11,1; 13,5; 18,0; 19,5; 19,8; 21,5° $\pm$ 0,2° 2$\theta$ vorliegen, gemessen bei der CuK$\alpha$ Wellenlänge.

4. Kristalline Form A von 1-[4-(4-{3-[(2R)-2-Methylpyrrolidin-1-yl]-propoxy}-phenoxy)-piperidin-1-yl]-ethanonmonocitratsalz gemäß Anspruch 2, **dadurch gekennzeichnet, dass** in dessen Infrarotspektren Absorptionsbanden in Wertebereichen von 3466; 3011; 2962; 1731; 1618; 1594; 1507; 1217; 1043; 665 $cm^{-1}$ $\pm$ 4 $cm^{-1}$ vorliegen.

5. Kristalline Form A von 1-[4-(4-{3-[(2R)-2-Methylpyrrolidin-1-yl]-propoxy}-phenoxy)-piperidin-1-yl]-ethanonmonocitratsalz nach Anspruch 2, **dadurch gekennzeichnet, dass** in dessen Raman-Spektren Absorptionsbanden in Wertebereichen von 3065; 2961; 2931; 1715; 1618; 1480; 1242; 1134; 859; 840; 722 $cm^{-1}$ $\pm$ 4 $cm^{-1}$ vorliegen.

6. Kristalline Form B von 1-[4-(4-{3-[(2R)-2-Methylpyrrolidin-1-yl]-propoxy}-phenoxy)-piperidin-1-yl]-ethanonmonocitratsalz nach Anspruch 2, **dadurch gekennzeichnet, dass** in dessen Röntgenpulverdiffraktogramm charakteristische Reflexe in Streuwinkel-Wertebereichen von 9,5; 11,3; 13,4; 13,7; 14,0; 17,8; 19,1; 20,4; 22,3° $\pm$ 0,2° 2$\theta$ vorliegen, gemessen bei der CuK$\alpha$ Wellenlänge.

7. Kristalline Form B von 1-[4-(4-{3-[(2R)-2-Methylpyrrolidin-1-yl]-propoxy}-phenoxy)-piperidin-1-yl]-ethanonmonocitratsalz gemäß Anspruch 2, **dadurch gekennzeichnet, dass** in dessen Infrarotspektren Absorptionsbanden in Wertebereichen von 2962; 2872; 1732; 1637; 1595; 1508; 1217; 1068; 1043; 818 $cm^{-1}$ $\pm$ 4 $cm^{-1}$ vorliegen.

8. Kristalline Form B von 1-[4-(4-{3-[(2R)-2-Methylpyrrolidin-1-yl]-propoxy}-phenoxy)-piperidin-1-yl]-ethanonmonocitratsalz gemäß Anspruch 2, **dadurch gekennzeichnet, dass** in dessen Raman-Spektren Absorptionsbanden in Wertebereichen von 3086; 2930; 2881; 1718; 1628; 1476; 1251; 857; 718 $cm^{-1}$ $\pm$ 4 $cm^{-1}$ vorliegen.

9. Salze nach Anspruch 1, wobei das Salz Dicitratsalz ist.

10. Kristalline Form von 1-[4-(4-{3-[(2R)-2-Methylpyrrolidin-1-yl]-propoxy}-phenoxy)-piperidin-1-yl]-ethanondicitratsalz nach Anspruch 9, **dadurch gekennzeichnet, dass** in dessen Röntgenpulverdiffraktogramm charakteristische Reflexe in Streuwinkel-Wertebereichen von 10,2; 12,0; 12,8; 14,1; 19,0; 19,3; 20,5; 22,8° $\pm$ 0,2° 2$\theta$ vorliegen, gemessen bei der CuK$\alpha$ Wellenlänge.

11. Kristalline Form von 1-[4-(4-{3-[(2R)-2-Methylpyrrolidin-1-yl]-propoxy}-phenoxy)-piperidin-1-yl]-ethanondicitratsalz gemäß Anspruch 9, **dadurch gekennzeichnet, dass** in dessen Infrarotspektren Absorptionsbanden in Wertebereichen von 3523; 3038; 2521; 1727; 1687; 1587; 1507; 1216; 782; 661 $cm^{-1}$ $\pm$ 4 $cm^{-1}$ vorliegen.

12. Kristalline Form von 1-[4-(4-{3-[(2R)-2-Methylpyrrolidin-1-yl]-propoxy}-phenoxy)-piperidin-1-yl]-ethanondicitratsalz gemäß Anspruch 9, **dadurch gekennzeichnet, dass** in dessen Raman-Spektren Absorptionsbanden in Wertebereichen von 3070; 2971; 2933; 1611; 1489; 936; 854; 782; 720; 660 $cm^{-1}$ $\pm$ 4 $cm^{-1}$ vorliegen.

13. Salze nach Anspruch 1, wobei das Salz Dihydrobromidsalz ist.

14. Kristalline Form von 1-[4-(4-{3-[(2R)-2-Methylpyrrolidin-1-yl]-propoxy}-phenoxy)-piperidin-1-yl]-ethanondihydrobromidsalz nach Anspruch 13, **dadurch gekennzeichnet, dass** in dessen Röntgenpulverdiffraktogramm charakteristische Reflexe in Streuwinkel-Wertebereichen von 5,6; 11,3; 16,4; 16,7; 17,0; 24,7; 28,5° $\pm$ 0,2° 2$\theta$ vorliegen, gemessen bei der CuK$\alpha$ Wellenlänge.

15. Kristalline Form von 1-[4-(4-{3-[(2R)-2-Methylpyrrolidin-1-yl]-propoxy}-phenoxy)-piperidin-1-yl]-ethanondihydrobromidsalz gemäß Anspruch 13, **dadurch gekennzeichnet, dass** in dessen Infrarotspektren Absorptionsbanden in Wertebereichen von 3419; 2930; 2614; 1685; 1616; 1508; 1232; 817 $cm^{-1}$ $\pm$ 4 $cm^{-1}$ vorliegen.

16. Kristalline Form von 1-[4-(4-{3-[(2R)-2-Methylpyrrolidin-1-yl]-propoxy}-phenoxy)-piperidin-1-yl]-ethanondihydrobromidsalz gemäß Anspruch 13, **dadurch gekennzeichnet, dass** in dessen Raman-Spektren Absorptionsbanden in Wertebereichen von 3072; 3022; 3045; 2935; 1613; 1265; 1164; 852 $cm^{-1}$ $\pm$ 4 $cm^{-1}$ vorliegen.

**17.** Salze nach Anspruch 1, wobei das Salz Sulfatsalz ist.

**18.** Kristalline Form von 1-[4-(4-{3-[(2R)-2-Methylpyrrolidin-1-yl]-propoxy}-phenoxy)-piperidin-1-yl]-ethanonsulfatsalz nach Anspruch 17, **dadurch gekennzeichnet, dass** in dessen Röntgenpulverdiffraktogramm charakteristische Reflexe in Streuwinkel-Wertebereichen von 7,8; 11,7; 15,6; 18,0; 18,5; 19,6; 19,9; 22,9° ± 0,2° 2θ vorliegen, gemessen bei der CuKα Wellenlänge.

**19.** Kristalline Form von 1-[4-(4-{3-[(2R)-2-Methylpyrrolidin-1-yl]-propoxy}-phenoxy)-piperidin-1-yl]-ethanonsulfatsalz gemäß Anspruch 17, **dadurch gekennzeichnet, dass** in dessen Infrarotspektren Absorptionsbanden in Wertebereichen von 3389; 2955; 2615; 2513; 1590; 1507; 1220; 1044; 855; 591 $cm^{-1}$ ± 4 $cm^{-1}$ vorliegen.

**20.** Kristalline Form von 1-[4-(4-{3-[(2R)-2-Methylpyrrolidin-1-yl]-propoxy}-phenoxy)-piperidin-1-yl]-ethanonsulfatsalz gemäß Anspruch 17, **dadurch gekennzeichnet, dass** in dessen Raman-Spektren Absorptionsbanden in Wertebereichen von 3076; 3059; 2935; 1613; 1584; 1453; 1257; 1053; 854; 723 $cm^{-1}$ ± 4 $cm^{-1}$ vorliegen.

**21.** Salze nach Anspruch 1, wobei das Salz Oxalatsalz ist.

**22.** Kristalline Form von 1-[4-(4-{3-[(2R)-2-Methylpyrrolidin-1-yl]-propoxy}-phenoxy)-piperidin-1-yl]-ethanonoxalatsalz nach Anspruch 21, **dadurch gekennzeichnet, dass** in dessen Röntgenpulverdiffraktogramm charakteristische Reflexe in Streuwinkel-Wertebereichen von 8,5; 14,8; 15,4; 16,5; 17,4; 20,8; 22,5° ± 0,2° 2θ vorliegen, gemessen bei der CuKα Wellenlänge.

**23.** Kristalline Form von 1-[4-(4-{3-[(2R)-2-Methylpyrrolidin-1-yl]-propoxy}-phenoxy)-piperidin-1-yl]-ethanonoxalatsalz gemäß Anspruch 21, **dadurch gekennzeichnet, dass** in dessen Infrarotspektren Absorptionsbanden in Wertebereichen von 3431; 2513; 1987; 1706; 1693; 1507; 1220; 832; 722 $cm^{-1}$ ± 4 $cm^{-1}$ vorliegen.

**24.** Kristallin von 1-[4-(4-{3-[(2R)-2-Methylpyrrolidin-1-yl]-propoxy}-phenoxy)-piperidin-1-yl]-ethanonoxalatsalz gemäß Anspruch 21, **dadurch gekennzeichnet, dass** in dessen Raman-Spektren Absorptionsbanden in Wertebereichen von 3077; 2935; 2883; 1611; 1477; 1443; 858; 842; 725 $cm^{-1}$ ± 4 $cm^{-1}$ vorliegen.

**25.** Verfahren zur Herstellung einer Verbindung nach einem der Ansprüche 1 bis 24, **dadurch gekennzeichnet, dass** die 1-[4-(4-{3-[(2R)-2-Methylpyrrolidin-1-yl]-propoxy}-phenoxy)-piperidin-1-yl]-ethanonbase in einem geeigneten Lösungsmittel oder Lösungsmittelgemisch gelöst wird, gefolgt von der Zugabe der Säure oder eines Salzes davon - gebildet aus einer Base, die schwächer als 1-[4-(4-{3-[(2R)-2-Methylpyrrolidin-1-yl]-propoxy}-phenoxy)-piperidin-1-yl]-ethanon ist - oder einer Lösung davon zu dem Gemisch, dann wird die Konzentration des Reaktionsgemischs gegebenenfalls erhöht oder nicht und das ausgefallene Produkt bei Raumtemperatur oder nach dem Abkühlen durch Filtration isoliert.

**26.** Verfahren zur Herstellung einer Verbindung nach einem der Ansprüche 1 bis 24, **dadurch gekennzeichnet, dass** die 1-[4-(4-{3-[(2R)-2-Methylpyrrolidin-1-yl]-propoxy}-phenoxy)-piperidin-1-yl]-ethanonbase in einem geeigneten Lösungsmittel oder Lösungsmittelgemisch gelöst wird, gefolgt von der Zugabe der Säure oder eines Salzes davon - gebildet aus einer Base, die schwächer als 1-[4-(4-{3-[(2R)-2-Methylpyrrolidin-1-yl]-propoxy}-phenoxy)-piperidin-1-yl]-ethanon ist - oder einer Lösung davon zu dem Gemisch, dann unter Verwendung eines der somit erhaltenen Lösung zugegebenen geeigneten Antilösungsmittels kristallisiert wird, um das ausgefallene Produkt bei Raumtemperatur oder nach dem Abkühlen durch Filtration zu isolieren.

**27.** Verfahren nach Anspruch 25, **dadurch gekennzeichnet, dass** die 1-[4-(4-{3-[(2R)-2-Methylpyrrolidin-1-yl]-propoxy}-phenoxy)-piperidin-1-yl]-ethanonmonocitrat- und -dicitratsalze gemäß den Ansprüchen 2 bis 12 nach dem folgenden Verfahren hergestellt werden:

(i) Auflösen der 1-[4-(4-{3-[(2R)-2-Methylpyrrolidin-1-yl]-propoxy}-phenoxy)-piperidin-1-yl]-ethanonbase in Aceton; und
(ii) Kristallisieren des Citratsalzes durch Zugabe einer Lösung aus Zitronensäure in Aceton zu der Lösung der Base in Aceton.

**28.** Verfahren nach Anspruch 25, **dadurch gekennzeichnet, dass** die 1-[4-(4-{3-[(2R)-2-Methylpyrrolidin-1-yl]-propoxy}-phenoxy)-piperidin-1-yl]-ethanonmonocitrat- und -dicitratsalze gemäß den Ansprüchen 2 bis 12 nach dem folgenden Verfahren hergestellt werden:

(i) Auflösen der 1-[4-(4-{3-[(2R)-2-Methylpyrrolidin-1-yl]-propoxy}-phenoxy)-piperidin-1-yl]-ethanonbase in Aceton; und
(ii) Kristallisieren des Citratsalzes durch Zugabe der Lösung aus der Base in Aceton zu einer Lösung aus Zitronensäure in Aceton.

**29.** Verfahren nach Anspruch 25, **dadurch gekennzeichnet, dass** das 1-[4-(4-{3-[(2R)-2-Methylpyrrolidin-1-yl]-propoxy}-phenoxy)-piperidin-1-yl]-ethanondicitratsalz gemäß den Ansprüchen 9 bis 12 nach dem folgenden Verfahren hergestellt wird:

(i) ausgehend von dem 1-[4-(4-{3-[(2R)-2-Methylpyrrolidin-1-yl]-propoxy}-phenoxy)-piperidin-1-yl]-ethanondihydrochloridsalz Freisetzen der Base und Eindampfen der Lösung nach Aceton-Lösungsmittelaustausch; und
(ii) Kristallisieren des Dicitratsalzes durch Zugabe der Lösung aus der Base in Aceton zu einer Lösung aus Zitronensäure in Aceton.

**30.** 1-[4-(4-{3-[(2R)-2-Methylpyrrolidin-1-yl]-propoxy}-phenoxy)-piperidin-1-yl]-ethanonsalze nach einem der Ansprüche 1 bis 24 zur Verwendung bei der Behandlung und/oder Vorbeugung von Zuständen, die eine Modulation von Histamin-$H_3$-Rezeptoren erfordern.

**31.** Verwendung der 1-[4-(4-{3-[(2R)-2-Methylpyrrolidin-1-yl]-propoxy}-phenoxy)-piperidin-1-yl]-ethanonsalze nach einem der Ansprüche 1 bis 24 zur Herstellung einer pharmazeutischen Zusammensetzung.

**32.** Pharmazeutische Zusammensetzung, umfassend eine therapeutisch wirksame Menge der 1-[4-(4-{3-[(2R)-2-Methylpyrrolidin-1-yl]-propoxy}-phenoxy)-piperidin-1-yl]-ethanonsalze nach einem der Ansprüche 1 bis 24 zusammen mit einem oder mehreren pharmazeutisch verträglichen Hilfsstoffen.

**33.** Pharmazeutischen Zusammensetzung nach Anspruch 32 zur Verwendung bei der Behandlung und/oder Vorbeugung von Zuständen, die eine Modulation von Histamin-$H_3$-Rezeptoren erfordern.

**34.** pharmazeutischen Zusammensetzung nach Anspruch 32 zur Verwendung bei der Behandlung und/oder Vorbeugung von Autismus-Spektrum-Störungen.

## Revendications

**1.** Sels de 1-[4-(4-{3-[(2R)-2-méthyl-pyrrolidine-1-yl]-propoxy}-phénoxy)-pipéridine-1-yl]-éthanone **caractérisés en ce qu'ils** sont basés sur l'analyse de sorption dynamique de vapeur (DVS) à 25 °C

a.) montrant une déliquescence uniquement au-dessus de 70 % d'humidité relative, c'est-à-dire que la valeur critique d'humidité relative (CRH), déterminée dans la plage de 10 à 90 % de HR de la courbe de sorption, indiquant le début de la déliquescence, est d'au moins 70 % ; ou
b.) ne montrant pas de déliquescence, c'est-à-dire **caractérisé par** une valeur de 0,5 > $\Delta m/\Delta HR$ dans la plage de 10 à 90 % de HR de la courbe de sorption, où
$\Delta m$ = m2-m1, la différence des variations de masse relative de quasi-équilibre m2 et m1 correspondant aux valeurs d'humidité relative HR2 et HR1 et où

$$\Delta HR = HR2\text{-}HR1 = 10,$$

dans lequel les sels sont choisis dans le groupe constitué des sels formés avec le bromure d'dihydrogène, l'acide sulfurique, l'acide oxalique et l'acide citrique.

**2.** Sels selon la revendication 1, le sel étant un sel monocitrate.

**3.** Forme cristalline A du sel de monocitrate de 1-[4-(4-{3-[(2R)-2-méthyl-pyrrolidine-1-yl]-propoxy}-phénoxy)-pipéridine-1-yl]-éthanone selon la revendication 2, **caractérisée en ce que** les réflexions caractéristiques dans son diffractogramme à rayons X sur poudre sont présentes dans des plages de valeurs d'angle de diffusion de 9,4 ; 11,1 ; 13,5 ; 18,0 ; 19,5 ; 19,8 ; 21,5° ± 0,2° 2θ, tel que mesuré à la longueur d'onde CuKα.

**4.** Forme cristalline A du sel de monocitrate de 1-[4-(4-{3-[(2R)-2-méthyl-pyrrolidine-1-yl]-propoxy}-phénoxy)-pipéridine-1-yl]-éthanone selon la revendication 2, **caractérisée en ce que** les bandes d'absorption dans leurs spectres infrarouges sont présentes dans des plages de valeurs de 3466 ; 3011 ; 2962 ; 1731 ; 1618 ; 1594 ; 1507 ; 1217 ; 1043 ; 665 $cm^{-1} \pm 4$ $cm^{-1}$.

**5.** Forme cristalline A du sel de monocitrate de 1-[4-(4-{3-[(2R)-2-méthyl-pyrrolidine-1-yl]-propoxy}-phénoxy)-pipéridine-1-yl]-éthanone selon la revendication 2, **caractérisée en ce que** les bandes d'absorption dans ses spectres Raman sont présentes dans des plages de valeurs de 3065 ; 2961 ; 2931 ; 1715 ; 1618 ; 1480 ; 1242 ; 1134 ; 859 ; 840 ; 722 $cm^{-1} \pm 4$ $cm^{-1}$.

**6.** Forme cristalline B du sel de monocitrate de 1-[4-(4-{3-[(2R)-2-méthyl-pyrrolidine-1-yl]-propoxy}-phénoxy)-pipéridine-1-yl]-éthanone selon la revendication 2, **caractérisée en ce que** les réflexions caractéristiques dans son diffractogramme à rayons X sur poudre sont présentes dans des plages de valeurs d'angle de diffusion de 9,5 ; 11,3 ; 13,4 ; 13,7 ; 14,0 ; 17,8 ; 19,1 ; 20,4 ; 22,3° ± 0,2° 2$\theta$, tel que mesuré à la longueur d'onde CuK$\alpha$.

**7.** Forme cristalline B du sel de monocitrate de 1-[4-(4-{3-[(2R)-2-méthyl-pyrrolidine-1-yl]-propoxy}-phénoxy)-pipéridine-1-yl]-éthanone selon la revendication 2, **caractérisée en ce que** les bandes d'absorption dans leurs spectres infrarouges sont présentes dans des plages de valeurs de 2962 ; 2872 ; 1732 ; 1637 ; 1595 ; 1508 ; 1217 ; 1068 ; 1043 ; 818 $cm^{-1} \pm 4$ $cm^{-1}$.

**8.** Forme cristalline B du sel de monocitrate de 1-[4-(4-{3-[(2R)-2-méthyl-pyrrolidine-1-yl]-propoxy}-phénoxy)-pipéridine-1-yl]-éthanone selon la revendication 2, **caractérisée en ce que** les bandes d'absorption dans ses spectres Raman sont présentes dans des plages de valeurs de 3086 ; 2930 ; 2881 ; 1718 ; 1628 ; 1476 ; 1251 ; 857 ; 718 $cm^{-1} \pm 4$ $cm^{-1}$.

**9.** Sels selon la revendication 1, le sel étant un sel dicitrate.

**10.** Forme cristalline du sel de dicitrate de 1-[4-(4-{3-[(2R)-2-méthyl-pyrrolidine-1-yl]-propoxy}-phénoxy)-pipéridine-1-yl]-éthanone selon la revendication 9, **caractérisée en ce que** les réflexions caractéristiques dans son diffractogramme à rayons X sur poudre sont présentes dans des plages de valeurs d'angle de diffusion de 10,2 ; 12,0 ; 12,8 ; 14,1 ; 19,0 ; 19,3 ; 20,5 ; 22,8° ± 0,2° 2$\theta$, tel que mesuré à la longueur d'onde CuK$\alpha$.

**11.** Forme cristalline du sel de dicitrate de 1-[4-(4-{3-[(2R)-2-méthyl-pyrrolidine-1-yl]-propoxy}-phénoxy)-pipéridine-1-yl]-éthanone selon la revendication 9, **caractérisée en ce que** les bandes d'absorption dans leurs spectres infrarouges sont présentes dans des plages de valeurs de 3523 ; 3038 ; 2521 ; 1727 ; 1687 ; 1587 ; 1507 ; 1216 ; 782 ; 661 $cm^{-1} \pm 4$ $cm^{-1}$.

**12.** Forme cristalline du sel de dicitrate de 1-[4-(4-{3-[(2R)-2-méthyl-pyrrolidine-1-yl]-propoxy}-phénoxy)-pipéridine-1-yl]-éthanone selon la revendication 9, **caractérisée en ce que** les bandes d'absorption dans ses spectres Raman sont présentes dans des plages de valeurs de 3070 ; 2971 ; 2933 ; 1611 ; 1489 ; 936 ; 854 ; 782 ; 720 ; 660 $cm^{-1} \pm 4$ $cm^{-1}$.

**13.** Sels selon la revendication 1, le sel étant un sel de dihydrobromure.

**14.** Forme cristalline du sel de dihydrobromure de 1-[4-(4-{3-[(2R)-2-méthyl-pyrrolidine-1-yl]-propoxy}-phénoxy)-pipéridine-1-yl]-éthanone selon la revendication 13, **caractérisée en ce que** les réflexions caractéristiques dans son diffractogramme à rayons X sur poudre sont présentes dans des plages de valeurs d'angle de diffusion de 5,6 ; 11,3 ; 16,4 ; 16,7 ; 17,0 ; 24,7 ; 28,5° ± 0,2° 2$\theta$, tel que mesuré à la longueur d'onde CuK$\alpha$.

**15.** Forme cristalline du sel de dihydrobromure de 1-[4-(4-{3-[(2R)-2-méthyl-pyrrolidine-1-yl]-propoxy}-phénoxy)-pipéridine-1-yl]-éthanone selon la revendication 13, **caractérisée en ce que** les bandes d'absorption dans leurs spectres infrarouges sont présentes dans des plages de valeurs de 3419 ; 2930 ; 2614 ; 1685 ; 1616 ; 1508 ; 1232 ; 817 $cm^{-1} \pm 4$ $cm^{-1}$.

**16.** Forme cristalline du sel de dihydrobromure de 1-[4-(4-{3-[(2R)-2-méthyl-pyrrolidine-1-yl]-propoxy}-phénoxy)-pipéridine-1-yl]-éthanone selon la revendication 13, **caractérisée en ce que** les bandes d'absorption dans ses spectres Raman sont présentes dans des plages de valeurs de 3072 ; 3022 ; 3045 ; 2935 ; 1613 ; 1265 ; 1164 ; 852 $cm^{-1} \pm 4$ $cm^{-1}$.

**17.** Sels selon la revendication 1, le sel étant un sel de sulfate.

**18.** Forme cristalline du sel de sulfate de 1-[4-(4-{3-[(2R)-2-méthyl-pyrrolidine-1-yl]-propoxy}-phénoxy)-pipéridine-1-yl]-éthanone selon la revendication 17, **caractérisée en ce que** les réflexions caractéristiques dans son diffractogramme à rayons X sur poudre sont présentes dans des plages de valeurs d'angle de diffusion de 7,8 ; 11,7 ; 15,6 ; 18,0 ; 18,5 ; 19,6 ; 19,9 ; 22,9° ± 0,2° 2θ, tel que mesuré à la longueur d'onde CuKα.

**19.** Forme cristalline de sel de sulfate de 1-[4-(4-{3-[(2R)-2-méthyl-pyrrolidine-1-yl]-propoxy}-phénoxy)-pipéridine-1-yl]-éthanone selon la revendication 17, **caractérisée en ce que** les bandes d'absorption dans leurs spectres infrarouges sont présentes dans des plages de valeurs de 3389 ; 2955 ; 2615 ; 2513 ; 1590 ; 1507 ; 1220 ; 1044 ; 855 ; 591 $cm^{-1}$ ± 4 $cm^{-1}$.

**20.** Forme cristalline de sel de sulfate de 1-[4-(4-{3-[(2R)-2-méthyl-pyrrolidine-1-yl]-propoxy}-phénoxy)-pipéridine-1-yl]-éthanone selon la revendication 17, **caractérisée en ce que** les bandes d'absorption dans ses spectres Raman sont présentes dans des plages de valeurs de 3076 ; 3059 ; 2935 ; 1613 ; 1584 ; 1453 ; 1257 ; 1053 ; 854 ; 723 $cm^{-1}$ ± 4 $cm^{-1}$.

**21.** Sels selon la revendication 1, le sel étant un sel d'oxalate.

**22.** Forme cristalline du sel d'oxalate de 1-[4-(4-{3-[(2R)-2-méthyl-pyrrolidine-1-yl]-propoxy}-phénoxy)-pipéridine-1-yl]-éthanone selon la revendication 21, **caractérisée en ce que** les réflexions caractéristiques dans son diffractogramme à rayons X sur poudre sont présentes dans des plages de valeurs d'angle de diffusion de 8,5 ; 14,8 ; 15,4 ; 16,5 ; 17,4 ; 20,8 ; 22,5° ± 0,2° 2θ, tel que mesuré à la longueur d'onde CuKα.

**23.** Forme cristalline de sel d'oxalate de 1-[4-(4-{3-[(2R)-2-méthyl-pyrrolidine-1-yl]-propoxy}-phénoxy)-pipéridine-1-yl]-éthanone selon la revendication 21, **caractérisée en ce que** les bandes d'absorption dans leurs spectres infrarouges sont présentes dans des plages de valeurs de 3431 ; 2513 ; 1987 ; 1706 ; 1693 ; 1507 ; 1220 ; 832 ; 722 $cm^{-1}$ ± 4 $cm^{-1}$.

**24.** Forme cristalline du sel d'oxalate de 1-[4-(4-{3-[(2R)-2-méthyl-pyrrolidine-1-yl]-propoxy}-phénoxy)-pipéridine-1-yl]-éthanone selon la revendication 21, **caractérisée en ce que** les bandes d'absorption dans ses spectres Raman sont présentes dans des plages de valeurs de 3077 ; 2935 ; 2883 ; 1611 ; 1477 ; 1443 ; 858 ; 842 ; 725 $cm^{-1}$ ± 4 $cm^{-1}$.

**25.** Procédé de préparation d'un composé selon l'une quelconque des revendications 1 à 24 **caractérisé en ce que** la base 1-[4-(4-{3-[(2R)-2-méthyl-pyrrolidine-1-yl]-propoxy}-phénoxy)-pipéridine-1-yl]-éthanone est dissoute dans un solvant approprié ou un mélange de solvants, puis l'on ajoute de l'acide ou un de ses sels - formé par une base plus faible que 1-[4-(4-{3-[(2R)-2-méthyl-pyrrolidine-1-yl]-propoxy}-phénoxy)-pipéridine-1-yl]-éthanone - ou une solution de celle-ci, au mélange, ensuite la concentration du mélange réactionnel est éventuellement augmentée ou non, à température ambiante ou après refroidissement, le produit précipité est isolé par filtration.

**26.** Procédé de préparation d'un composé selon l'une quelconque des revendications 1 à 24 **caractérisé en ce que** la base 1-[4-(4-{3-[(2R)-2-méthyl-pyrrolidine-1-yl]-propoxy}-phénoxy)-pipéridine-1-yl]-éthanone est dissoute dans un solvant approprié ou un mélange de solvants, puis l'on ajoute de l'acide ou un de ses sels - formé par une base plus faible que 1-[4-(4-{3-[(2R)-2-méthyl-pyrrolidine-1-yl]-propoxy}-phénoxy)-pipéridine-1-yl]-éthanone - ou une solution de celle-ci, au mélange, puis cristallisée à l'aide d'un antisolvant approprié ajouté à la solution ainsi obtenue, à température ambiante ou après refroidissement, pour isoler par filtration le produit précipité.

**27.** Procédé selon la revendication 25, **caractérisé en ce que** les sels de monocitrate et dicitrate de 1-[4-(4-{3-[(2R)-2-méthyl-pyrrolidine-1-yl]-propoxy}-phénoxy)-pipéridine-1-yl]-éthanone selon les revendications 2 à 12 sont préparés selon le procédé suivant :

(i) dissolution de la base 1-[4-(4-{3-[(2R)-2-méthyl-pyrrolidine-1-yl]-propoxy}-phénoxy)-pipéridine-1-yl]-éthanone dans l'acétone ; et
(ii) cristallisation du sel de citrate en ajoutant une solution d'acide citrique dans l'acétone à la solution de la base dans l'acétone.

**28.** Procédé selon la revendication 32, **caractérisé en ce que** les sels de monocitrate et dicitrate de 1-[4-(4-{3-[(2R)-2-méthyl-pyrrolidine-1-yl]-propoxy}-phénoxy)-pipéridine-1-yl]-éthanone selon les revendications 2 à 12 sont préparés selon le procédé suivant :

(i) dissolution de la base 1-[4-(4-{3-[(2R)-2-méthyl-pyrrolidine-1-yl]-propoxy}-phénoxy)-pipéridine-1-yl]-éthanone dans l'acétone ; et
(ii) cristallisation du sel de citrate en ajoutant la solution de la base dans l'acétone à une solution d'acide citrique dans l'acétone.

**29.** Procédé selon la revendication 25, **caractérisé en ce que** le sel de dicitrate de 1-[4-(4-{3-[(2R)-2-méthyl-pyrrolidine-1-yl]-propoxy}-phénoxy)-pipéridine-1-yl]-éthanone selon les revendications 9 à 12 est préparé selon le procédé suivant :

(i) à partir du sel de dichlorhydrate de 1-[4-(4-{3-[(2R)-2-méthyl-pyrrolidine-1-yl]-propoxy}-phénoxy)-pipéridine-1-yl]-éthanone, la base est libérée et la solution est évaporée après échange du solvant acétone ; et
(ii) cristallisation du sel de dicitrate en ajoutant la solution de la base dans l'acétone à une solution d'acide citrique dans l'acétone.

**30.** Sels de 1-[4-(4-{3-[(2R)-2-méthyl-pyrrolidine-1-yl]-propoxy}-phénoxy)-pipéridine-1-yl]-éthanone selon l'une quelconque des revendications 1 à 24 pour utilisation dans le traitement et/ou la prévention d'affections nécessitant la modulation des récepteurs de l'histamine $H_3$.

**31.** Utilisation des sels de 1-[4-(4-{3-[(2R)-2-méthyl-pyrrolidine-1-yl]-propoxy}-phénoxy)-pipéridine-1-yl]-éthanone selon l'une quelconque des revendications 1 à 24 pour la fabrication d'une composition pharmaceutique.

**32.** Composition pharmaceutique comprenant une quantité thérapeutiquement efficace des sels de 1-[4-(4-{3-[(2R)-2-méthyl-pyrrolidine-1-yl]-propoxy}-phénoxy)-pipéridine-1-yl]-éthanone selon l'une quelconque des revendications 1 à 24, conjointement avec un ou plusieurs excipients pharmaceutiquement acceptables.

**33.** Composition pharmaceutique selon la revendication 32 pour utilisation dans le traitement et/ou la prévention d'affections nécessitant la modulation des récepteurs de l'histamine $H_3$.

**34.** Composition pharmaceutique selon la revendication 32 pour utilisation dans le traitement et/ou la prévention des troubles du spectre autistique.

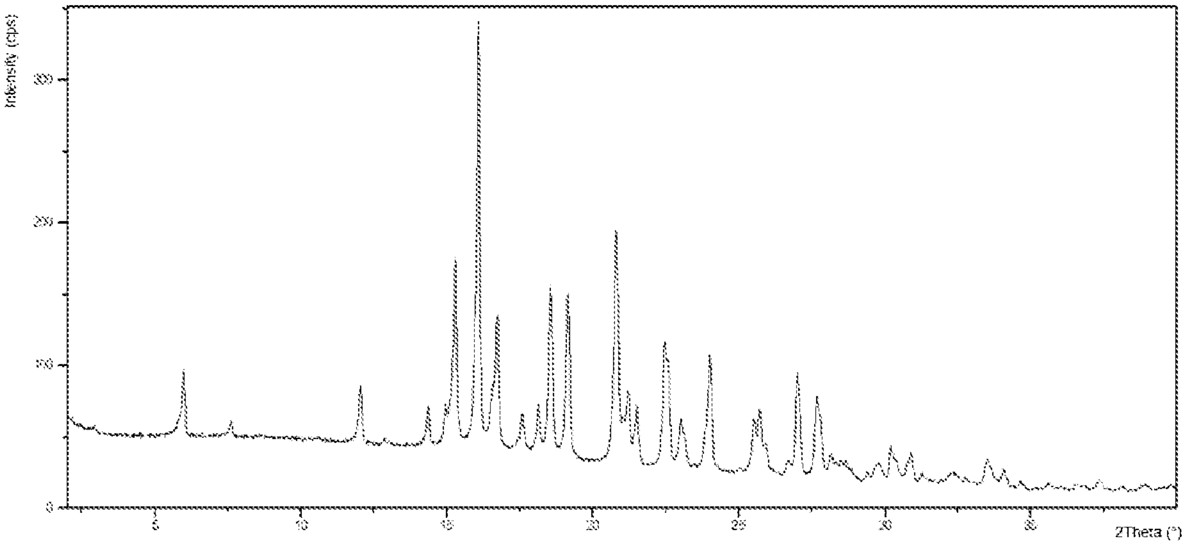


Figure 1

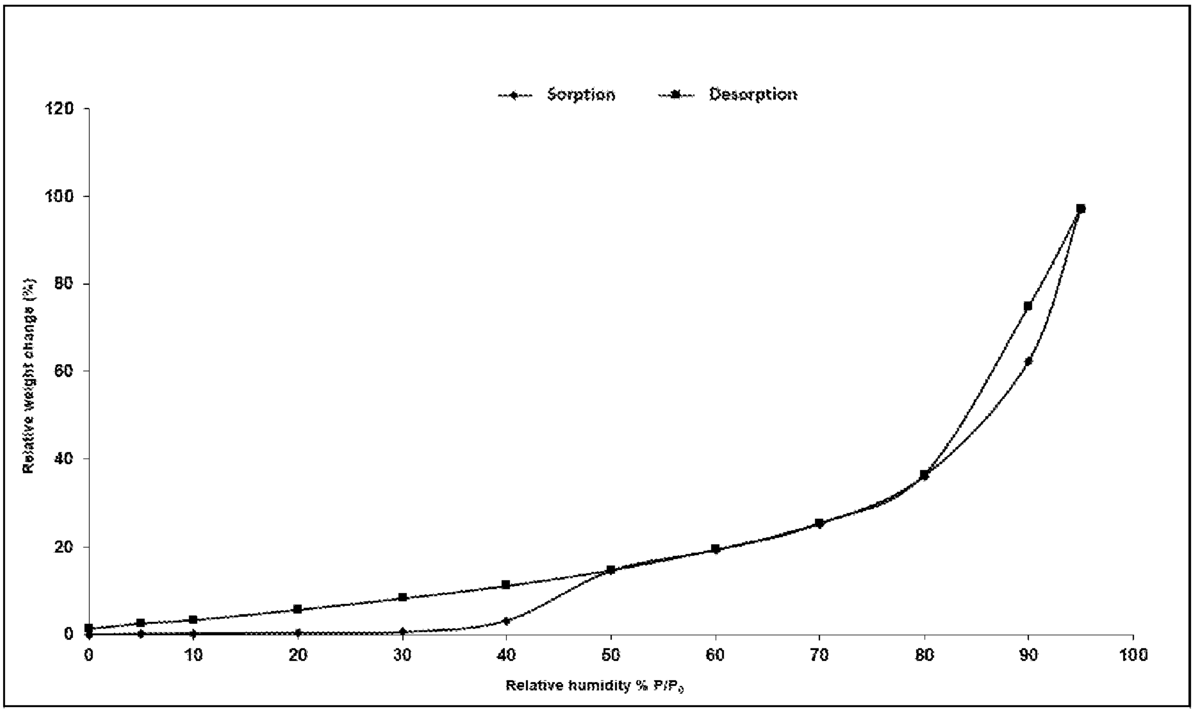


Figure 2

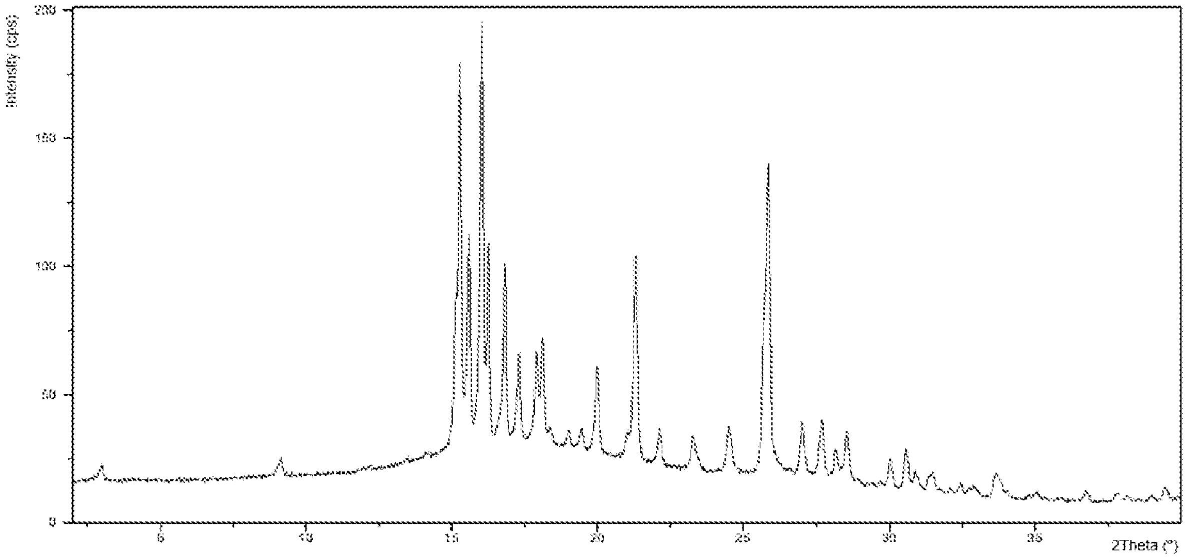


**Figure 3**

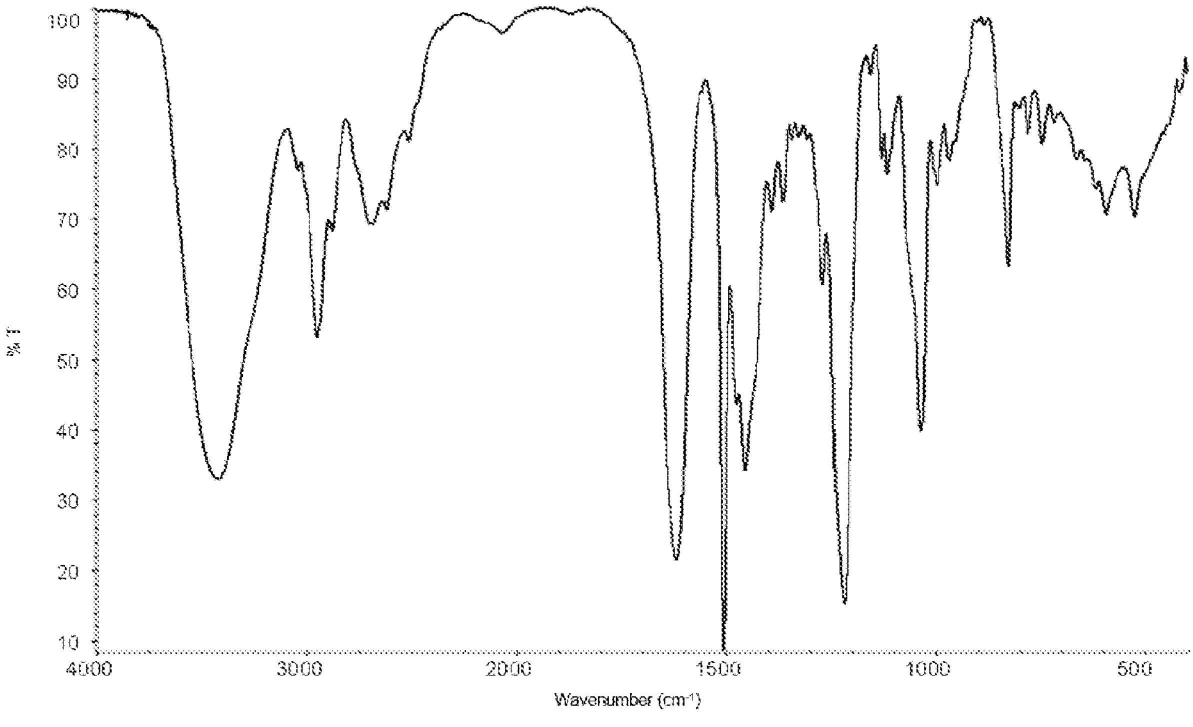


**Figure 4**

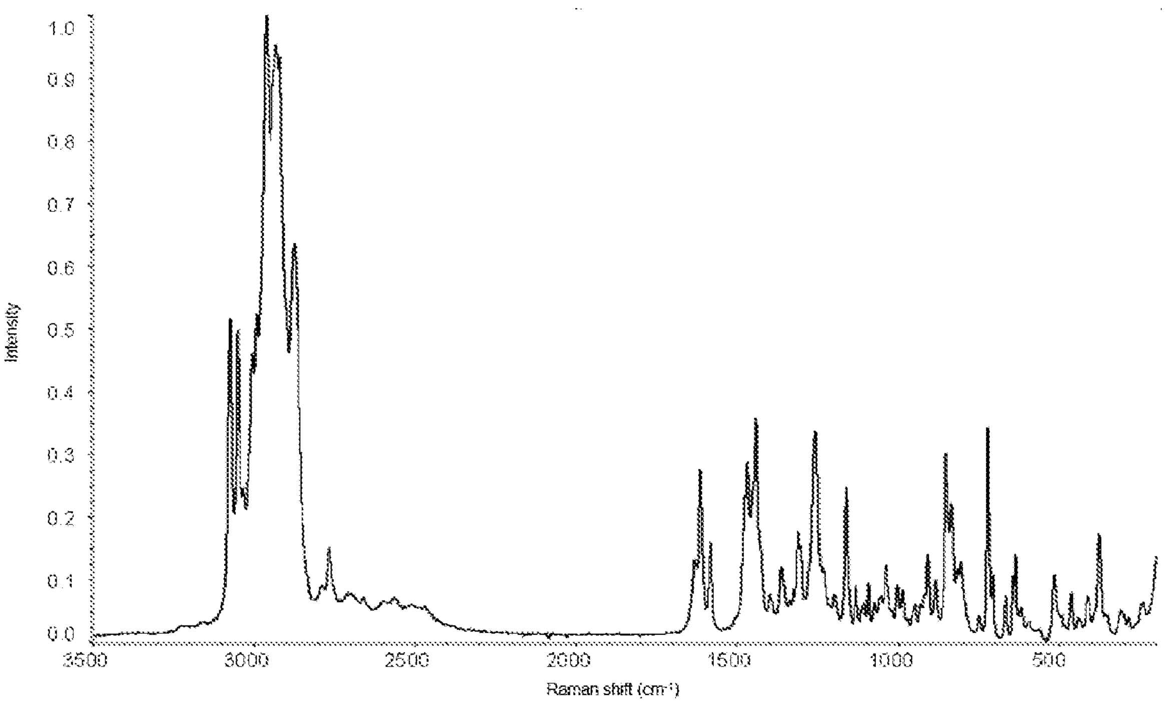


Figure 5

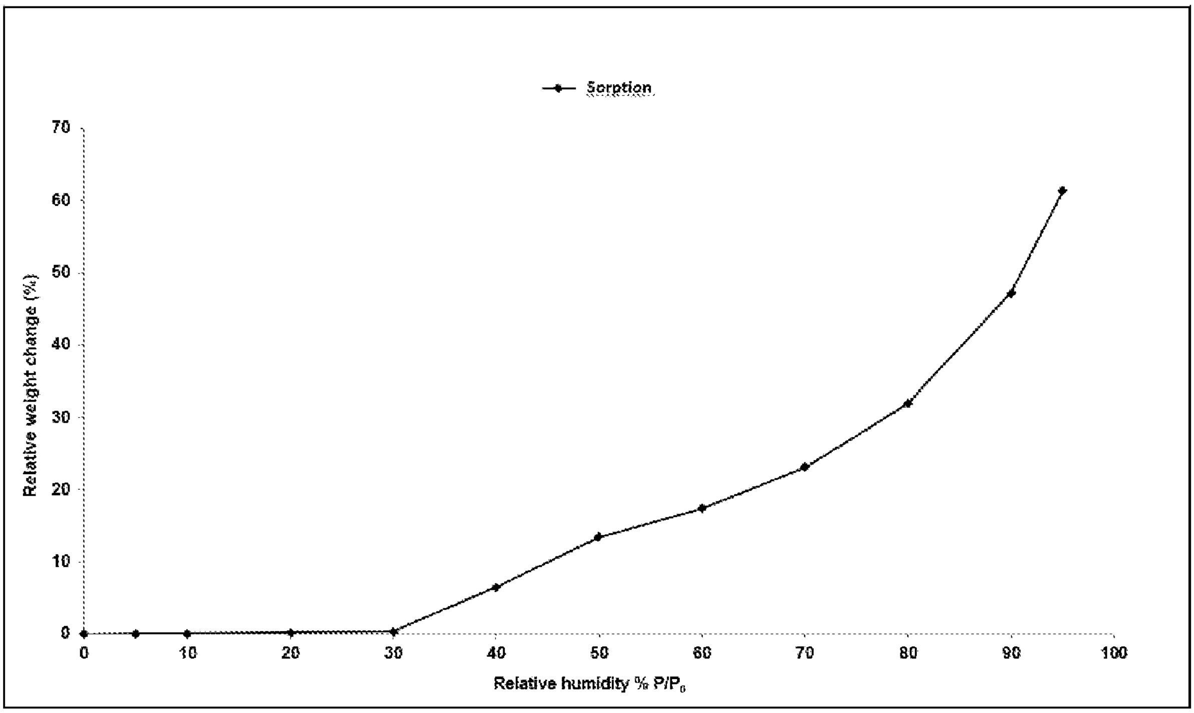


Figure 6

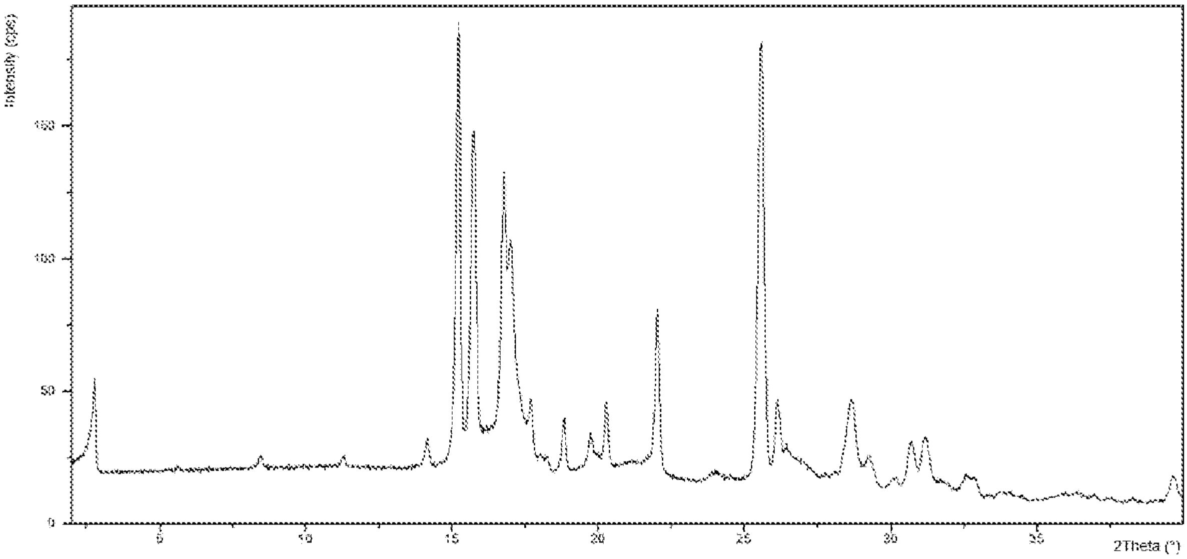


Figure 7

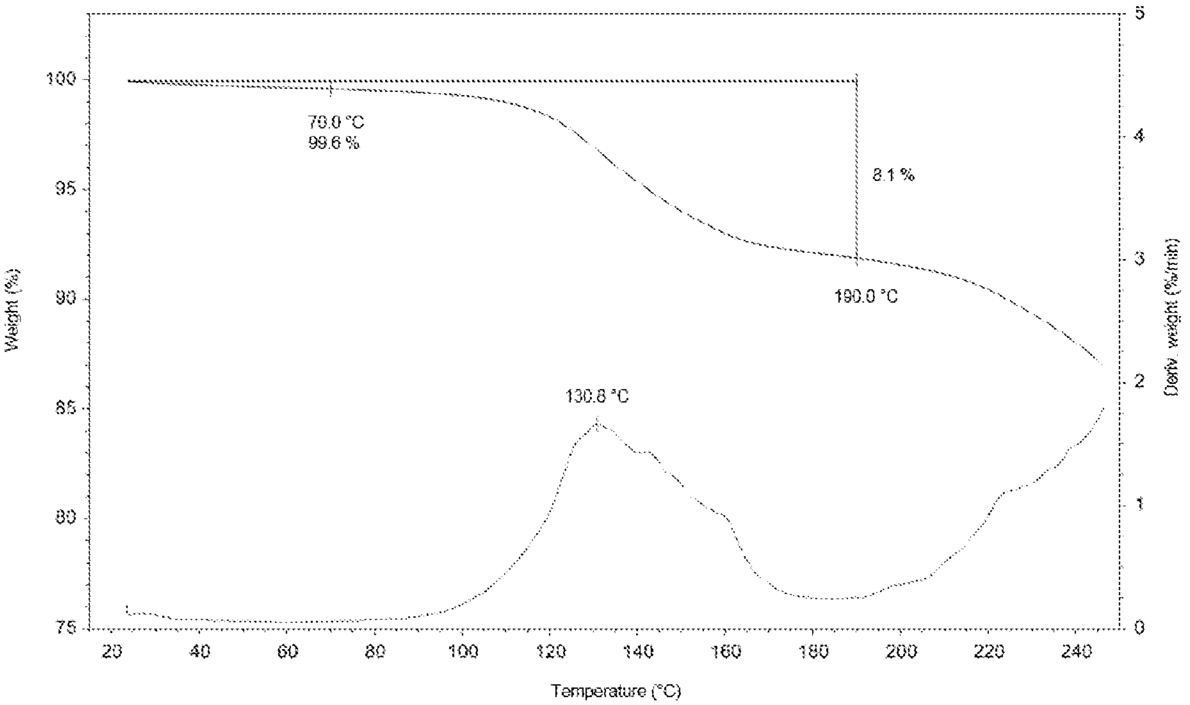


Figure 8

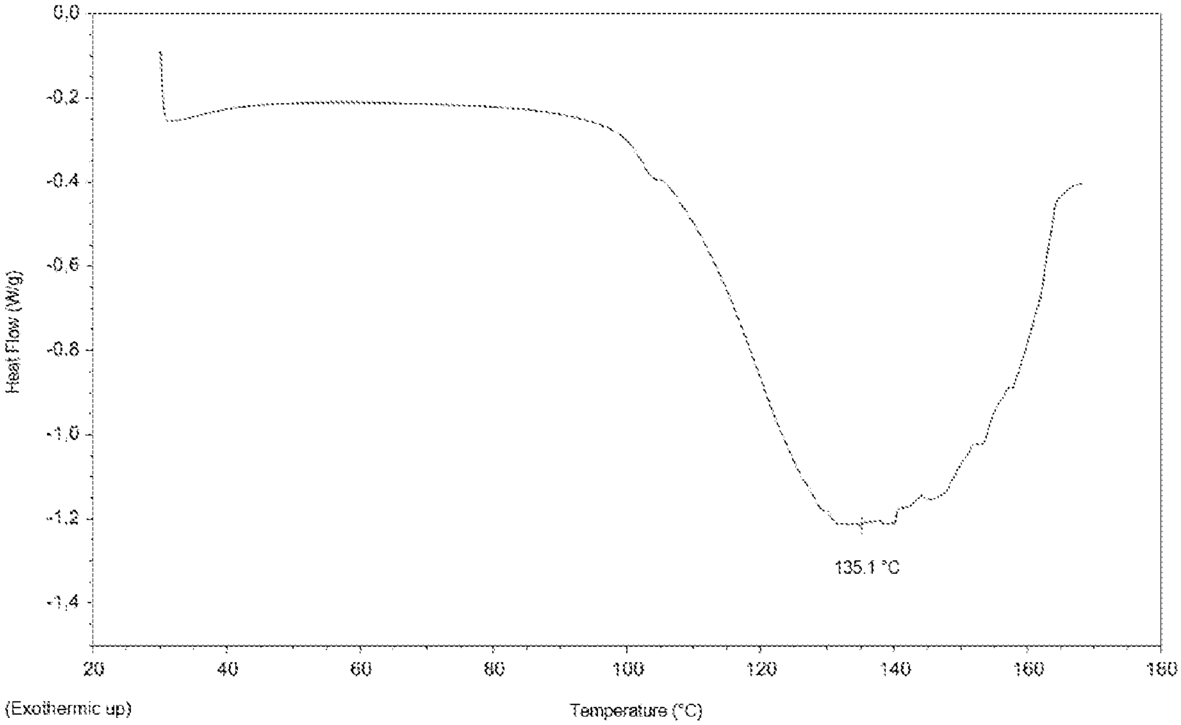


**Figure 9**

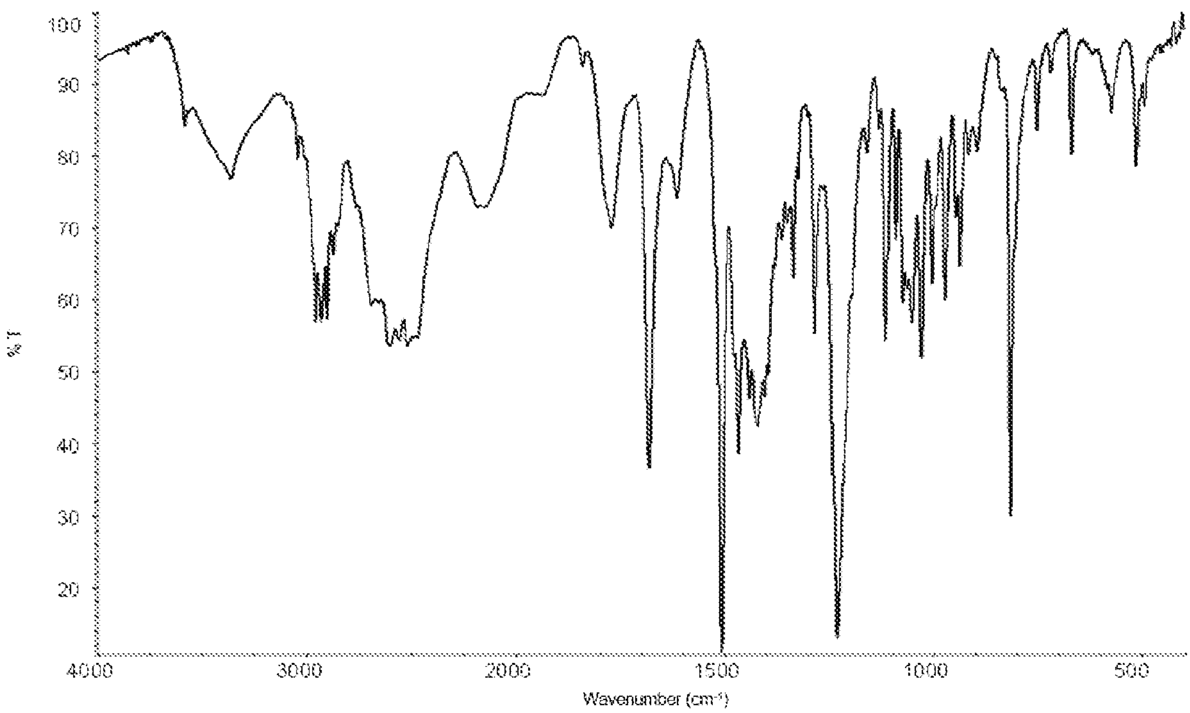


**Figure 10**

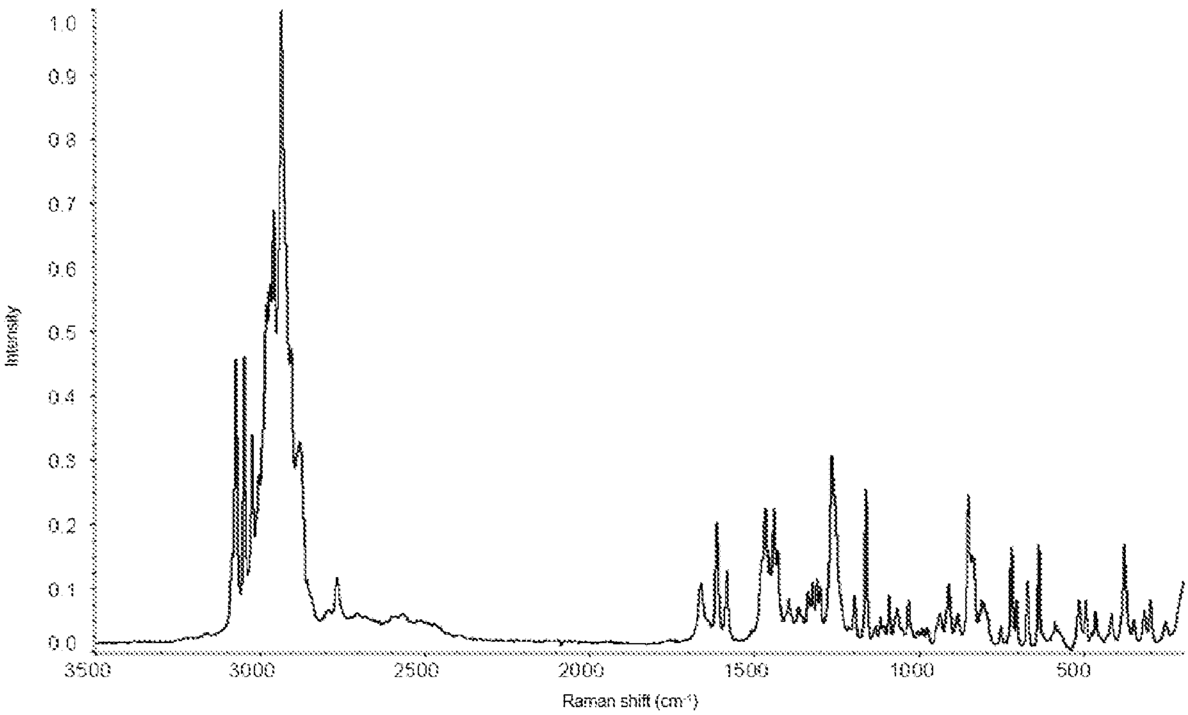


Figure 11

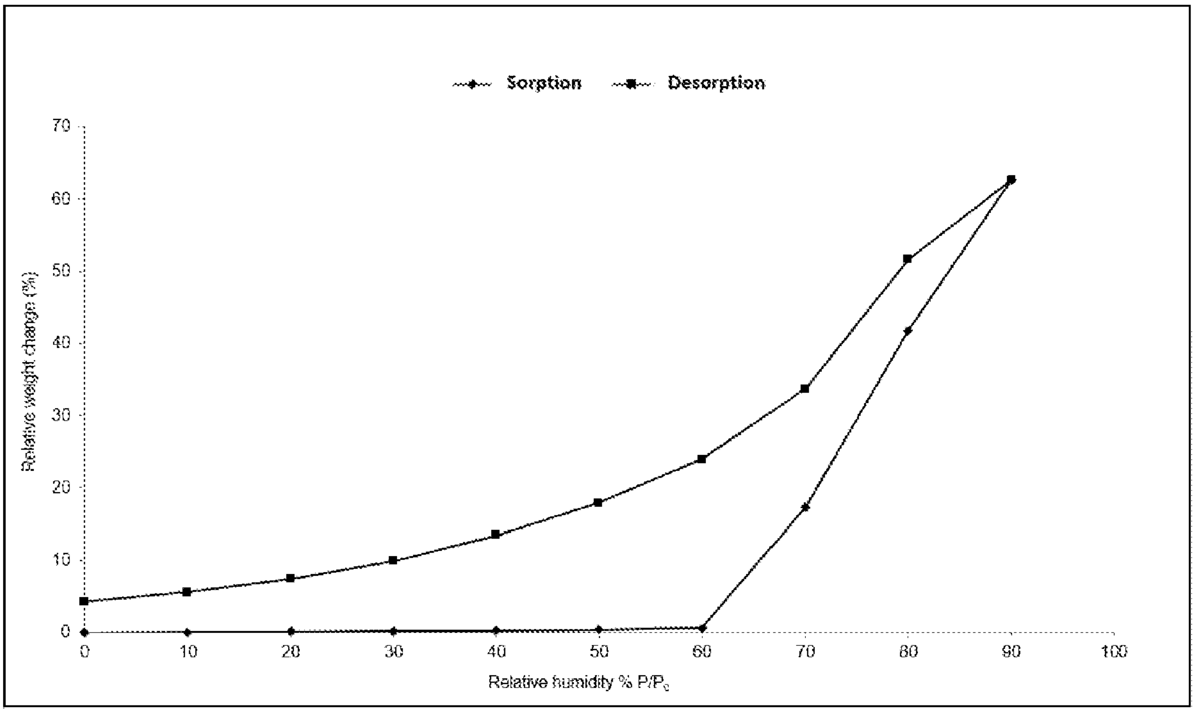


Figure 12

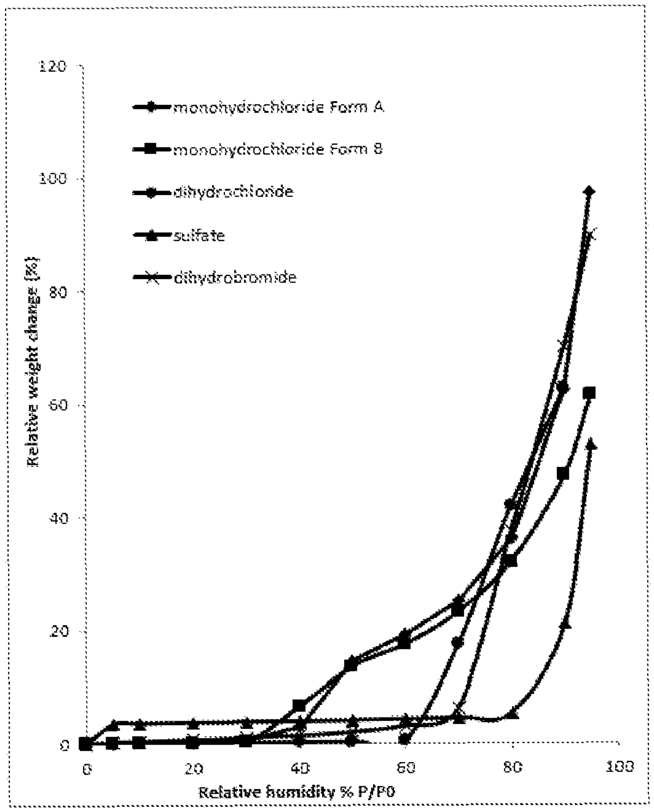


Figure 13 *(a)*

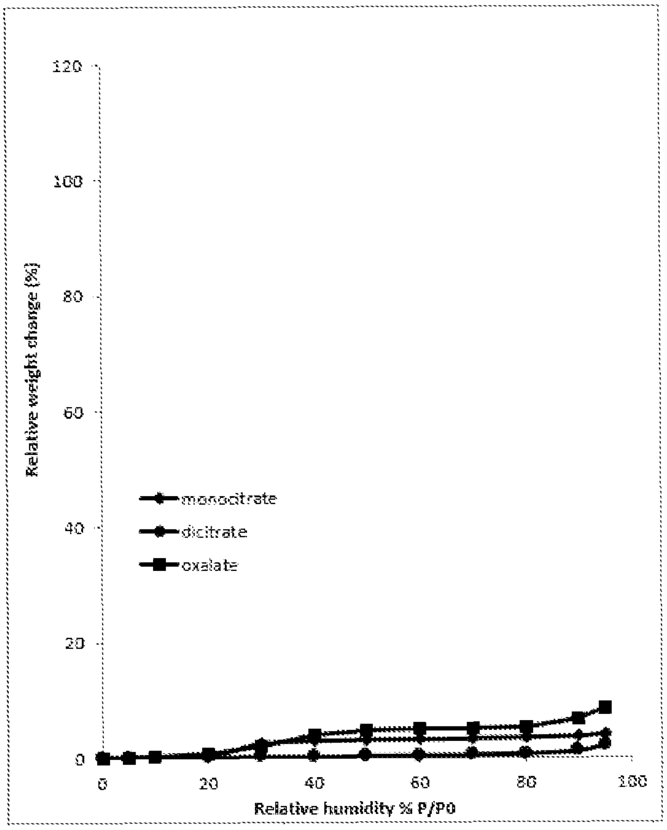


Figure 14 *(b)*

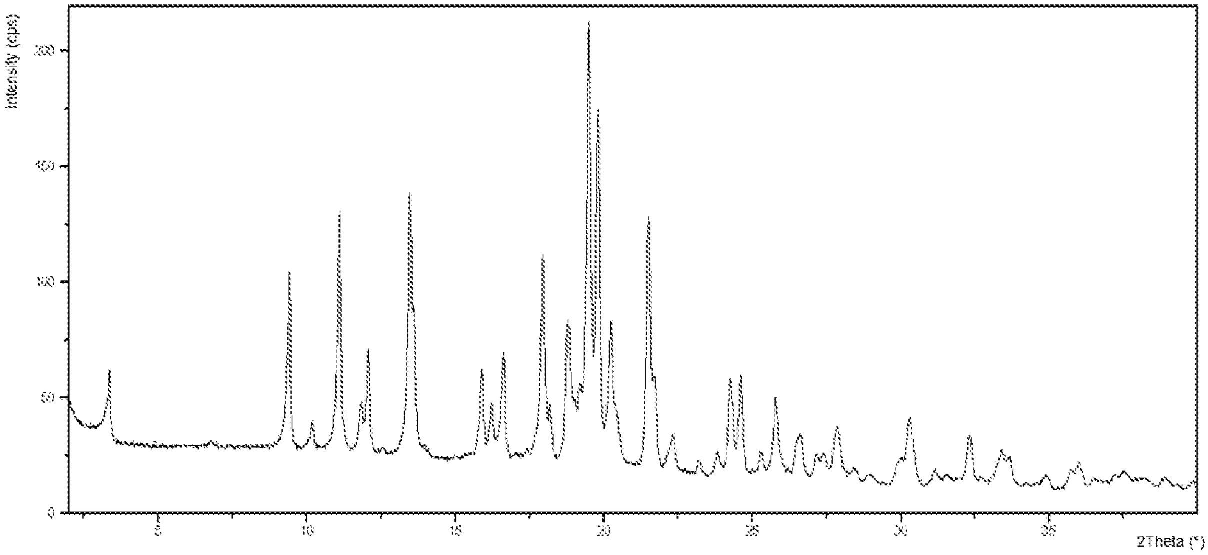


Figure 15

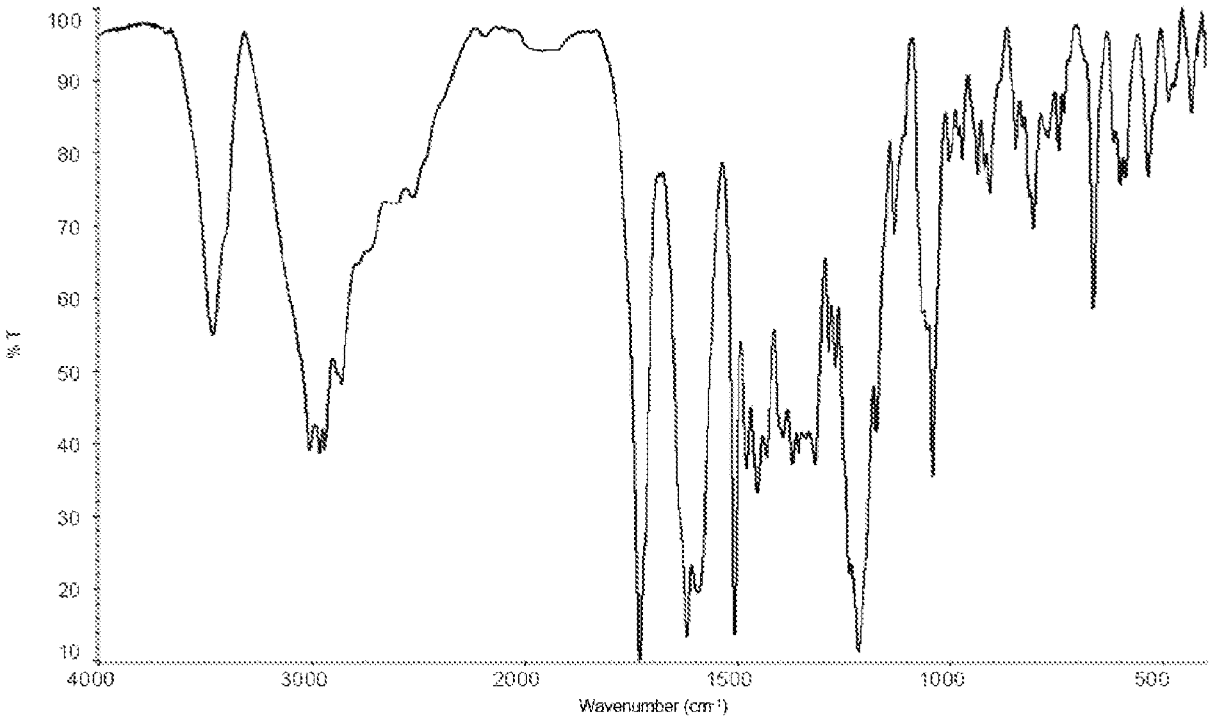


Figure 16

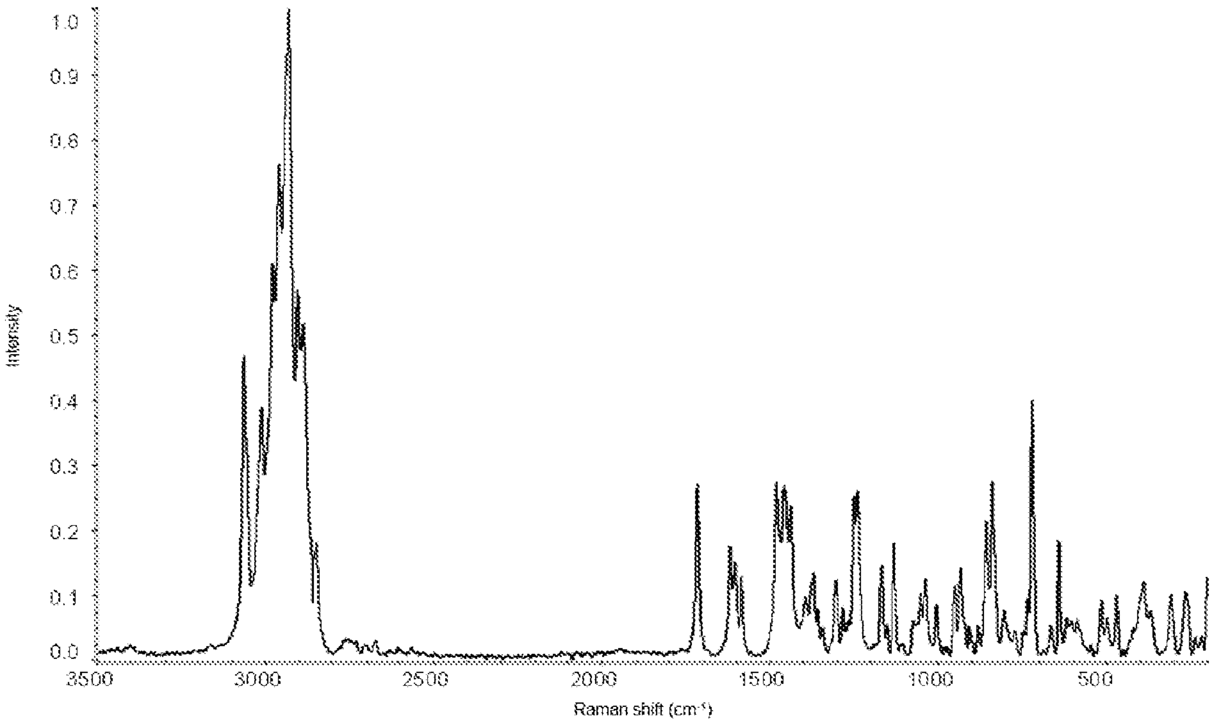


Figure 17

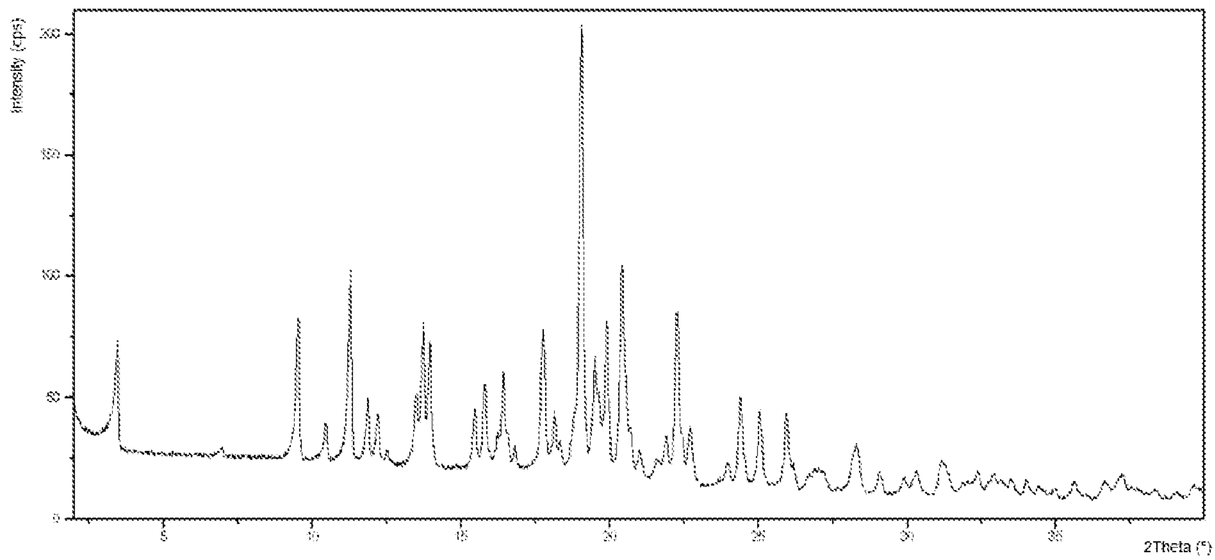


Figure 18

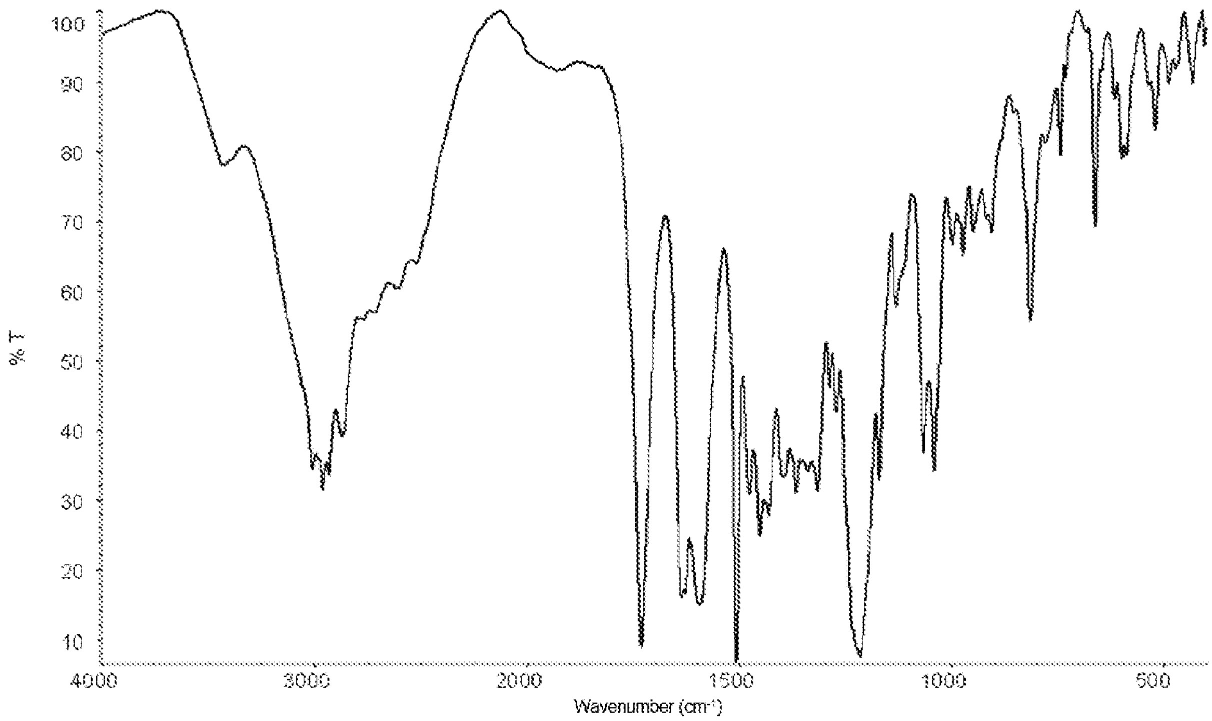


Figure 19

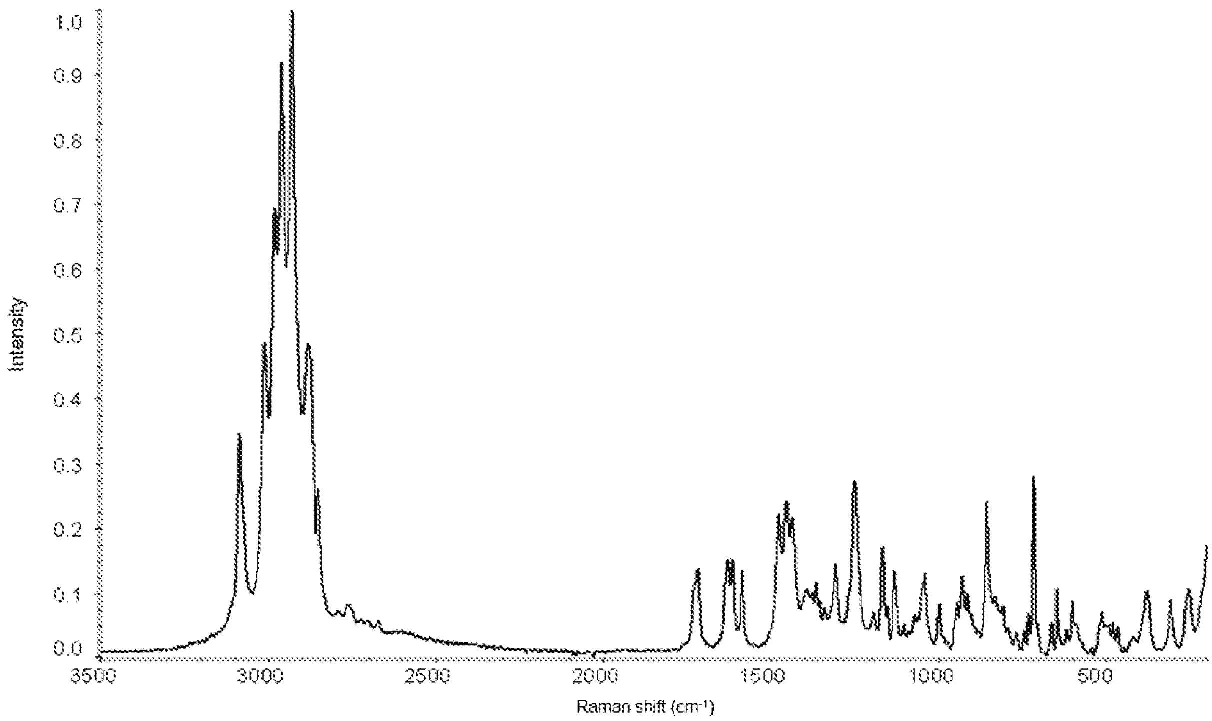


Figure 20

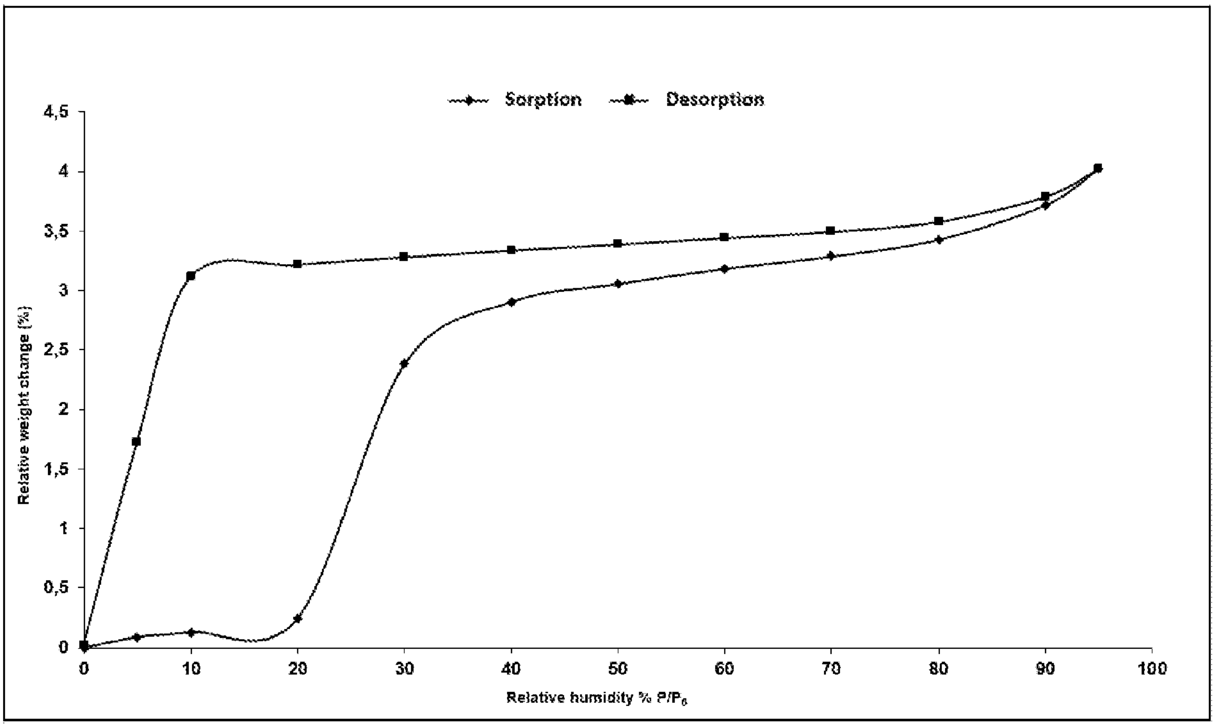


Figure 21

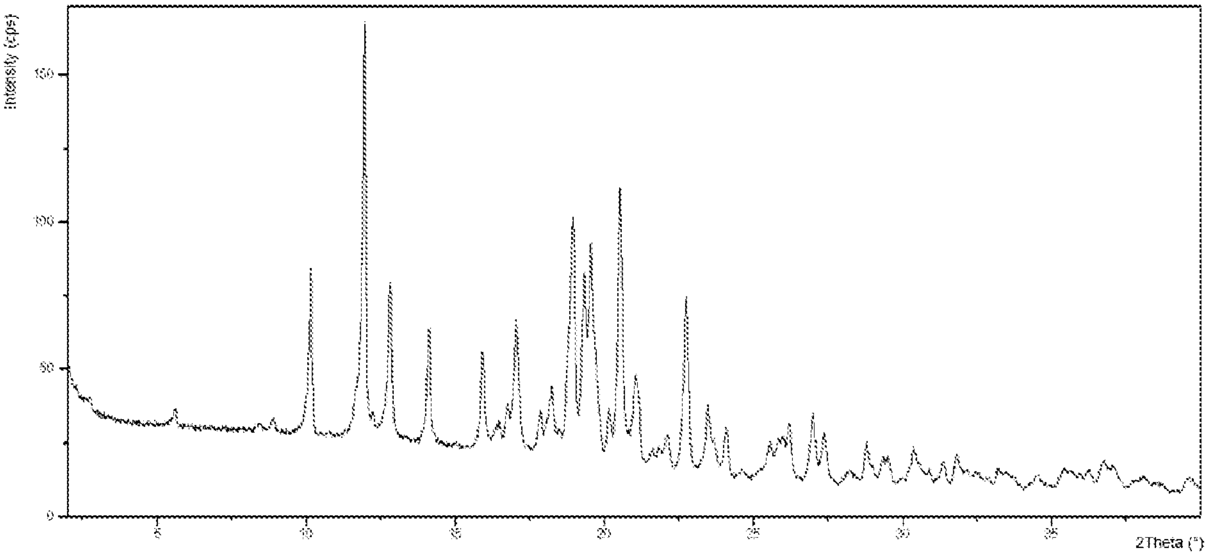


Figure 22

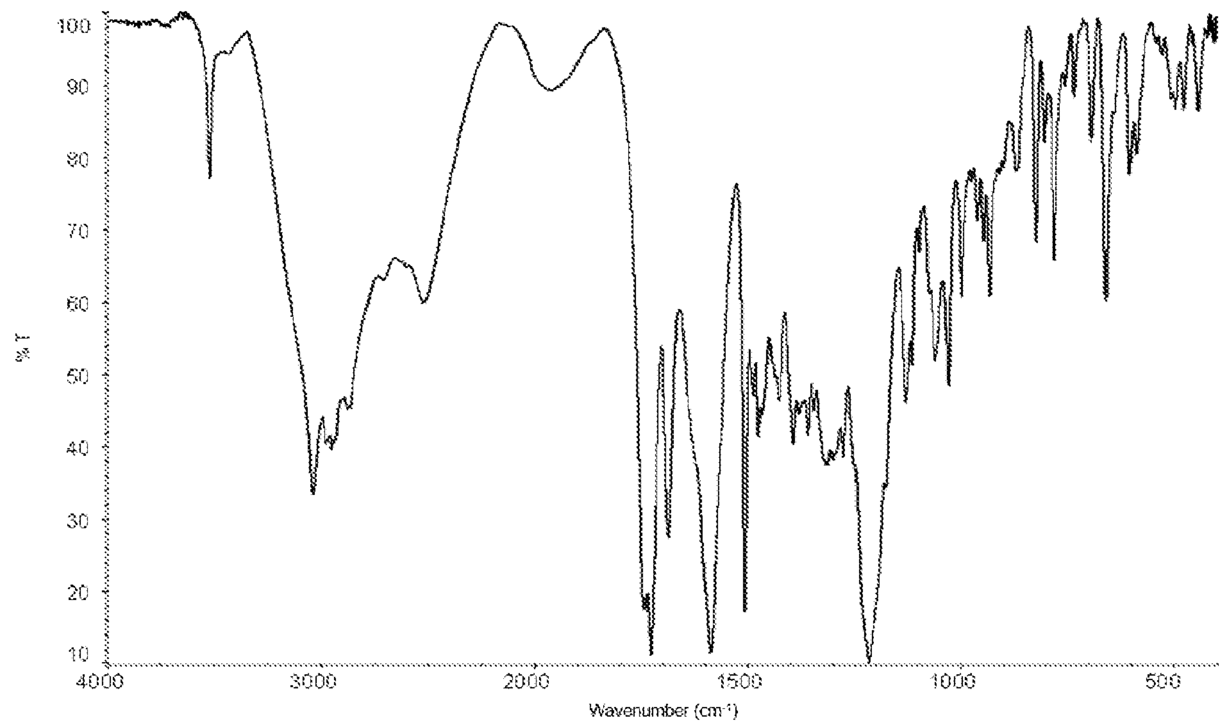


Figure 23

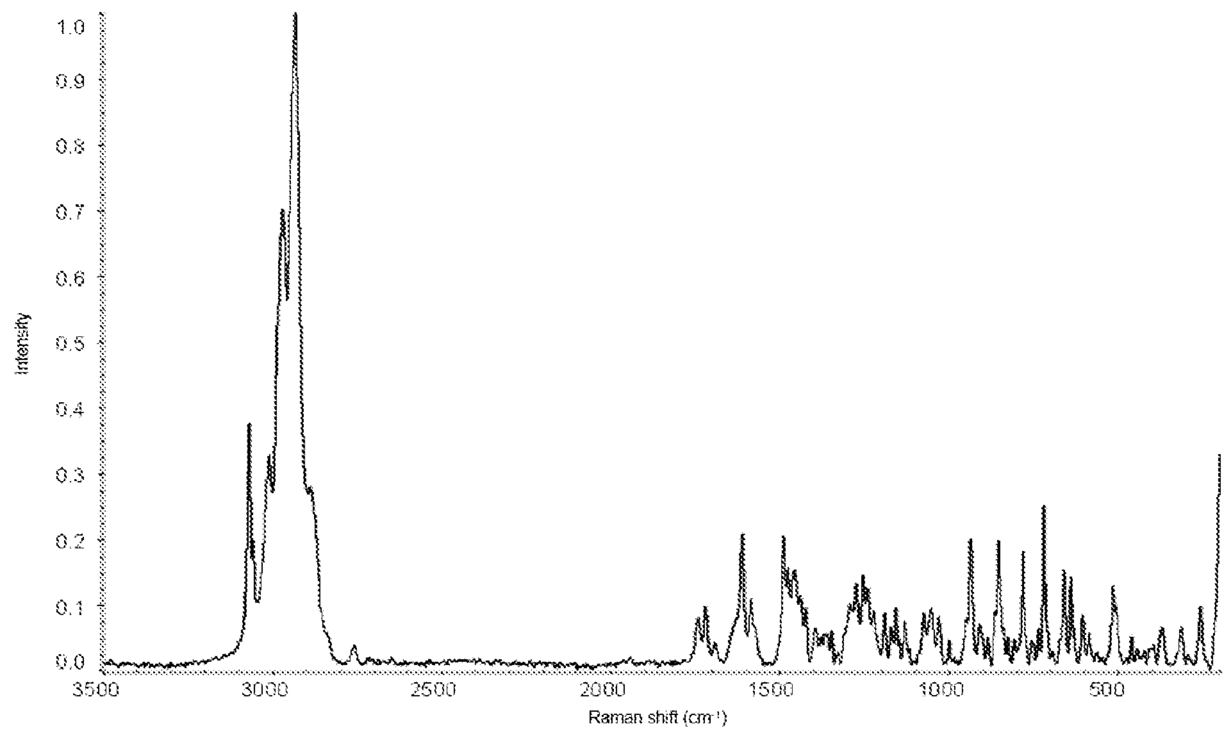


Figure 24

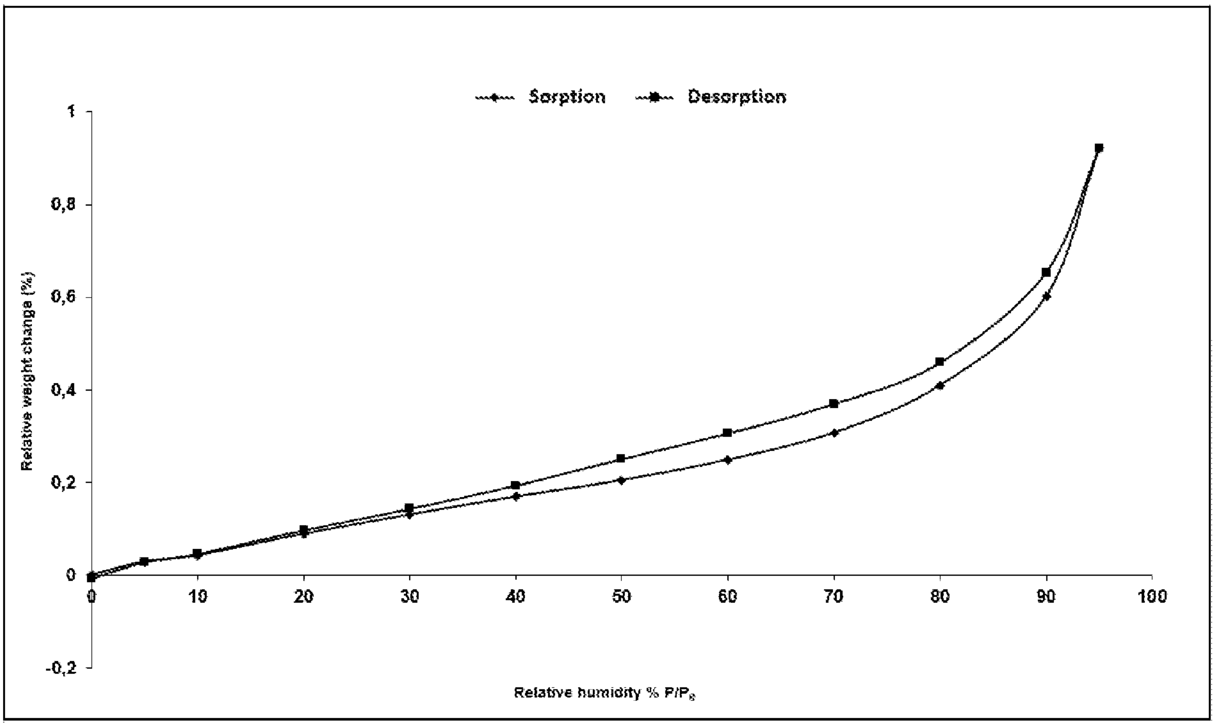


Figure 25

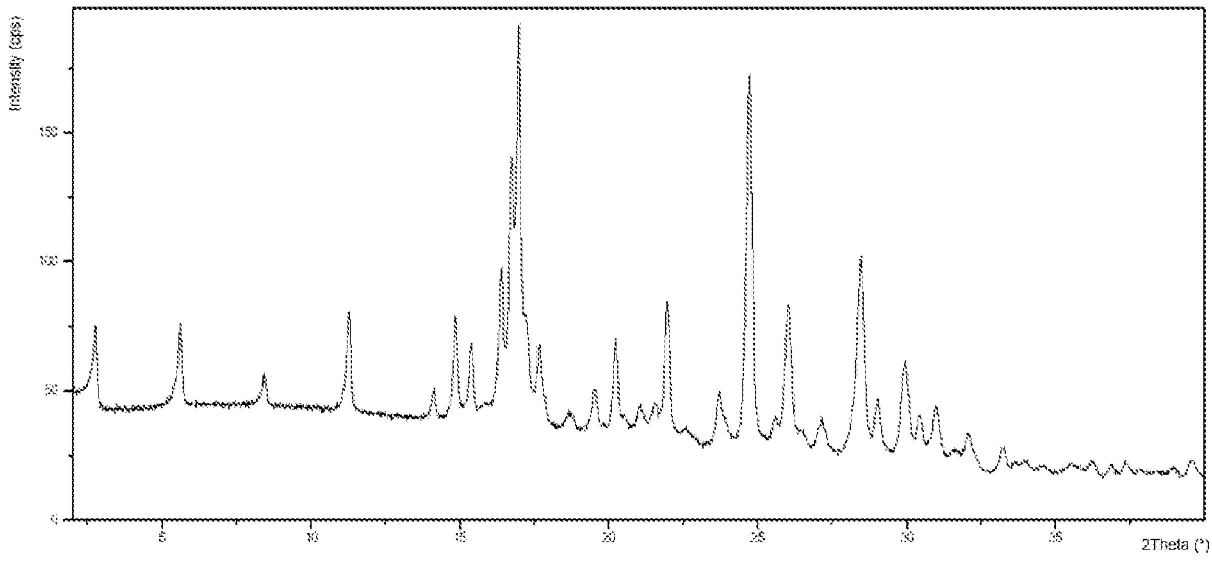


Figure 26

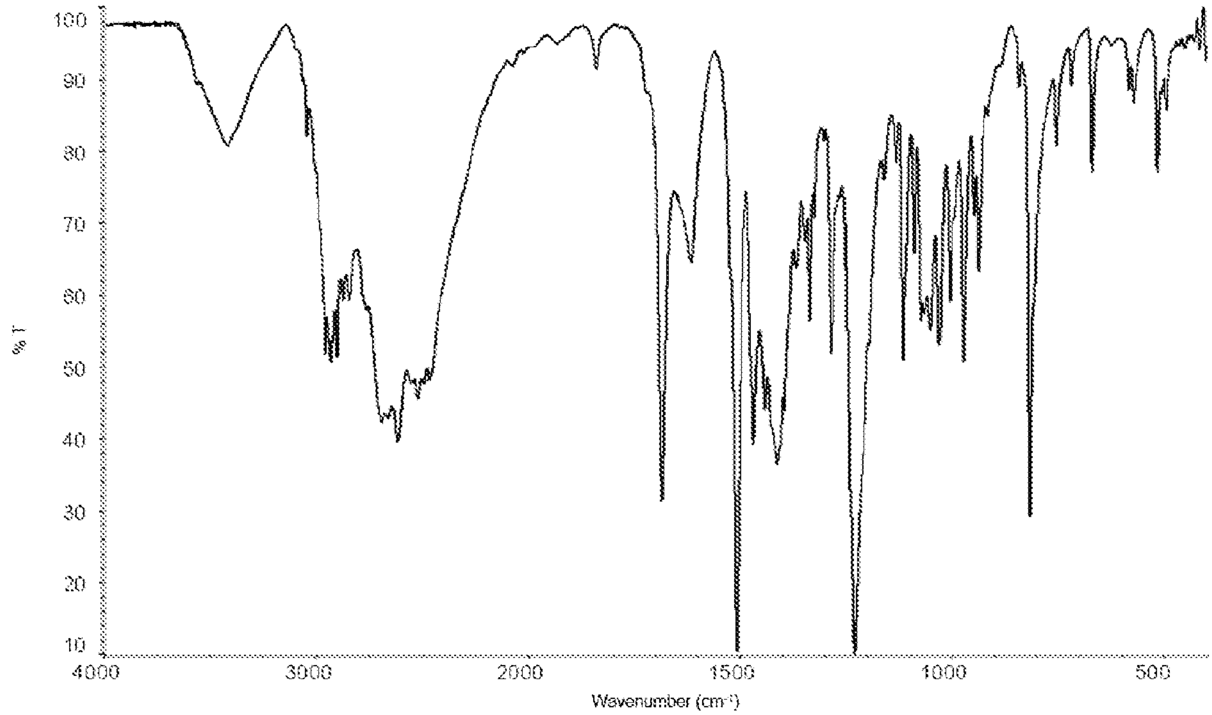


Figure 27

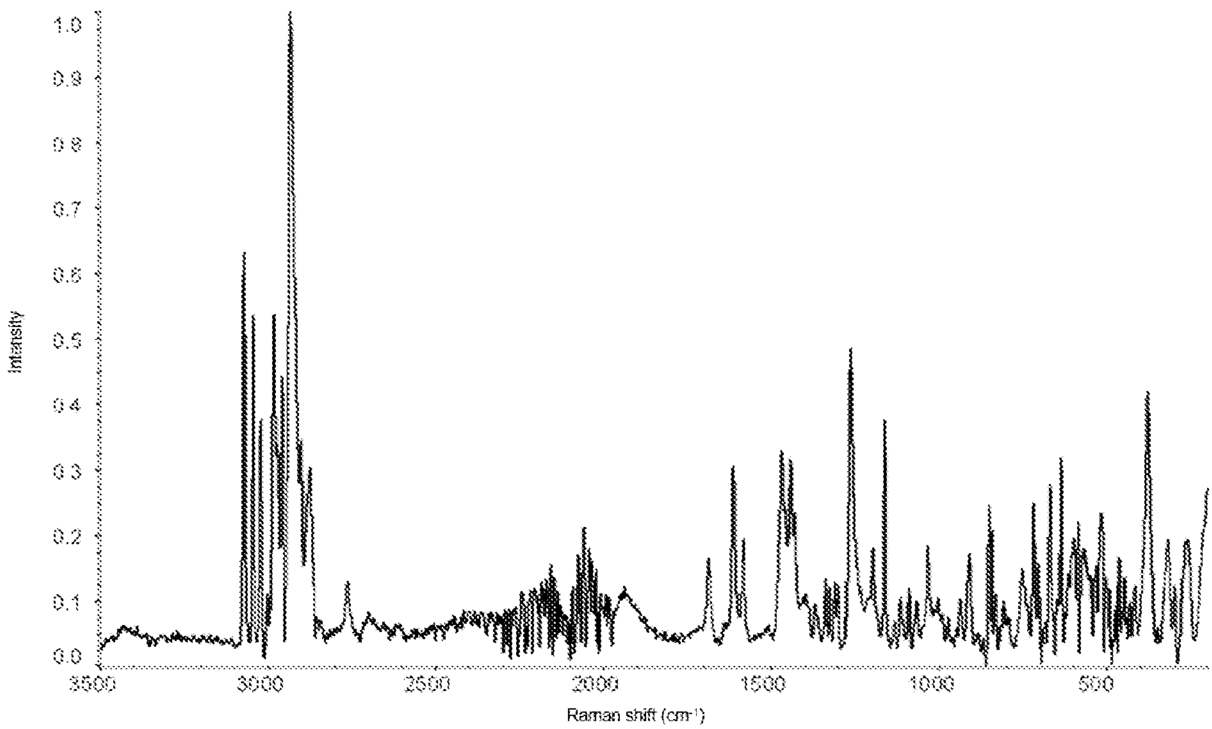


Figure 28

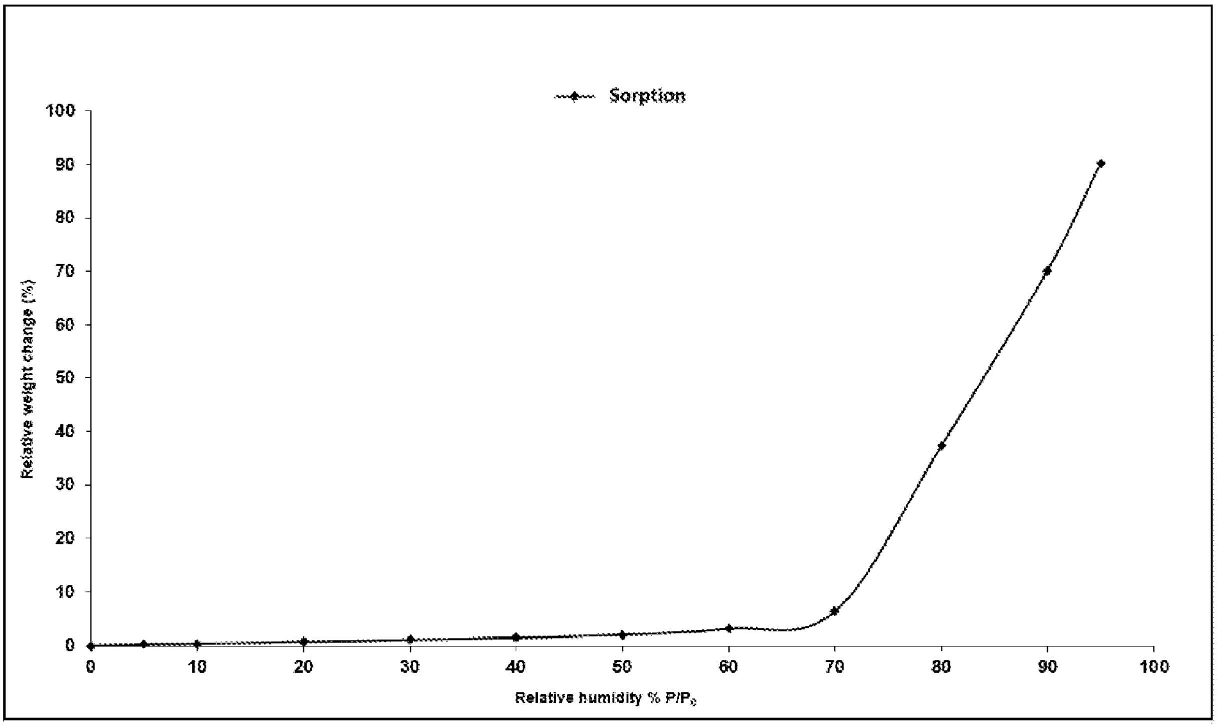


Figure 29

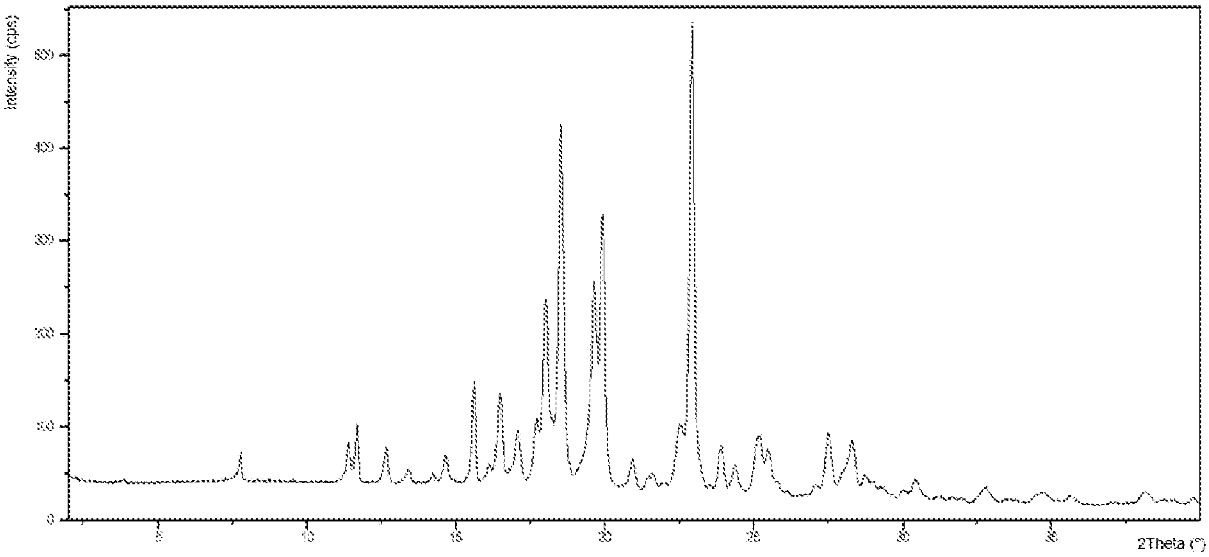


Figure 30

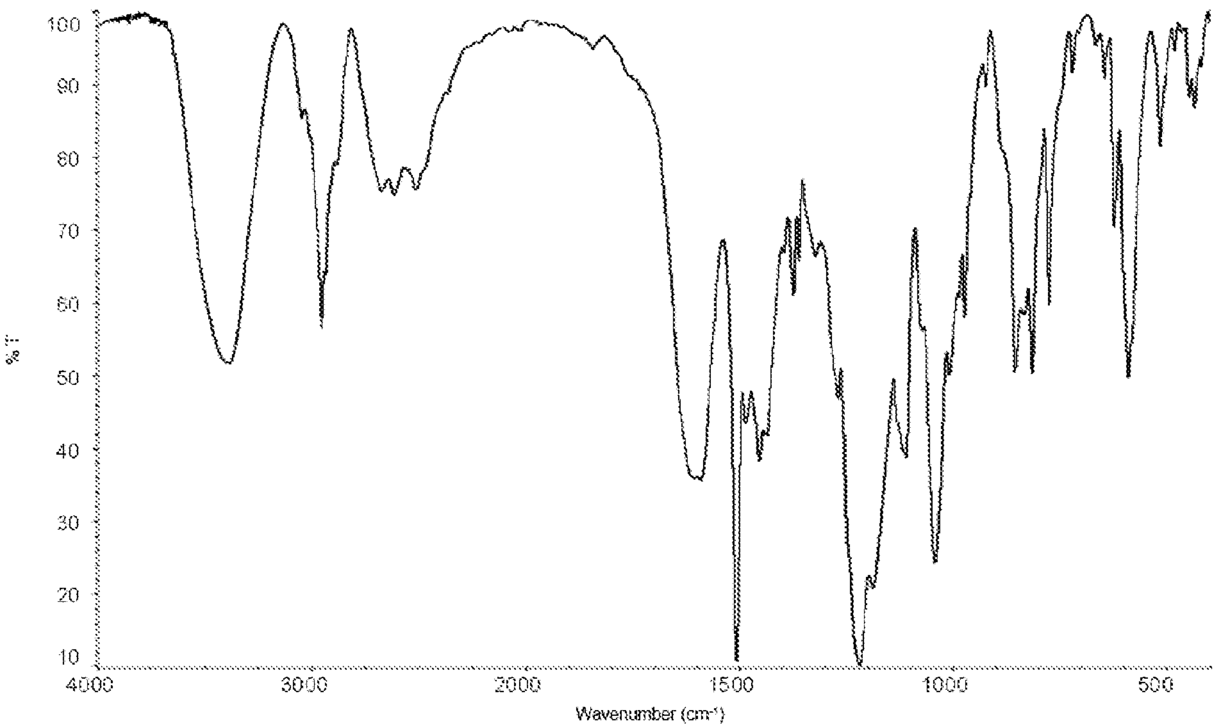


Figure 31

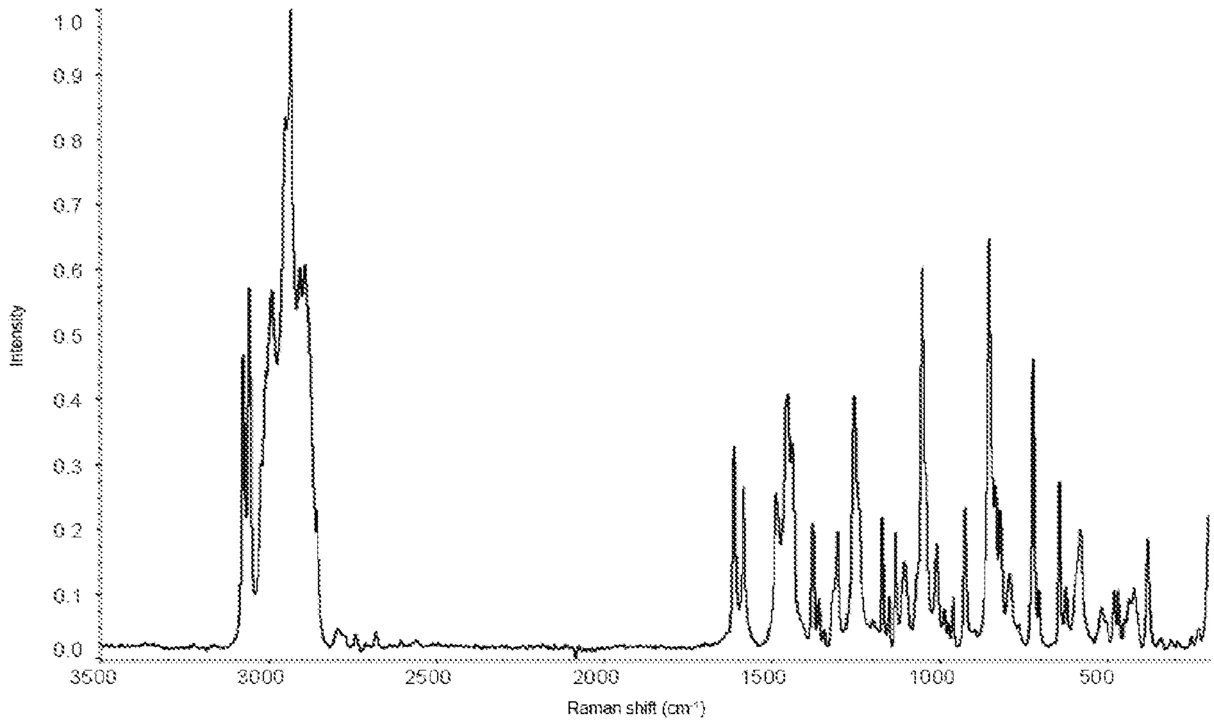


Figure 32

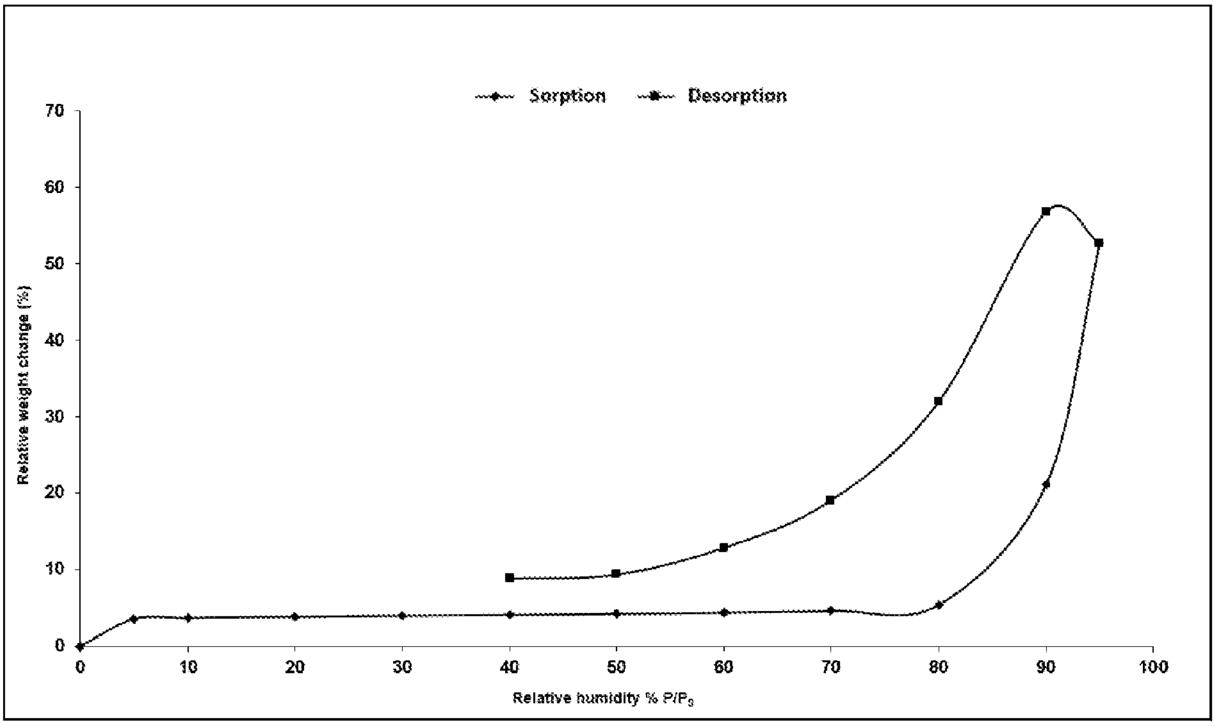


Figure 33

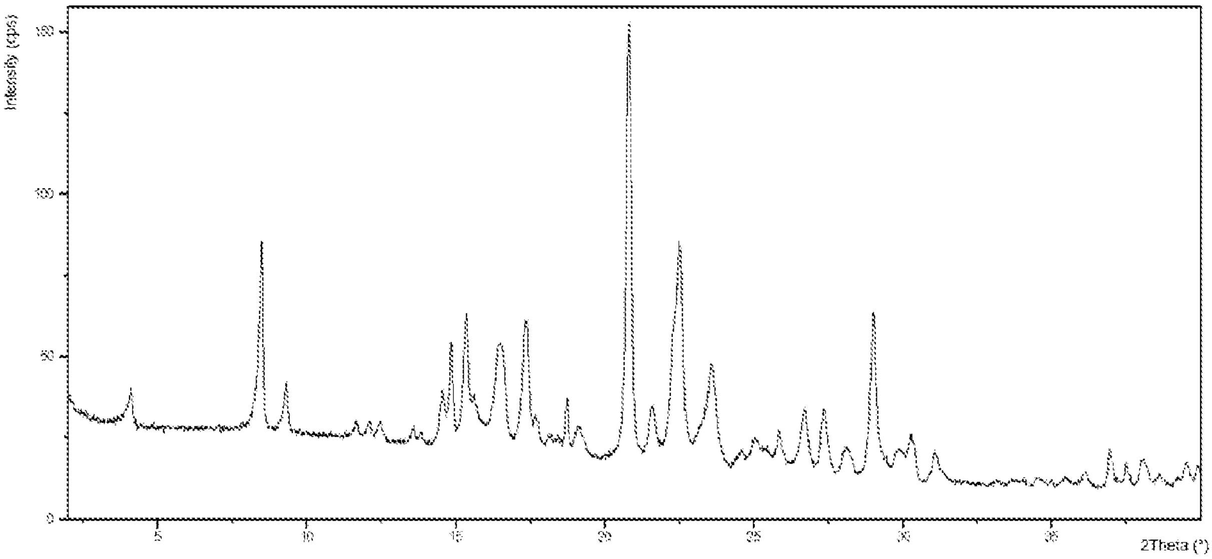


Figure 34

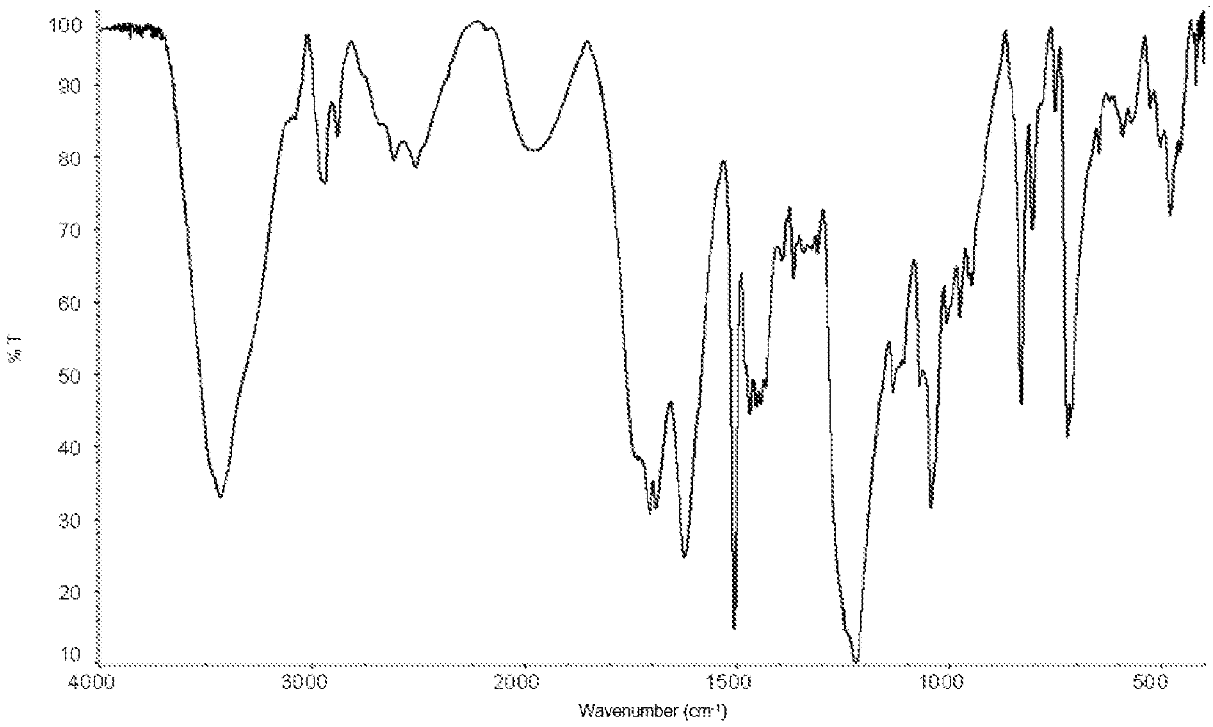


Figure 35

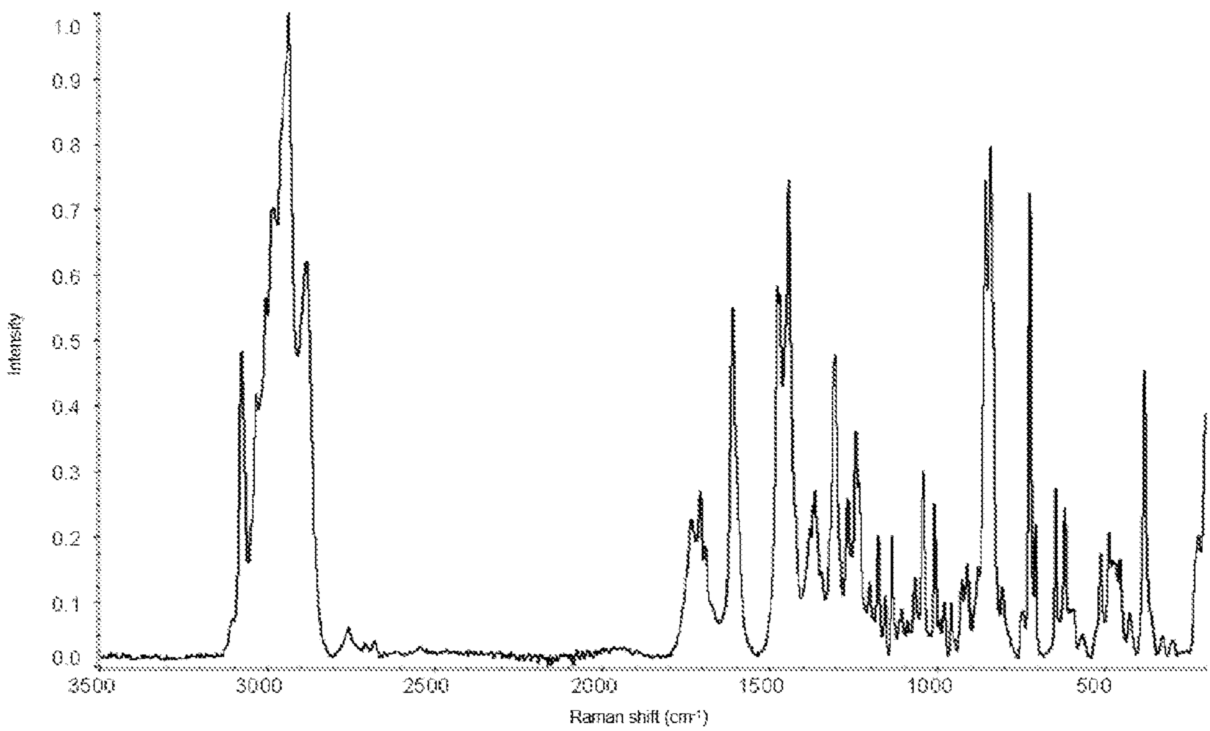


Figure 36

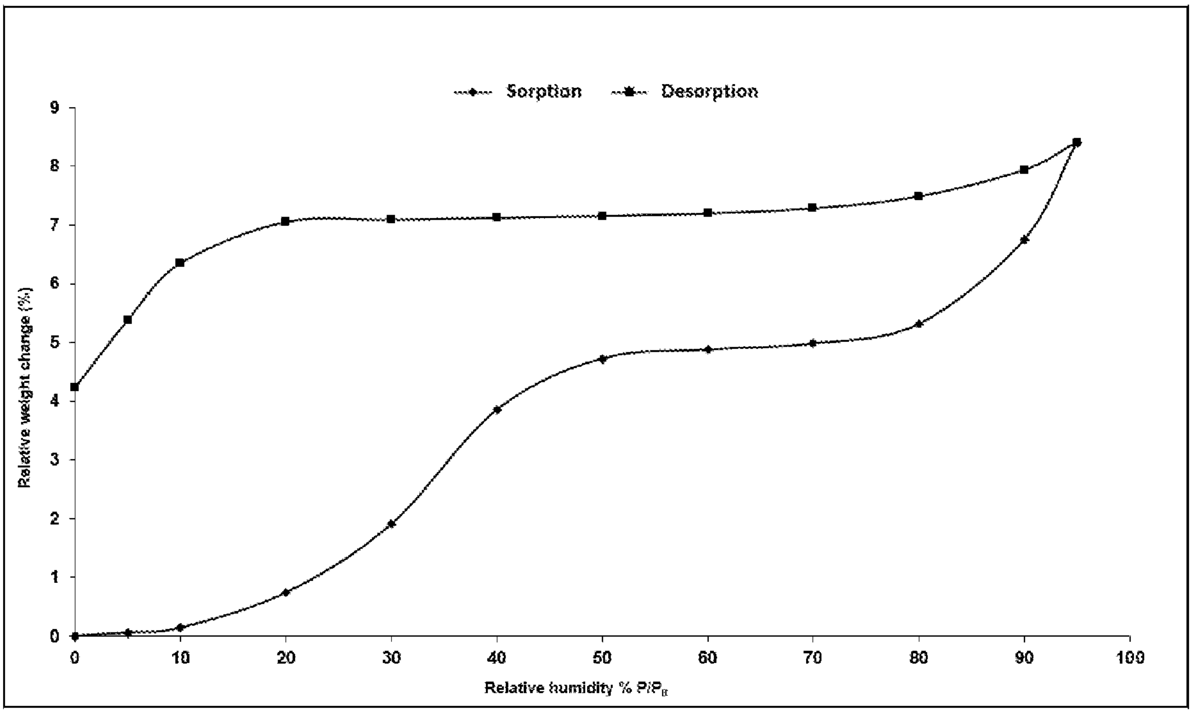
Sorption
Desorption
Relative weight change (%)
Relative humidity % P/P0
0
1
2
3
4
5
6
7
8
9
0
10
20
30
40
50
60
70
80
90
100

Figure 37

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014136075 A


### Non-patent literature cited in the description


- **LEURS et al.** *Nat. Rev. Drug. Disc,* 2005, vol. 4 (2), 107-120
- **BERLIN et al.** *J. Med. Chem.,* 2011, vol. 54 (1), 26-53
- **KUHNE et al.** *Exp. Opin. Inv. Drugs,* 2011, vol. 20 (12), 1629-1648
- **BARBIER ; BRADBURY.** *CNS Neurol. Disord. Drug Targets,* 2007, vol. 6 (1), 31-43
- **KOLLB-SIELECKA et al.** *Sleep Med.,* 2017, vol. 33, 125-129
- **NEWMAN et al.** *J. Pharm. Sci.,* 2007, vol. 97 (3), 1047-1059
- **MAUER et al.** *Pharm. Dev. Techn,* 2010, vol. 15 (6), 582-594